# EUROPEAN PATENT APPLICATION

(11) **EP 0 825 187 A1**
(43) Date of publication of application: **25.02.1998**
(21) Application number: 97114342.5
(22) Date of filing: 20.08.1997
(51) Int. Cl.: C07D 237/34, C07D 487/04, C07D 409/04, A61K 31/50

(54) **Condensed pyridazinyl guanidines, their production and use**

(30) Priority: 22.08.1996 JP 221553/96
(71) Applicant: TAKEDA CHEMICAL INDUSTRIES, LTD., Chuo-ku, Osaka 541 (JP)
(72) Inventor: Shiraishi, Mitsuru, Hyogo 661 (JP); Imamiya, Eiko, Hyogo 665 (JP); Kusumoto, Keiji, Mishima-gun, Osaka 618 (JP); Ichimori, Yuzo, Sakai, Osaka 592 (JP)
(74) Representative: von Kreisler, Alek, Dipl.-Chem.

(57) **Abstract**

Pyridazinyl guanidines of the formula: 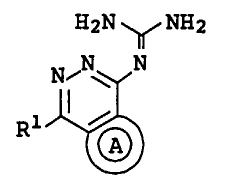 wherein ring A is a benzene ring or a nitrogen-containing 6-membered aromatic ring, each of which may be substituted; and R¹ is an aromatic ring group which may be substituted, or a salt thereof, which have activity for inhibiting Na-H exchange and are useful as a prophylactic/therapeutic agent for ischemic cardiovascular diseases such as myocardial infarction and arrythmia.

## Description

The present invention relates to novel condensed ring pyridazinylguanidine derivatives which are useful as medicines, their production and agent. The novel condensed ring pyridazinylguanidine derivatives of the present invention has prophylactic (preventive) and therapeutic (treating) effects on myocardial infarction and accompanying hypofunctions, arrythmia, unstable angina pectoris, cardiac hypertrophy, reobstruction after PTCA (percutaneous transluminal coronary angioplasty), hypertension and accompanying tissue disorders, etc., on the basis of activities for inhibiting sodium-hydrogen (Na-H) exchange.

### Background Art

Na-H exchange inhibitors, assumed to exhibit ameliorating and cell-protecting action in cell disorders under ischemic conditions, especially on the myocardium, are drawing attention in the field of therapeutic drugs for ischemic diseases.

Amiloride, an acylguanidine derivative and potassium-retaining diuretic, possesses weak activities for inhibiting Na-H exchange and potent activities for inhibiting sodium channel.

As Na-H exchange inhibitors, various acylguanidine derivatives are disclosed in Japanese Patent Unexamined Publication No. 228082/1994, WO 96/04241, EP 708091, EP 708088, etc.

However, condensed ring pyridazinylguanidine derivatives exhibiting excellent activities for inhibiting Na-H exchange have not been known yet.

Amiloride causes hypotension and salt excretion, which activities are undesirable for the treatment of heart rate disorder, by its potent activities for inhibiting sodium channel.

### Detailed Description of the Invention

The present invention is to provide novel condensed ring pyridazinylguanidine derivatives having preventing and treating effects on myocardial infarction and accompanying dysfunctions, arrythmia, unstable angina pectoris, cardiac hypertrophy, reobstruction after PTCA, hypertension and accompanying tissue disorders, etc., on the basis of activities for inhibiting Na-H exchange.

The present inventors studied on various compounds possessing activities for inhibiting Na-H exchange, and as the result, for the first time synthesized a compound of the formula: 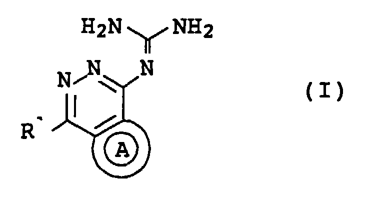 wherein ring A is a benzene ring or a nitrogen-containing 6-membered aromatic ring, each of which may be substituted; and R¹ is an aromatic ring group which may be substituted, or a salt thereof (hereinafter referred to as compound (I)), whose chemical structure is characterized by that a guanidyl group is bound directly to the 3-position of condensed ring pyridazinyl, and found that this compound (I) unexpectedly exhibits excellent activities for inhibiting Na-H exchange based on the unique chemical structure and has clinically desirable pharmaceutical effects. The present inventors made further investigation based on this finding, and accomplished the present invention.

Accordingly, the present invention relates to:
1) the compound (I),
2) the compound of the above 1), wherein the ring A is a benzene ring, pyridine ring, pyrazine ring or pyridazine ring which may be substituted,
3) the compound of the above 1), wherein the ring A is a benzene ring, a pyridine ring, a pyrazine ring or a pyridazine ring, each of which may be substituted with (1) a halogen atom, (2) a hydroxy group, (3) a nitro group, (4) a cyano group, (5) a lower alkyl group which may be substituted with halogen, (6) a lower alkoxy group which may be substituted with halogen, (7) a lower acyl group, (8) a mercapto group which may be substituted with a lower alkyl group, (9) an amino group which may be substituted with a lower alkyl group, a lower alkoxy group, a halogeno lower alkyl group, a C₃₋₆ cyclo alkyl group, a hydroxy group, a carbamoyl group, a phenyl group, a phenyl-C₁₋₆ alkyl group, a lower alkanoyl group, a C₃₋₆ cycloalkylcarbonyl group, a benzoyl group, a phenyl C₂₋₄ alkanoyl group, a lower alkoxy-carbonyl group, a phenoxy-carbonyl group, a phenyl-C₁₋₄ alkoxy-carbonyl group, a lower alkylsulfinyl group, a C₃₋₆ cycloalkylsulfinyl group, a phenylsulfinyl group, a lower sulfinyl group, a C₃₋₆ cycloalkylsulfonyl group, a lower alkoxysulfonyl group or a phenylsulfonyl group or (10) a phenyl group which may be substituted with a halogen atom, a hydroxy group, a nitro group, a cyano group, a lower alkyl group which may be substituted with a halogen atom, a lower alkoxy group which may be substituted with a halogen atom, a lower acyl group or a mercapto group which may be substituted with a lower alkyl group,
4) the compound of the above 1), which is a compound of the formula: 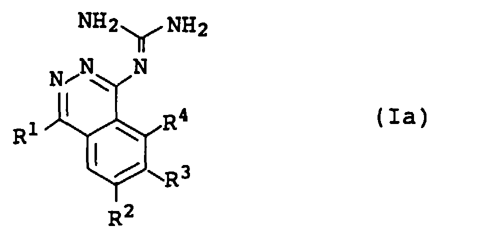 wherein R², R³ and R⁴ are independently (1) a halogen atom, (2) a hydroxy group, (3) a nitro group, (4) a cyano group, (5) a lower alkyl group which may be substituted with halogen, (6) a lower alkoxy group which may be substituted with halogen, (7) a lower acyl group, (8) a mercapto group which may be substituted with a lower alkyl group, (9) an amino group which may be substituted or (10) a phenyl group which may be substituted; and the other symbol is as defined above, or a salt thereof (hereinafter referred to as compound (Ia)),
5) the compound of the above 1), wherein R¹ is a phenyl group, a pyridyl group or a thienyl group, each of which may be substituted with (1) a halogen atom, (2) a hydroxy group, (3) a nitro group, (4) a cyano group, (5) a lower alkyl group which may be substituted with halogen, (6) a lower alkoxy group which may be substituted with halogen, (7) a lower acyl group or (8) a mercapto group which may be substituted with a lower alkyl goroup,
6) the compound of the above 1), wherein R¹ is a phenyl group or a thienyl group which may be substituted with a halogen atom or a lower alkyl group,
7) the compound of the above 4), wherein R¹ is a phenyl group substituted with a lower alkyl group at the 2-position of the phenyl group,
8) the compound of the above 4), wherein R² and R³ are hydrogen atoms,
9) the compound of the above 4), wherein R⁴ is a halogen atom or a lower alkyl group,
10) the compound of the above 4), which is 8-bromo-4-(2-methylphenyl)phthalazin-1-ylguanidine or a salt thereof,
11) the compound of the above 4), which is 8-methyl-4-(2-methylphenyl)phthalazin-1-ylguanidine or a salt thereof,
12) a pharmaceutical composition which comprises the compound (I),
13) a pharmaceutical composition of the above 12), which is an Na-H exchange inhibitor,
14) a pharmaceutical composition of the above 13), wherein the Na-H exchange inhibitor is a preventing and treating agent for an ischemic cardiovascular disease,
15) a pharmaceutical composition of the above 14), wherein the ischemic cardiovascular disease is myocardial infarction or arrythmia, and
16) a process for producing the compound (I) which comprises reacting a compound of the formula: 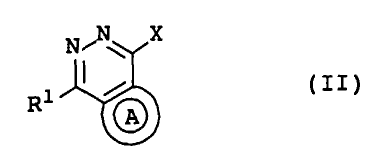 wherein X is a leaving group and the other symbols are as defined above or a salt thereof (hereinafter referred to as compound (II)), and a compound of the formula: 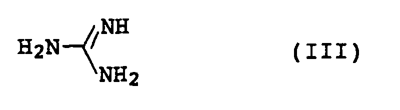 or a salt thereof (hereinafter referred to as compound (III)).

In the above formula (I), ring A is a benzene ring or a nitrogen-containing 6-membered aromatic ring, each of which may be substituted.

The nitrogen-containing 6-membered aromatic ring represented by ring A is exemplified by pyridine rings, pyrazine rings and pyridazine rings.

Here, the ring A is condensed with the 4- and 5-positions (side d) of a pyridazine ring having a guanidyl group at the 3-position; for example, a pyridine ring is condensed at the 2- and 3-positions (side b) or the 3- and 4-positions (side c), a pyrazine ring is condensed at the 2- and 3-positions (side b), and a pyridazine ring is condensed at the 3- and 4-positions (side c) or at the 4-and 5-positions (side d).

The benzene ring or nitrogen-containing 6-membered aromatic ring represented by ring A may be substituted with 1 to 4 (preferably 1 or 2) identical or different substituents selected from (1) halogen atoms, (2) hydroxy groups, (3) nitro groups, (4) cyano groups, (5) lower alkyl groups which may be substituted with halogen atoms, (6) lower alkoxy groups which may be substituted with halogen atoms, (7) lower acyl groups, (8) mercapto groups which may be substituted with lower alkyl groups, (9) amino groups which may be substituted, and (10) phenyl groups which may be substituted, at any substitutable positions.

The halogen atoms mentioned in (1) above include, for example, chlorine, bromine, fluorine and iodine.

The lower alkyl groups mentioned in (5) above, which may be substituted with halogen atoms (e.g., chlorine, bromine, fluorine, iodine), include, for example, C₁₋₆ alkyl groups (e.g., methyl, ethyl, propyl, isopropyl, butyl, isobutyl, sec-butyl, tert-butyl, pentyl, hexyl) and halogeno-C₁₋₆ alkyl groups (e.g., CF₃, CF₂CF₃, CH₂F, CHF₂).

The lower alkoxy groups mentioned in (6) above, which may be substituted with halogen atoms (e.g., chlorine, bromine, fluorine, iodine), include C₁₋₆ alkoxy groups (e.g., methoxy, ethoxy, propoxy, isopropoxy, butoxy, sec-butoxy, tert-butoxy, pentyloxy, isopentyloxy, neopentyloxy, hexyloxy) and halogeno-C₁₋₆ alkoxy groups (e.g., CF₃O, CHF₂O).

The lower acyl groups mentioned in (7) above include, for example, lower acyl groups derived from carboxylic acids, sulfinic acids or sulfonic acids.

Here, the lower acyl groups derived from carboxylic acids include, for example, lower (C₁₋₆) alkyl-carbonyl groups (alkanoyl groups) (e.g., formyl, acetyl, propionyl, butyryl, isobutyryl, valeryl, pivaloyl), C₃₋₆ cycloalkylcarbonyl groups (e.g., cyclopropylcarbonyl, cyclobutylcarbonyl, cyclopentylcarbonyl, cyclohexylcarbonyl) and benzoyl group.

The lower acyl groups derived from sulfinic acids include, for example, lower (C₁₋₆) alkylsulfinyl groups (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl), C₃₋₆ cycloalkylsulfinyl groups (e.g., cyclopropylsulfinyl, cyclobutylsulfinyl, cyclopentylsulfinyl, cyclohexylsulfinyl) and phenylsulfinyl group.

The lower acyl groups derived from sulfonic acids include, for example, lower (C₁₋₆) alkylsulfonyl groups (e.g., methylsulfonyl, ethylsulfonyl, propylsulfonyl, isopropylsulfonyl, butylsulfonyl, isobutylsulfonyl, tert-butylsulfonyl, pentylsulfonyl, hexylsulfonyl), C₃₋₆ cycloalkylsulfonyl groups (e.g., cyclopropylsulfonyl, cyclobutylsulfonyl, cyclopentylsulfonyl, cyclohexylsulfonyl) and phenylsulfonyl group.

The mercapto groups mentioned in (8) above, which may be substituted with lower alkyl groups (C₁₋₆ alkyl groups such as e.g., methyl, ethyl, propyl, isopropyl, butyl, isobutyl, tert-butyl, pentyl and hexyl), include, for example, mercapto groups and C₁₋₆ alkylthio groups (e.g., methylthio, ethylthio, propylthio, isopropylthio, butylthio, isobutylthio, tert-butylthio, pentylthio, hexylthio).

The amino groups which may be substituted mentioned in (9) above, include, for example, amino groups which may be substituted with 1 or 2 identical or different substituents selected from lower (C₁₋₆) alkyl groups (e.g., methyl, ethyl, propyl, isopropyl, butyl, isobutyl, sec-butyl, tert-butyl, pentyl, hexyl), lower (C₁₋₆) alkoxy groups (e.g., methoxy, ethoxy, propoxy, isopropoxy, butoxy, isobutoxy, sec-butoxy, tert-butoxy, pentyloxy, hexyloxy), halogeno-lower (C₁₋₆) alkyl groups (e.g., CF₃, CF₃CF₂, CH₂F, CHF₂), C₃₋₆ cycloalkyl groups (e.g., cyclopropyl, cyclobutyl, cyclopentyl, cyclohexyl), hydroxy groups, carbamoyl groups, phenyl groups, phenyl-C₁₋₆ alkyl groups (e.g., benzyl, phenethyl, 3-phenylpropyl, 4-phenylbutyl), lower (C₁₋₆) alkanoyl groups (e.g., formyl, acetyl, propionyl, butyryl, isobutyryl, valeryl, pivaloyl), C₃₋₆ cycloalkyl-carbonyl groups (e.g., cyclopropylcarbonyl, cyclobutylcarbonyl, cyclopentylcarbonyl, cyclohexylcarbonyl), benzoyl group, phenyl-C₂₋₄ alkanoyl groups (e.g., phenylacetyl, phenylpropionyl), lower (C₁₋₆) alkoxy-carbonyl groups (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl), phenoxy-carbonyl groups, phenyl-C₁₋₄ alkoxy-carbonyl groups (e.g., benzyloxycarbonyl, phenylethoxycarbonyl), lower (C₁₋₆) alkylsulfinyl groups (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl), C₃₋₆ cycloalkylsulfinyl groups (e.g., cyclopropylsulfinyl, cyclobutylsulfinyl, cyclopentylsulfinyl, cyclohexylsulfinyl), phenylsulfinyls, lower (C₁₋₆) alkylsulfonyl groups (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl), C₃₋₆ cycloalkylsulfonyl groups (e.g., cyclopropylsulfonyl, cyclobutylsulfonyl, cyclopentylsulfonyl, cyclohexylsulfonyl), lower (C₁₋₆) alkoxysulfonyl groups (e.g., methoxysulfonyl, ethoxysulfonyl, propoxysulfonyl, isopropoxysulfonyl, butoxysulfonyl, isobutoxysulfonyl, sec-butoxysulfonyl, tert-butoxysulfonyl, pentyloxysulfonyl, hexyloxysulfonyl) and phenylsulfonyl group.

Also, two such substituents may form a cyclic amino group in cooperation with nitrogen atoms; such cyclic amino groups include, for example, pyrrolidino, piperidino, morpholino and thiomorpholino.

The phenyl groups mentioned in (10) above, which may be substituted, include, for example, phenyl groups which may be substituted with 1 to 4 (preferably 1 or 2) identical or different substituents selected from halogen atoms, hydroxy groups, nitro groups, cyano groups, lower alkyl groups which may be substituted with halogen atoms, lower alkoxy groups which may be substituted with halogen atoms, lower acyl groups, and mercapto groups which may be substituted lower alkyl groups, at any substitutable positions.

Here, the halogen atoms, lower alkyl groups which may be substituted with halogen atoms, lower alkoxy groups which may be substituted with halogen atoms, lower acyl groups, and mercapto groups which may be substituted with lower alkyl groups, all mentioned as substituents for the phenyl groups mentioned in (10) above, are identical to the (1) halogen atoms, (5) lower alkyl groups which may be substituted with halogen atoms, (6) lower alkoxy groups which may be substituted with halogen atoms, (7) lower acyl groups, and (8) mercapto groups which may be substituted for by lower alkyl groups, all mentioned as substituents for the benzene ring or nitrogen-containing 6-membered aromatic ring represented by ring A above.

Preferable example of the ring A include a benzene ring which may be substituted.

In the above formula (I), R¹ represents an aromatic ring group which may be substituted.

The aromatic ring group represented by R¹ is exemplified by phenyl groups, pyridyl groups (2-, 3- or 4-pyridyl) and thienyl groups (2- or 3-thienyl).

The aromatic ring group represented by R¹ may be substituted with 1 to 4 (preferably 1 or 2) identical or different substituents selected from halogen atoms, hydroxy groups, nitro groups, cyano groups, lower alkyl groups which may be substituted with halogen atoms, lower alkoxy groups which may be substituted with halogen atoms, lower acyl groups, mercapto groups which may be substituted with lower alkyl groups, and phenyl groups which may be substituted, at any substitutable positions.

Here, the halogen atoms, lower alkyl groups which may be substituted with halogen atoms, lower alkoxy groups which may be substituted halogen atoms, lower acyl groups, mercapto groups which may be substituted with lower alkyl groups, and phenyl groups which may be substituted, all mentioned as substituents for the aromatic ring group represented by R¹, are identical to the (1) halogen atoms, (5) lower alkyl groups which may be substituted with halogen atoms, (6) lower alkoxy groups which may be substituted with halogen atoms, (7) lower acyl groups, (8) mercapto groups which may be substituted with lower alkyl groups, and (10) phenyl groups which may be substituted all mentioned as substituents for the benzene ring or nitrogen-containing 6-membered aromatic ring represented by ring A above.

Preferable example of compound (I) include compound (Ia).

In the above formula (Ia), R², R³ and R⁴ represents independently (1) a halogen atom, (2) a hydroxy group, (3) a nitro group, (4) a cyano group, (5) a lower alkyl group which may be substituted with halogen, (6) a lower alkoxy group which may be substituted with halogen, (7) a lower acyl group, (8) a mercapto group which may be substituted with a lower alkyl group, (9) an amino goroup which may be substituted or (10) a phenyl group which may be substituted.

Here, (1) a halogen atom, (5) a lower alkyl group which may be substituted with halogen, (6) a lower alkoxy group which may be substituted with halogen, (7) a lower acyl group, (8) a mercapto group which may be substituted with a lower alkyl group, (9) an amino group which may be substituted and (10) a phenyl group which may be substituted, represented by R², R³ and R⁴, are identical to (1) a halogen atom, (5) a lower alkyl group which may be substituted with halogen, (6) a lower alkoxy group which may be substituted with halogen, (7) a lower acyl group, (8) a mercapto group which may be substituted with a lower alkyl group, (9) an amino group which may be substituted and (10) a phenyl group which may be substituted, all mentioned as substituents for the benzene ring or nitrogen-containing 6-membered aromatic ring represented by ring A above.

Preferable examples of R² and R³ include hydrogen atoms.

Preferable examples of R⁴ include a halogen atom or a lower alkyl group.

More preferable examples of the compound (I) include
8-iodo-4-phenylphthalazin-1-ylguanidine or a salt thereof,
8-bromo-4-phenylphthalazin-1-ylguanidine or a salt thereof,
8-bromo-4-(2-methylphenyl)phthalazin-1-ylguanidine or a salt thereof,
8-iodo-4-(2-methylphenyl)phthalazin-1-ylguanidine or a salt thereof,
8-bromo-4-(4-fluoro-2-methylphenyl)phthalazin-1-ylguanidine or a salt thereof,
8-bromo-4-(3-thienyl)phthalazin-1-ylguanidine or a salt thereof,
4-(4-fluorophenyl)-8-iodophthalazin-1-ylguanidine or a salt thereof,
or 8-methyl-4-(2-methylphenyl)phthalazin-1-ylguanidine or a salt thereof.

Among others, the most preferable examples of the compound (I) include 8-bromo-4-(2-methylphenyl)phthalazin-1-ylguanidine or a salt thereof, or 8-methyl-4-(2-methylphenyl)phthalazin-1-ylguanidine or a salt thereof.

Salts of compound (I) or synthesis intermediates thereof include pharmaceutically acceptable salts such as inorganic acid salts, e.g., hydrochloride, hydrobromide, sulfate, nitrate, phosphate; organic acid salts, e.g., acetate, tartrate, citrate, fumarate, maleate, toluenesulfonate, methanesulfonate; metal salts, e.g., sodium salt, potassium salt, calcium salt, aluminum salt; and salts with bases, e.g., triethylamine salt, guanidine salt, ammonium salt, hydrazine salt, quinine salt, cinchonine salt.

Compound (I) may be used as a hydrate.

In the present specification, the starting material compound or synthesis intermediate of compound (I), or a salt thereof, is also referred to as the starting material compound or synthesis intermediate of compound (I) with "or a salt thereof" omitted.

Also, compound (I) is equivalent to compound (I') in terms of chemical structure.

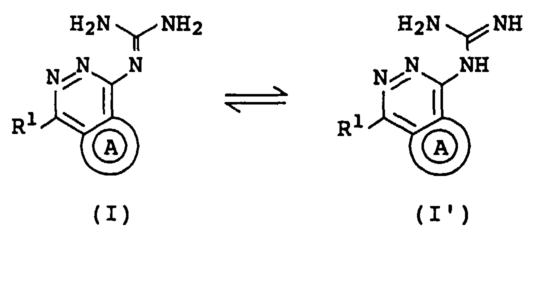

Compound (I) can be produced by, for example, reacting a compound of the formula: 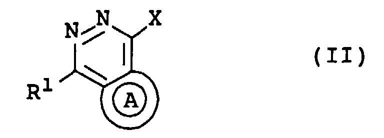 wherein X is a leaving group and the other symbols are as defined in above or a salt thereof, with a guanidine of the formula: 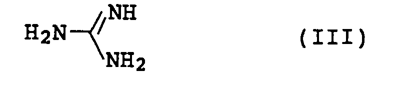 or a salt thereof.

In the above formula (II), the leaving group represented by X is exemplified by halogen atoms, and hydroxy groups esterified by sulfonic acid. Here, the halogen atoms include chlorine, bromine and iodine; the hydroxy groups esterified by sulfonic acid include hydroxy groups esterified by reactive groups such as trifluoromethanesulfonyl, methanesulfonyl and p-toluenesulfonyl.

Compound (III) is normally used at about 1 to 4 mol per mol of compound (II). This reaction can be facilitated by the addition of a base such as triethylamine, pyridine, lithium hydride, sodium hydride, sodium methoxide, sodium ethoxide, potassium tert-butoxide, sodium carbonate or potassium carbonate, which base is added at 1 to about 5 mol per mol of compound (II) as necessary.

For example, this condensation reaction can be carried out in an inert solvent such as methanol, ethanol, propanol, isopropanol, n-butanol, tetrahydrofuran, diethyl ether, dimethoxyethane, 1,4-dioxane, toluene, benzene, xylene, dichloromethane, chloroform, 1,2-dichloroethane, dimethylformamide (DMF), dimethylsulfoxide (DMSO) or pyridine, or a mixed solvent thereof. The reaction is carried out in a temperature range from about 0°C to 180°C. Also, this reaction is preferably carried out in an inert gas (e.g., nitrogen, argon) atmosphere.

Compound (II), used as the starting material, can be produced by a known method or a method based thereon, e.g., the reaction shown by reaction scheme I or a method shown in the reference examples below.

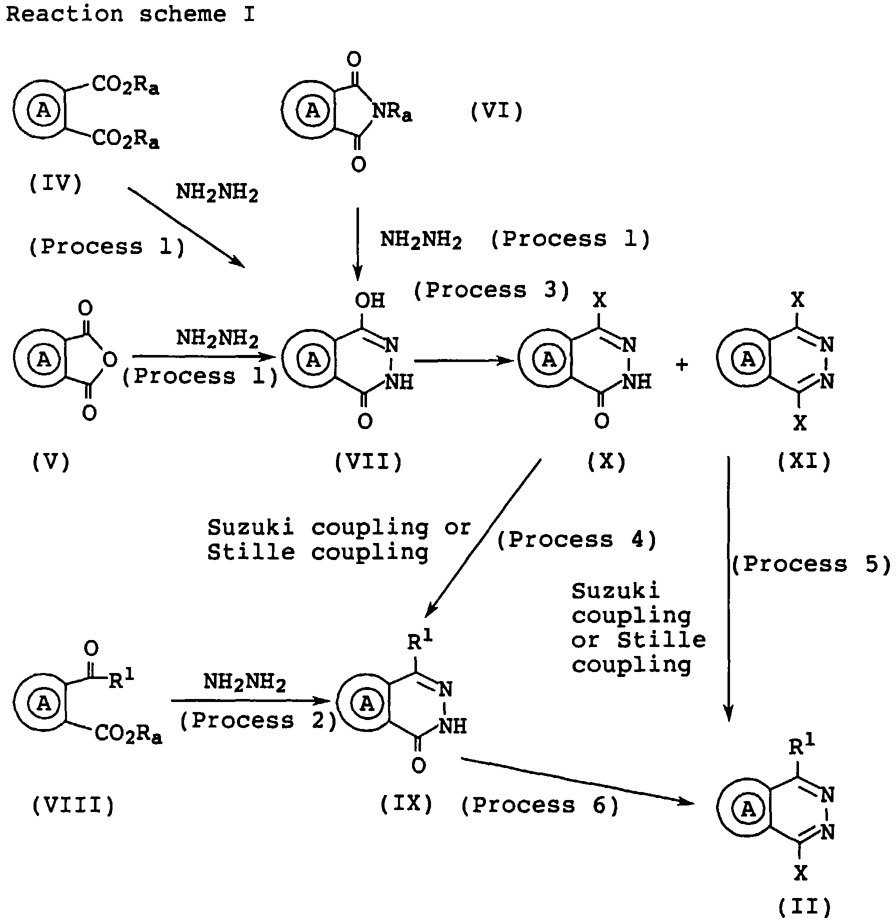

Each process of reaction scheme I is hereinafter described.

### (Process 1) (production of compound (VII))

Compound (VII) of the formula: 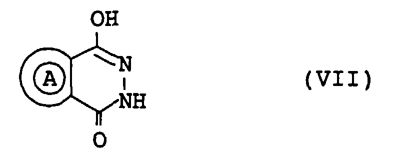 wherein the symbols are defined as above, can be obtained by reacting compound (IV), (V) or (VI), represented by the formula: 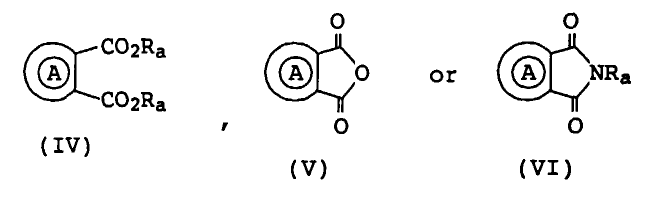 wherein Ra is a hydrogen atom or a lower alkyl group and the other symbols are defined as above, with hydrazine.

The lower alkyl group represented by Ra include, for example, C₁₋₆ alkyl groups such as methyl, ethyl, propyl, isopropyl, butyl, isobutyl, sec-butyl, tert-butyl, pentyl and hexyl.

This reaction can be carried out in an inert solvent, e.g., methanol, ethanol, propanol, isopropanol, n-butanol, tetrahydrofuran, diethyl ether, dimethoxyethane, 1,4-dioxane, toluene, benzene, xylene, dichloromethane, chloroform, 1,2-dichloroethane, DMSO or pyridine, or a mixed solvent thereof. The reaction is carried out in a temperature range from about 20°C to 180°C.

With respect to the hydrazine mentioned above, a hydrazine hydrate is commonly used at 1 to 5 mol per mol of compound (IV), (V) or (VI).

### (Process 2) (production of compound (IX))

Compound (IX) of the formula: 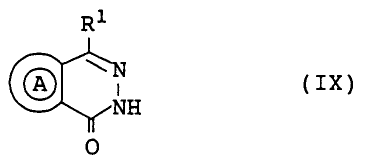 wherein the symbols are defined as above, can be obtained by reacting compound (VIII) of the formula: 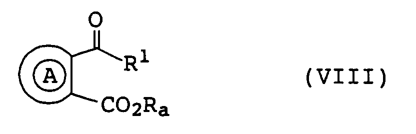 wherein the symbols are defined as above, with hydrazine.

This reaction is carried out in the same manner as process 1.

### (Process 3) (production of compound (X) or (XI))

Compound (X) of the formula: 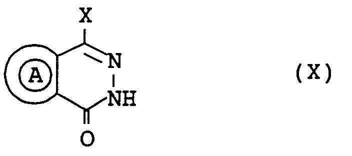 wherein the symbols are defined as above, or compound (XI) of the formula: 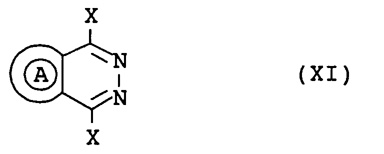 wherein the symbols are defined as above, can be obtained by reacting compound (VII) and a halogenated phosphorous compound such as phosphorus oxychloride or phosphorus pentachloride or a sulfonic anhydride such as trifluoromethanesulfonic anhydride. The reagent is used at about 1 to 5 mol per mol of compound (VII); the amount of compound (XI) produced increases as the amount of reagent used increases.

This reaction can be carried out in an inert solvent, e.g., dichloromethane, chloroform, toluene, benzene, xylene, 1,2-dichloroethane, diethyl ether, tetrahydrofuran or dimethoxyethane, or a mixed solvent thereof. The reaction is carried out in a temperature range from about -30°C to 180°C. For example, when phosphorus oxychloride is used, the reaction can be facilitated by adding DMF, quinoline, dimethylaniline or the like as necessary. When trifluoromethanesulfonic anhydride is used, the reaction can be facilitated by adding triethylamine, pyridine or the like.

### (Process 4) (production of compound (IX))

Compound (IX) of the formula: 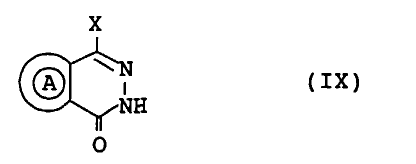 wherein the symbols are defined as above, can be obtained by subjecting compound (X) to Suzuki's reaction (cross condensation reaction of arylboric acid and aryl halide, for example, in the presence of palladium catalyst) or Stille's reaction (cross condensation reaction of aryltin compound and aryl halide, for example, in the presence of palladium catalyst), for example.

### (Process 5) (production of compound (II))

Compound (II) of the formula: 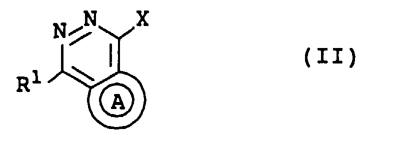 wherein the symbols are defined as above, can be obtained by a reaction from compound (XI) in the same manner as process 4. Specifically, compound (II) can be obtained using an arylboric acid or aryltin compound at about 1 to 1.5 mol per mol of compound (XI).

### (Process 6) (production of compound (II))

This reaction, from compound (IX), is carried out in the same manner as process 3.

Here, compounds (IV), (V), (VI) and (VIII) can be produced by a known method or a method based thereon.

When the benzene ring or nitrogen-containing 6-membered aromatic ring represented by ring A has an acyl group derived from a carboxylic acid as a substituent in all the production methods described above, the acyl group can be re-converted to a carbonylacyl group by protecting the carbonyl moiety with a 1,3-dioxolan-2-yl group, for example, by a commonly known method, followed by the reaction, then deprotecting the carbonyl moiety by a commonly known method, e.g., acid hydrolysis in the case of a 1,3-dioxolan-2-yl group.

When the reaction product contains a protecting group for an amino group, a hydroxy group or a carboxyl group, the protecting group can be removed by an appropriately chosen known means such as deprotection with acid, deprotection with base, deprotection with hydrazine, deprotection by reduction or deprotection with sodium N-methyldithiocarbamate.

Here, useful amino group-protecting groups include, for example, lower (C₁₋₆) alkyl-carbonyl groups (e.g., formyl, acetyl, propionyl, butyryl, isobutyryl, valeryl, pivaloyl) and benzoyl.

Useful hydroxy group-protecting groups include, for example, methoxydimethylmethyl, trimethylsilyl, tert-butyldimethylsilyl, trimethylsilylethoxymethyl (SEM), methoxymethyl, benzyloxymethyl and tetrahydropyranyl (THP).

Useful carboxyl group-protecting groups include, for example, lower (C₁₋₆) alkyl groups (e.g., methyl, ethyl, propyl, isopropyl, butyl, isobutyl, sec-butyl, tert-butyl, pentyl, hexyl) and C₇₋₁₂ aralkyl groups (e.g., benzyl, phenethyl, 4-phenylpropyl, 4-phenylbutyl, 1-naphthylmethyl). Also, the carboxyl group may be protected by conversion to a 2-oxazoline ring.

In the above compound (I) or the starting material compound or synthesis intermediates thereof, basic compounds can be converted to salts using acid by a conventional method. The appropriate acid for this reaction is preferably an acid capable of providing a pharmacologically acceptable salt. Examples of such acids include inorganic acids such as hydrochloric acid, hydrobromic acid, phosphoric acid, sulfuric acid, nitric acid and sulfamic acid, and organic acids such as acetic acid, tartaric acid, citric acid, fumaric acid, maleic acid, p-toluenesulfonic acid, methanesulfonic acid and glutamic acid. When the compound obtained is a salt, it may be converted to a free base by a conventional method.

Also, in the compound (I) above or the starting material compound or synthesis intermediates thereof, those compounds having an acidic group such as -COOH, -SO₂H or -SO₃H can be converted to salts by a conventional method. Preferable examples of such salts include salts with alkali metals, alkaline earth metals, ammonium, substitutional ammonium etc., more specifically exemplified by salts with sodium, potassium, lithium, calcium, magnesium, aluminum, zinc, ammonium, tri-C₁₋₆ alkylammoniums (e.g., trimethylammonium, triethylammonium), triethanolammonium etc.

Unless otherwise stated, the above reactions are each carried out using the starting material normally in an equimolar amount, reaction time being normally about 1 to 24 hours.

The thus-obtained compound (I) or starting material compound thereof may be isolated from the reaction mixture by ordinary means of separation and purification such as extraction, concentration, neutralization, filtration, crystallization, recrystallization and column (or thin-layer) chromatography.

Compound (I) of the present invention exhibits excellent activity for inhibiting Na-H exchange and cell disorder ameriolating activity/cell-protecting activity based thereon (especially on the myocardium) in animals, especially in mammals (e.g., humans, monkeys, dogs, cats, rabbits, guinea pigs, rats, mice) and are useful as a preventing and treating agent for ischemic diseases (e.g., myocardial infarction and accompanying dysfunctions, arrythmia, unstable angina pectoris, reocclusion after PTCA), cardiac hypertrophy, hypertension and accompanying tissue disorders, etc. (preferably prophylactic/therapeutic agent for ischemic cardiovascular diseases such as myocardial infarction and arrythmia).

Because compound (I), used as the active ingredient in the present invention, is of low toxicity, well absorbable even in oral administration, and excellent in stability, it can be orally or non-orally safely used as such, or as pharmaceutical compositions such as powders, granules, tablets, capsules (including soft capsules and microcapsules), liquid preparations, injectable preparations and suppositories in combination with an appropriate pharmaceutically acceptable carrier, excipient and diluent, when used as a pharmaceutical as described above.

Compositions for pharmaceuticals can be prepared as pharmaceutical preparations by ordinary methods. The content ratio of compound (I) in the pharmaceutical composition of the present invention is about 0.01 to about 20% (w/w).

In the present specification, "non-oral" includes subcutaneous injection, intravenous injection, intramuscular injection, intraperitoneal injection and drip infusion. Injectable preparations, e.g., aqueous or oily suspensions for aseptic injection, can be prepared by methods known in relevant fields, using an appropriate dispersing agent or wetting agent and a suspending agent. The aseptic injectable preparation thus obtained may be an aseptically injectable solution or suspension in a diluent or solvent which permits non-toxic non-oral administration, such as an aqueous solution. Acceptable vehicles or solvents include water, Ringer's solution and isotonic saline. It is also possible to use aseptic non-volatile oils in common use as solvents or suspending media. For this purpose, any non-volatile oil or fatty acid can be used, including natural, synthetic or semi-synthetic fatty oils or acids, and natural, synthetic or semi-synthetic mono- or di- or tri-glycerides.

Suppositories for rectal administration may be produced as a mixture of the drug and an appropriate non-irritative shaping agent, such as cacao butter or polyethylene glycol, which is solid at normal temperatures and which is liquid at intestinal temperatures and melts and releases the drug in the rectum.

Solid dosage forms for oral administration include the above-mentioned forms such as powders, granules, tablets, pills and capsules. In these dosage forms, the active ingredient compound may be mixed with at least one additive such as sucrose, lactose, cellulose sugar, mannitol, maltitol, dextran, starch, agar, alginate, chitin, chitosan, pectin, gum tragacanth, gum arabic, gelatin, collagen, casein, albumin, synthetic or semi-synthetic polymer or glyceride. Such dosage forms may contain additional additives as usual, including inert diluents, lubricants such as magnesium stearate, preservatives such as paraben and sorbic acid, antioxidants such as ascorbic acid, α-tocopherol and cysteine, disintegrants, binders, thickening agents, buffers, sweeteners, flavoring agents and perfumes. Tablets and pills may be produced with enteric coating. Liquid dosage forms for oral administration include pharmaceutically acceptable emulsions, syrups, elixirs, suspensions and solutions, which may contain inert diluents, such as water, in common use in relevant fields.

Although varying depending on the subject of administration, route of administration and symptoms, the dose is normally about 0.001 to 10 mg/kg, preferably 0.001 to 1.0 mg/kg, and more preferably about 0.01 to 0.2 mg/kg, (about 0.06 to 600 mg/man, preferably 0.6 to 60 mg/man, and more preferably 0.6 to 12 mg/man) based on compound (I), per administration in the case of oral administration in a patient (adult weighing about 60 kg) with myocardial infarction or arrythmia. It is desirable that such dosage be given about 1 to 3 times per day, depending on symptoms. In acute onset of disease, e.g., just after onset of myocardial infarction, higher doses and especially higher dosing frequencies, e.g., 4 administrations per day, may be necessary. In the case of a patient with myocardial infarction under intensive care treatment, in particular, 100 mg/patient per day may be necessary for intravenous administration.

The present invention is hereinafter described in more detail by means of the following reference examples of starting material production and working examples of compound (I) production, which are not to be construed as limitative. In the reference examples and working examples below, "room temperature" means 0 to 30°C and the abbreviations used are defined as follows:
- mp: : Melting point
- s: : Singlet
- d: : Doublet
- t: : Triplet
- dd: : Double doublet
- m: : Multiplet
- CDCl₃: : Heavy chloroform
- DMSO: : Dimethyl sulfoxide
- DMF: : Dimethylformamide
- Pd-C: : Palladium carbon

### Examples

### Reference Example 1

To 4-(4-methylphenyl)-1-(2H)phthalazinone (mp 255-256°C; 1.89 g), phosphorus oxychloride (6.2 g) was added, followed by stirring at 120 to 125°C for 4 hours. The reaction mixture was concentrated under reduced pressure. To the residue, ethyl acetate (150 ml) and cold water (50 ml) were added, and while the mixture was stirred at room temperature, excess sodium hydrogen carbonate was added, followed by stirring for 30 minutes. The ethyl acetate layer was washed with water and dried (anhydrous sodium sulfate), after which it was concentrated under reduced pressure. The crystal precipitated was collected by filtration and recrystallized from ethyl acetate to yield 1-chloro-4-(4-methylphenyl)phthalazine (1.58 g) as a colorless prismatic crystal.
- mp: : 157-158°C
- IR (Nujol): : 1665, 1610, 1515, 1385, 1290 (cm⁻¹)
- ¹H-NMR (CDCl₃): : δ 2.48 (3H, s), 7.39 (2H, d), 7.63 (2H, d), 7.88-8.13 (3H, m), 8.38 (1H, dd)

### Reference Example 2

To 3-(4-methylbenzoyl)pyridine-2-carboxylic acid (3.28 g) and hydrazine hydrate (910 mg), ethanol (40 ml) was added, followed by refluxing under heating for 20 hours. The reaction mixture was concentrated under reduced pressure; the residue was recrystallized from methanol to yield 5-(4-methylphenyl)-(7H)pyrido[2,3-d]pyridazin-8-one (2.81 g) as a colorless powdery crystal.
- IR (Nujol): : 3170, 3120, 1695, 1670 (cm⁻¹)
- ¹H-NMR (DMSO-d₆): : δ 2.42 (3H, s), 7.37 (2H, d), 7.49 (2H, d), 7.87 (1H, dd), 8.11 (1H, dd), 9.10 (1H, dd)

### Reference Example 3

To 5-(4-methylphenyl)-(7H)pyrido[2,3-d]pyridazin-8-one (2.37 g) and phosphorus oxychloride (12.2 g) was added DMF (7 drops), followed by stirring at 120 to 125°C for 4.5 hours. The reaction mixture was concentrated under reduced pressure. To the residue were added ethyl acetate (150 ml) and cold water (50 ml) and while the mixture was stirred at room temperature, excess sodium hydrogen carbonate was added, followed by stirring for 1 hour. The ethyl acetate layer was washed with water and dried (anhydrous sodium sulfate), after which it was concentrated under reduced pressure. The residue was subjected to silica gel column chromatography and purified by elution with ethyl acetate/hexane to yield 8-chloro-5-(4-methylphenyl)pyrido[2,3-d]pyridazine (2.40 g).
- IR (Nujol): : 1355, 1305, 1005 (cm⁻¹)
- ¹H-NMR (CDCl₃): : δ 2.49 (3H, s), 7.41 (2H, d), 7.63 (2H, d), 7.87 (1H, dd), 8.47 (1H, dd), 9.36 (1H, dd)

### Reference Example 4

Fifty grams of 3-nitrophthalic anhydride was dispersed in methanol (200 ml). While this dispersion was stirred in an ice bath, pyridine (23 ml) was added, followed by stirring under the same conditions for 1 hour. The reaction mixture was concentrated under reduced pressure. The residue was dissolved by the addition of water (500 ml) and acidified with hydrochloric acid. The crystal precipitated was collected by filtration and washed with water, after which it was dried under reduced pressure at 90 to 100°C for 4 hours to yield 2-methoxycarbonyl-3-nitrobenzoic acid (50.1 g) as a colorless powdery crystal.
- IR (Nujol): : 1730, 1690, 1535, 1350, 1280 (cm⁻¹)
- ¹H-NMR (CDCl₃/DMSO-d₆): : δ 3.93 (3H, s), 7.70 (1H, dd), 8.30 (2H, d)

### Reference Example 5

2-Methoxycarbonyl-3-nitrobenzoic acid (33.8 g) was dispersed in benzene (70 ml). Thionyl chloride (23 ml) was added, followed by refluxing under heating for 2 hours. The reaction mixture was concentrated under reduced pressure to yield methyl 2-chloroformyl-6-nitrobenzoate (36.5 g) as a colorless needle crystal.
- IR (Nujol): : 1740, 1535, 1290, 980 (cm⁻¹)
- ¹H-NMR (CDCl₃): : δ 4.03 (3H, s), 7.80 (1H, t), 8.53 (2H, t)

### Reference Example 6

Methyl 2-chloroformyl-6-nitrobenzoate (33 g) was dissolved in benzene (110 ml). While this solution was stirred at room temperature, anhydrous aluminum chloride (45.2 g) was added, followed by stirring under the same conditions for 3 hours. After the reaction mixture was poured into ice-water (500 g), benzene (200 ml) was added, and the insoluble substances were filtered off. The upper layer was washed with water and concentrated under reduced pressure. To the residue was added methanol (150 ml). The crystal precipitated was collected by filtration, washed with a small amount of methanol and dried to yield methyl 2-benzoyl-6-nitrobenzoate (31.8 g) as a yellow prismatic crystal.
- IR (Nujol): : 1740, 1665, 1535, 1280 (cm⁻¹)
- ¹H-NMR (CDCl₃): : δ 3.70 (3H, s), 7.27-7.83 (7H, m), 8.17 (1H, dd)

### Reference Example 7

To methyl 2-benzoyl-6-nitrobenzoate (6.25 g) in a mixed solvent consisting of methanol (50 ml) and benzene (30 ml) was added 50% hydrated 10% Pd-C (5 g), followed by stirring under normal pressure in a hydrogen stream. When hydrogen absorption stopped, the catalyst was filtered off, and the filtrate was concentrated under reduced pressure. The residue was partitioned with aqueous potassium carbonate and ethyl acetate, and the upper layer was washed with water and concentrated under reduced pressure. To the residue was added methanol (30 ml) and the crystal precipitated was collected by filtration. The crystal was washed with a small amount of methanol and dried to yield methyl 6-amino-2-benzoylbenzoate (4.3 g) as a colorless prismatic crystal.
- IR (Nujol): : 3470, 3360, 1700, 1660, 1600, 1260 (cm⁻¹)
- ¹H-NMR (CDCl₃): : δ 3.40 (3H, s), 5.77 (2H, broad), 6.53-6.87 (2H, m), 7.27-7.57 (4H, m), 7.67-7.87 (2H, m)

### Reference Example 8

Methyl 6-amino-2-benzoylbenzoate (5.6 g) was dissolved in acetone (60 ml), and concentrated hydrochloric acid (12 ml) and water (10 ml) were added. While this mixture was stirred in an ice bath, a solution of sodium nitrite (1.65 g) in water (10 ml) was added dropwise, followed by stirring under the same conditions for 0.5 hours. While the reaction mixture was stirred at -7 to -5°C, a solution of potassium iodide (4.5 g) in water (15 ml) was added dropwise, followed by stirring under the same conditions for 0.5 hours. The reaction mixture was concentrated under reduced pressure and the residue was extracted with ether. The ether solution was dried (anhydrous magnesium sulfate) and concentrated under reduced pressure to yield methyl 2-benzoyl-6-iodobenzoic acid (7.4 g).
- IR (Neat): : 1735, 1665, 1450, 1270 (cm⁻¹)
- ¹H-NMR (CDCl₃): : δ 3.67 (3H, s), 7.03-8.10 (8H, m)

### Reference Example 9

Methyl 2-benzoyl-6-iodobenzoate (7.5 g) and 80% hydrazine hydrate (2.3 g) were refluxed under heating in ethanol (30 ml) for 6 hours. After the mixture was cooled in air, the crystal precipitated was collected by filtration, washed with ethanol and dried to yield 8-iodo-4-phenyl-(2H)-phthalazin-1-one (5.4 g) as a light-yellow prismatic crystal.
- mp: : 268-270°C (EtOH)
- IR (Nujol): : 3170, 1650, 1305 (cm⁻¹)
- ¹H-NMR (DMSO-d₆): : δ 7.45-7.64 (7H, m), 8.44 (1H, d)

### Reference Example 10

To 8-iodo-4-phenyl-(2H)-phthalazin-1-one (3.1 g) was added phosphorus oxychloride (7.65 g), followed by stirring at 125 to 130°C for 4 hours. The reaction mixture was concentrated under reduced pressure. To the residue, were added ethyl acetate (150 ml) and cold water (50 ml) and excess sodium hydrogen carbonate was added, followed by stirring at room temperature for 1 hour. The ethyl acetate layer was washed with water and dried (anhydrous sodium sulfate), after which it was concentrated under reduced pressure. The residue was subjected to silica gel chromatography and purified by elution with hexane/ethyl acetate to yield 1-chloro-8-iodo-4-phenylphthalazine (0.96 g) as a colorless prismatic crystal.
- mp: : 173-174°C
- IR (Nujol): : 1625, 1565, 1530, 1380, 1360 (cm⁻¹)
- ¹H-NMR (CDCl₃): : δ 7.45 (1H, t), 7.55-7.70 (5H, m), 8.05 (1H, dd), 8.75 (1H, dd)

### Reference Example 11

A mixture of 1,4-dichlorophthalazine (398 mg), 4-methoxyphenyl boric acid (m.p. 207-208 °C; 304 mg) and Pd(pddb)Cl₂ (40 mg) was stirred in a mixture of toluene (5 ml) and 2M sodium carbonate (5 ml) for 14 hours at 70 °C under argon atmosphere. The reaction mixture was cooled in air, to which were then added ethyl acetate (50 ml) and water (10 ml). The mixture was shaken and left standing to form two layers. The upper layer was washed with water, which was concentrated under reduced pressure. The concentrate was purified by means of a silica gel chromatography, eluting with ethyl acetate/hexane, to give 1-chloro-4-(4-methoxyphenyl)phthalazine (242 mg).
- mp: : 148-149°C (EtOAc)
- IR (Nujol): : 1605, 1510, 1450, 1380, 1350, 1280, 1250 (cm⁻¹)
- ¹H-NMR(CDCl₃): : δ 3.92 (3H, s), 7.11 (2H, d), 7.70 (2H, d), 7.90-8.05 (2H, m), 8.14 (1H, d), 8.39 (1H, d)

### Reference Example 12

A mixture of 1,4-dichlorophthalazine (398 mg), 2-methoxyphenyl boric acid (m.p. 103-104 °C; 304 mg) and Pd(pddb)Cl₂ (40 mg) was stirred in a mixture of toluene (5 ml) and 2M sodium carbonate (5 ml) for 21 hours at 70 °C under argon atmosphere. The reaction mixture was cooled in air, to which were then added ethyl acetate (50 ml) and water (10 ml). The mixture was shaken and left standing to form two layers. The upper layer was washed with water, which was concentrated under reduced pressure. The concentrate was purified by means of a silica gel chromatography, eluting with ethyl acetate/hexane, to give 1-chloro-4-(2-methoxyphenyl)phthalazine (235 mg).
- mp: : 157-158°C (EtOAc)
- IR (Nujol): : 1600, 1380, 1285, 1245 (cm⁻¹)
- ¹H-NMR(CDCl₃): : δ 3.70 (3H, s), 7.08 (1H, d), 7.16 (1H, t), 7.42-7.59 (2H, m), 7.68 (1H, d), 7.86 (1H, t), 7.98 (1H, t), 8.36 (1H, d)

### Reference Example 13

A mixture of 1,4-dichlorophthalazine (398 mg), 2-methylphenyl boric acid (m.p.165-166°C; 272 mg) and Pd(pddb)Cl₂ (40 mg) was stirred in a mixture of toluene (5 ml) and 2M sodium carbonate (5 ml) for 23 hours at 70 °C under argon atmosphere. The reaction mixture was cooled in air, to which were then added ethyl acetate (50 ml) and water (50 ml). The mixture was shaken and left standing to form two layers. The upper layer was washed with water, which was concentrated under reduced pressure. The concentrate was purified by means of a silica gel chromatography, eluting with ethyl acetate/hexane, to give 1-chloro-4-(2-methylphenyl)phthalazine (120 mg).
- mp: : 126-127°C (EtOAc)
- IR (Nujol): : 1600, 1515, 1450, 1380, 1290 (cm⁻¹)
- ¹H-NMR(CDCl₃): : δ 2.12 (3H,s ), 7.32-7.51 (4H, m), 7.65 (1H, d), 7.88 (1H, t), 8.01 (1H, t), 8.40 (1H, d)

### Reference Example 14

A mixture of 1,4-dichlorophthalazine (398 mg), 3-nitrophenyl boric acid (334 mg) and Pd(pddb)Cl₂ (40 mg) was stirred in a mixture of toluene (5 ml) and 2M sodium carbonate (5 ml) for 15 hours at 70 °C under argon atmosphere. The reaction mixture was cooled in air. The resulting precipitate was collected by filtration, which was washed with water and ethyl acetate successively to give 1-chloro-4-(3-nitrophenyl)phthalazine (230 mg).
- mp: : 235-237°C
- IR (Nujol): : 1540, 1380, 1345, 1295 (cm⁻¹)
- ¹H-NMR(CDCl₃): : δ 7.94 (1H, m), 8.00-8.29 (4H, m), 8.42-8.60 (3H, m)

### Reference Example 15

To a mixed solvent consisting of benzene (100 ml) and nitroethane (20 ml) were added 4-nitrophthalic anhydride (9.65 g) and anhydrous aluminum chloride (17.3 g). The mixture was stirred for 2.5 hours at 75 °C. The reaction mixture was poured into ice (300 g), to which was added ethyl acetate (300 ml). The mixture was shaken and left standing to form two layers. The upper layer was washed with water and concentrated under reduced pressure. The concentrate was dissolved in ethanol (80 ml), to which was added hydrazine hydrate (2.7 g). The mixture was heated for 2 hours under reflux, to which was further added acetic acid (3 ml). The mixture was heated for 13 hours under reflux. The resulting crystalline precipitate was collected by filtration, which was washed with an aqueous solution of sodium hydrogencarbonate and water successively, followed by drying to give yellow powdery crystals (3.65 g).

To this powdery product (1.87 g) was added phosphorus oxychloride (20.5 g). The mixture was stirred for one hour at temperatures ranging from 120 to 125 °C. The reaction mixture was concentrated under reduced pressure. To the concentrate were added ethyl acetate (70 ml) and cold water (30 ml). To the mixture was added an excess amount of sodium hydrogencarbonate while stirring at room temperature. The whole mixture was stirred for one hour. The resulting crystalline product was collected by filtration and washed with water, which was then washed with ethyl acetate and dried to give 1-chloro-7-nitro-4-phenylphthalazine (1.0 g) as pale yellow prisms.
- mp: : 193-194°C
- IR (Nujol): : 1530, 1350, 1340 (cm⁻¹)
- ¹H-NMR(CDCl₃): : δ 7.62-7.67 (3H, m), 7.72-7.76 (2H, m), 8.31 (1H, d), 8.68 (1H, dd), 9.26 (1H, d)

### Reference Example 16

A mixture of methyl 2-benzoyl-6-nitrobenzoate (4.65 g) and hydrazine hydrate (750 mg) was heated for 5 hours under reflux in ethanol (25 ml). The reaction mixture was cooled in air. The resulting crystalline product was collected by filtration, washed with ethanol and dried to give 8-nitro-4-phenyl-(2H)phthalazin-1-one (3.15 g) as yellow prisms.
- mp: : 236-237°C (dec.)
- IR (Nujol): : 3160, 1665, 1535 (cm⁻¹)
- ¹H-NMR(DMSO-d₆): : δ 7.60 (5H, s), 7.87 (1H, d), 8.07 (1H, t), 8.19 (1H, d)

### Reference Example 17

To 8-nitro-4-phenyl-(2H)phthalazin-1-one (1.70 g) were added phosphorus oxychloride (7.65 g) and dimethyl aniline (1.6 g). The mixture was stirred for 2.5 hours at 110 °C. The reaction mixture was concentrated under reduced pressure. To the concentrate were added ethyl acetate (150 ml), tetrahydrofuran (50 ml), cold water (50 ml) and an excess amount of sodium hydrogencarbonate. The mixture was stirred for one hour at room temperature. The upper layer was washed with water, dried (anhydrous sodium sulfate) and then concentrated under reduced pressure. The concentrate was washed with hexane, which was recrystallized from tetrahydrofuran to yield 1-chloro-8-nitro-4-phenylphthalazine (0.90 g).
- mp: : 167-168°C
- IR (Nujol): : 1540, 1365, 1335, 1290 (cm⁻¹)
- ¹H-NMR(DMSO-d₆): : δ 7.61-7.73 (5H, m), 8.00 (1H, t), 8.10 (1H, dd), 8.30 (1H, dd)

### Reference Example 18

Methyl 6-amino-2-benzoylbenzoate (3.06 g) was dissolved in acetone (30 ml), to which was added a 47% aqueous solution of hydrogen bromide (14.5 g). To the mixture was added dropwise, under ice-cooling, a solution of sodium nitrite (917 mg) in water (5.5 ml). The mixture was stirred for 0.5 hour under the same conditions. To the reaction mixture was added water (7 ml), to which was added cuprous oxide (0.1 g) little by little while stirring at -15 °C, followed by stirring for 0.5 hour under the same conditions. The reaction mixture was warmed up to room temperature, to which was added water (20 ml). The mixture was concentrated under reduced pressure. The concentrate was subjected to extraction with ethyl acetate. The extract was washed with water, dried (anhydrous magnesium sulfate) and concentrated under reduced pressure to leave a yellowish orange oily product. This oily product was dissolved in ethanol (35 ml), to which was added hydrazine hydrate (600 mg), and the mixture was heated for 13 hours under reflux. The reaction mixture was cooled in air, and the resulting crystalline precipitate was collected by filtration, washed with ethanol and dried to yield 8-bromo-4-phenyl-(2H)phthalazin-1-one (1.47 g) as yellow prisms.
- mp: : 267-268°C
- IR (Nujol): : 3160, 3080, 1660 (cm⁻¹)
- ¹H-NMR(DMSO-d₆): : δ 7.54(5H,s), 7.60(1H,d), 7.71(1H,t), 8.11(1H,d)

### Reference Example 19

To a solution of methyl 6-amino-2-benzoylbenzoate (3.06 g) in acetone (30 ml) was added conc. hydrochloric acid (7.0 ml). To the mixture was added dropwise, under ice-cooling, a solution of sodium nitrite (917 mg) in water (5.5 ml). The mixture was stirred for 0.5 hour under the same conditions. To the reaction mixture was added cuprous oxide (0.1 g) little by little, while stirring at -15 °C. The whole mixture was stirred for 0.5 hour under the same conditions. The reaction mixture was warmed up to room temperature, to which was added water (20 ml). The mixture was concentrated under reduced pressure. The concentrate was subjected to extraction with ethyl acetate. The extract was washed with water, dried (anhydrous magnesium sulfate) and concentrated under reduced pressure to leave a yellowish orange oily product. This oily product was dissolved in ethanol (15 ml), to which was added hydrazine hydrate (234 mg). The mixture was heated for 4.5 hours under reflux. The reaction mixture was cooled in air, and the resulting crystalline product was collected by filtration, washed with ethanol and dried to yield 8-chloro-4-phenyl-(2H)phthalazin-1-one (0.53 g) as yellow prisms.
- mp: : 238-240 °C
- IR (Nujol): : 3160, 3080, 1660, 1445, 1300 (cm⁻¹)
- ¹H-NMR(DMSO-d₆): : δ 7.54-7.57 (6H, m), 7.79 (1H, t), 7.87 (1H, dd)

### Reference Example 20

To 8-bromo-4-phenyl-(2H)phthalazin-1-one (1.45 g) were added phosphorus oxychloride (9.9 g) and dimethyl aniline (2.42 g). The mixture was stirred for one hour at 115 °C. The reaction mixture was concentrated under reduced pressure. To the concentrate were added ethyl acetate (100 ml) and cold water (50 ml). The mixture was shaken and, then, left standing to form two layers. To the upper layer was added an excess amount of sodium hydrogencarbonate. The mixture was stirred for one hour at room temperature. The upper layer was washed with water, dried (anhydrous sodium sulfate) and, then, concentrated under reduced pressure. The concentrate was recrystallized from ethyl acetate to yield 1-chloro-8-bromo-4-phenylphthalazine (1.21 g) as yellow prisms.
- mp: : 174-175°C
- IR (Nujol): : 1510, 1440, 1360, 1280 (cm⁻¹)
- ¹H-NMR(CDCl₃): : δ 7.56-7.71 (6H, m), 8.04 (1H, dd), 8.31 (1H, dd)

### Reference Example 21

To 8-chloro-4-phenyl-(2H)phthalazin-1-one (523 mg) were added phosphorus oxychloride (4.2 g) and dimethyl aniline (1.0 g). The mixture was stirred for one hour at 115 °C. The reaction mixture was concentrated under reduced pressure. To the concentrate were added ethyl acetate (50 ml) and cold water (30 ml). The mixture was shaken and left standing to form two layers. To the upper layer was added an excess amount of sodium hydrogencarbonate, and the mixture was stirred for one hour at room temperature. The upper layer was washed with water, dried (anhydrous sodium carbonate) and concentrated under reduced pressure. The concentrate was recrystallized from ethyl acetate to give 1,8-dichloro-4-phenylphthalazine (370 mg) as colorless prisms.
- mp: : 183-184°C
- IR (Nujol): : 1515, 1445, 1360, 1280 (cm⁻¹)
- ¹H-NMR(CDCl₃): : δ 7.56-7.68 (5H, m), 7.78 (1H, t), 8.00 (1H, dd), 8.04 (1H, dd)

### Reference Example 22

To a mixture of methyl 2-benzoyl-6-iodobenzoate (1.83 g) and phenylboric acid (976 mg) were added toluene (15 ml) and 2M sodium carbonate (13 ml). To the whole mixture was added, at room temperature under argon atmosphere, Pd(Ph₃P)₄ (173 mg). The reaction mixture was stirred for 4 hours at 110 °C and then cooled in air, to which were added ethyl acetate (50 ml) and water (10 ml). The mixture was shaken and left standing to form two layers. The upper layer was washed with water and concentrated under reduced pressure. The concentrate was dissolved in ethanol (10 ml), to which was added hydrazine hydrate (0.3 ml). The mixture was heated for two hours under reflux. The reaction mixture was cooled in air, and the resulting crystalline precipitate was collected by filtration, which was washed with ethanol and dried to give 4,8-diphenyl-(2H)phthalazin-1-one (900 mg) as colorless prisms.
- mp: : 276-277°C
- IR (Nujol): : 3370, 1670 (cm⁻¹)
- ¹H-NMR(DMSO-d₆): : δ 7.30-7.40 (5H,m), 7.54-7.67 (7H, m), 7.88 (1H, t), 12.56 (1H, s)

### Reference Example 23

To 4,8-diphenyl-(2H)phthalazin-1-one (0.80 g) were added phosphorus oxychloride (4.95 g) and dimethylaniline (0.73 g). The mixture was stirred for one hour at 115 °C. The reaction mixture was concentrated under reduced pressure. To the concentrate were added ethyl acetate (30 ml) and cold water (20 ml). Insolubles were collected by filtration and washed with water and ethyl acetate, successively, followed by drying to give 1-chloro-4,8-diphenylphthalazine (545 mg) as pale yellow prisms.
- mp: : 186-187°C
- IR (Nujol): : 1440, 1360, 1280, 1270 (cm⁻¹)
- ¹H-NMR(CDCl₃): : δ 7.34-7.40 (2H,m), 7.44-7.49 (3H, m), 7.58-7.62 (3H, m), 7.72-7.83 (2H, m), 7.83-7.93 (2H, m), 8.09 (1H, dd)

### Reference Example 24

Dimethyl 4-hydroxyphthalate (4.2 g) was dissolved in acetic acid (30 ml). To the solution was added, at room temperature, bromine (2.1 ml). The mixture was stirred for two hours. To the reaction mixture were added isopropyl ether (350 ml) and water (500 ml), which was shaken and, then, left standing to form two layers. The upper layer was washed with water and concentrated under reduced pressure. The concentrate was washed with benzene and hexane, successively, which was then dried to give dimethyl 3,5-dibromo-4-hydroxyphthalate (8.72 g) as colorless prisms.
- mp: : 113-114°C
- IR (Nujol): : 3300, 1725, 1710, 1540, 1430, 1300, 1270, 1255, 1160 (cm⁻¹)
- ¹H-NMR(CDCl₃): : δ 3.90 (3H, s), 4.00 (3H, s), 6.45 (1H, broad), 8.21 (1H, s)

### Reference Example 25

To a mixture of dimethyl 3,5-dibromo-4-hydroxyphthalate (3.68 g) and potassium carbonate (2.0 g) was added DMF (8 ml). To the whole mixture was added methyl iodide (2.8 g) at room temperature, which was stirred for 12 hours. To the reaction mixture were added ethyl acetate (100 ml) and water (100 ml), which was shaken and then left standing to form two layers. The upper layer was washed with an aqueous solution of sodium hydrogencarbonate and water, successively, which was concentrated under reduced pressure. The concentrate was washed with methanol and dried to give dimethyl 3,5-dibromo-4-methoxyphthalate (3.43 g) as colorless needles.
- mp: : 77-78°C
- IR (Nujol): : 1745, 1725, 1430, 1290, 1270, 1100 (cm⁻¹)
- ¹H-NMR(CDCl₃): : δ 3.90 (3H, s), 3.95 (3H, s), 3.99 (3H, s), 8.22 (1H, s)

### Reference Example 26

To a mixture of dimethyl 3,5-dibromo-4-hydroxyphthalate (3.68 g) and potassium carbonate was added DMF (8 ml). To the whole mixture was added butyl bromide (1.78 g), which was stirred for two hours at 110 °C. The reaction mixture was cooled in air, to which were added ethyl acetate (100 ml) and water (100 ml). The mixture was shaken and, then, left standing to form two layers. The upper layer was washed with an aqueous solution of sodium hydrogencarbonate and water, successively, which was dried (anhydrous magnesium sulfate), followed by concentration under reduced pressure to give dimethyl 3,5-dibromo-4-butoxyphthalate (4.10 g) as a colorless oily product.
- IR (Neat): : 1740, 1725, 1430, 1265, 1250, 1150, 1100 (cm⁻¹)
- ¹H-NMR(CDCl₃): : δ 1.00 (3H, t), 1.52-1.65 (2H, m), 1.82-1.95 (2H, m), 3.90 (3H, s), 3.99 (3H, s), 4.06 (3H, s), 8.22 (1H, s)

### Reference Example 27

Dimethyl 3,5-dibromo-4-methoxyphthalate (3.06 g) was dissolved in methanol/tetrahydrofuran (1:1) (40 ml). To the solution was added, under ice-cooling, 2N sodium hydroxide (4.0 ml). The mixture was stirred for 16 hours at temperatures ranging from ice-cooling to room temperature. The reaction mixture was concentrated under reduced pressure. To the concentrate was added hot water (50 ml) to make a solution. The solution was cooled in air, to which were added 2N hydrochloric acid (4 ml) and ethyl acetate (250 ml). The mixture was shaken and, then, left standing to form two layers. The upper layer was washed with water, dried (anhydrous magnesium sulfate) and concentrated under reduced pressure to give 3,5-dibromo-4-methoxy-2-methoxycarbonyl benzoic acid (2.80 g) as colorless prisms.
- mp: : 174-175°C
- IR (Nujol): : 1745, 1705, 1290 (cm⁻¹)
- ¹H-NMR(CDCl₃): : δ 3.97 (3H, s), 3.98 (3H, s), 6.38 (1H, broad), 8.31 (1H, s)

### Reference Example 28

Dimethyl 3,5-dibromo-4-butoxyphthalate (4.09 g) was dissolved in methanol/tetrahydrofuran (1:1) (120 ml). To the solution was added, under ice-cooling, 2N sodium hydroxide (4.83 ml). The mixture was then stirred for 14 hours at temperatures ranging from ice-cooling to room temperature. The reaction mixture was concentrated under reduced pressure. The concentrate was dissolved in water (70 ml), to which were added 2N hydrochloric acid (6 ml) and ethyl acetate (100 ml). The mixture was shaken and, then, left standing to form two layers. The upper layer was washed with water, dried (anhydrous magnesium sulfate) and concentrated under reduced pressure. The concentrate was washed with isopropyl ether/hexane (3:5) and dried to give. 3,5-dibromo-4-butoxy-2-methoxycarbonyl benzoic acid (3.25 g) as colorless prisms.
- mp: : 161-162°C
- IR (Nujol): : 1750, 1700, 1405, 1350, 1275, 1255, 1005 (cm⁻¹)
- ¹H-NMR(CDCl₃): : δ 1.01 (3H,t), 1.49-1.67 (2H, m), 1.82-1.96 (2H, m), 3.97 (3H, s), 4.09 (2H, t), 5.05 (1H, broad), 8.30 (1H, s)

### Reference Example 29

To a dispersion of 3,5-dibromo-4-methoxy-2-methoxycarbonyl benzoic acid (3.45 g) in benzene (11 ml) were added thionyl chloride (1.43 g) and DMF (three drops). The mixture was heated for two hours under reflux. The reaction mixture was concentrated under reduced pressure. To the concentrate were added benzene (50 ml) and water (15 ml). The mixture was shaken and, then, left standing. The upper layer was washed with water, dried (anhydrous magnesium sulfate) and concentrated under reduced pressure to give methyl 4,6-dibromo-2-chloroformyl-6-methoxybenzoate (3.63 g) as colorless prisms.
- mp: : 105-106°C
- IR (Nujol): : 1745, 1730, 1530, 1360, 1290, 1280, 1255, 1205, 1120 (cm⁻¹)
- ¹H-NMR(CDCl₃): : δ 3.98 (3H, s), 3.99 (3H, s), 8.44 (1H, s)

### Reference Example 30

To a dispersion of 3,5-dibromo-4-butoxy-2-methoxycarbonyl benzoic acid (3.2 g) in benzene (8 ml) were added thionyl chloride (1.43 g) and DMF (three drops). The mixture was heated for one hour under reflux. The reaction mixture was concentrated under reduced pressure. To the concentrate were added benzene (50 ml) and water (15 ml). The mixture was shaken and, then left standing to form two layers. The upper layer was washed with water, dried (anhydrous magnesium sulfate), and concentrated under reduced pressure to give methyl 4,6-dibromo-5-butoxy-2-chloroformylbenzoate (3.39 g) as a colorless oily product.
- IR (Nujol): : 1750, 1745, 1430, 1285, 1255, 1245, 1195 (cm⁻¹)
- ¹H-NMR(CDCl₃): : δ 1.01 (3H, t), 1.49-1.67 (2H, m), 1.83-1.97 (2H, m), 3.98 (3H, s), 4.12 (2H, t), 8.44 (1H, s)

### Reference Example 31

Methyl 4,6-dibromo-5-methoxy-2-chloroformyl benzoate (3.15 g) and tributylphenyl tin (3.12 g) were dissolved in toluene (12 ml). To the solution was added Pd(Ph₃P)₂Cl₂ (100 mg). The mixture was stirred for 15 hours at 60 °C under argon atmosphere. The reaction mixture was cooled in air and, then, concentrated under reduced pressure. The concentrate was purified by means of a silica gel chromatography eluting with ethyl acetate/hexane to give methyl 2-benzoyl-4,6-dibromo-5-methoxybenzoate (1.89 g).
- mp: : 106-107°C
- IR (Nujol): : 1730, 1655, 1445, 1280 (cm⁻¹)
- ¹H-NMR(CDCl₃): : δ 3.74 (3H, t), 3.98 (3H, s), 4.10 (2H, t), 7.46-7.54 (2H, m), 7.60-7.68 (1H, m), 7.73 (1H, s), 7.74-7.79 (2H, m)

### Reference Example 32

To a solution of methyl 4,6-dibromo-5-butoxy-2-chloroformyl benzoate (3.3 g) and tributylphenyltin (3.12 g) in toluene (12 ml) was added Pd(Ph₃P)₂Cl₂ (100 mg). The mixture was stirred for 15 hours at 60 °C under argon atmosphere. The reaction mixture was cooled in air, which was then concentrated under reduced pressure. The concentrate was purified by means of a silica gel chromatography eluting with ethyl acetate/hexane to give methyl 2-benzoyl-4,6-dibromo-5-butoxybenzoate (1.89 g) as a colorless oily product.
- IR (Neat): : 1740, 1655, 1270, 1110, 1020 (cm⁻¹)
- ¹H-NMR(CDCl₃): : δ 1.02 (3H, t), 1.50-1.69 (2H, m), 1.84-1.98 (2H, m), 3.75 (3H, s), 4.10 (2H, t), 7.46-7.54 (2H, m), 7.60-7.68 (1H, m), 7.72 (1H, s), 7.74-7.80 (2H, m)

### Reference Example 33

Methyl 2-benzoyl-4,6-dibromo-5-methoxybenzoate (1.38 g) and hydrazine hydrate (1.65 mg) were heated in ethanol for 14 hours under reflux. The reaction mixture was cooled in air. The resulting crystalline precipitate was collected by filtration, which was washed with a small volume of ethanol and, then, dried to give 6,8-dibromo-7-methoxy-4-phenyl-(2H)phthalazin-1-one (1.19 g) as colorless prisms.
- mp: : 235-236°C
- IR (Nujol): : 1670, 1340 (cm⁻¹)
- ¹H-NMR(DMSO-d₆): : δ 3.91 (3H, s), 7.56 (5H, s), 7.72 (1H, s)

### Reference Example 34

Methyl 2-benzoyl-4,6-dibromo-5-butoxybenzoate (1.00 g) and hydrazine hydrate (107 mg) were heated in ethanol (10 ml) for 20 hours under reflux. The resulting crystalline precipitate was collected by filtration, which was washed with a small volume of ethanol and, then, dried to give 6,8-dibromo-7-butoxy-4-phenyl-(2H)phthalazin-1-one (770 mg) as colorless prisms.
- mp: : 270-271°C
- IR (Nujol): : 1665, 1340 (cm⁻¹)
- ¹H-NMR(DMSO-d₆): : δ 1.03 (3H, t), 1.50-1.70 (2H, m), 1.87-2.00 (2H, m), 4.11(2H,t), 7.48-7.58 (5H, m), 7.87 (1H, s), 9.96 (1H, broad)

### Reference Example 35

Phosphorus oxychloride (2.22 g) and dimethylaniline (1.21 g) were added to 6,8-dibromo-7-methoxy-4-phenyl-(2H)phthalazin-1-one (1.19 g). The mixture was stirred for one hour at 110 °C. The reaction mixture was concentrated under reduced pressure. To the concentrate were added ethyl acetate (50 ml), cold water (20 ml) and an excess amount of sodium hydrogencarbonate. The mixture was stirred for one hour at room temperature. The upper layer was washed with water and dried (anhydrous sodium sulfate), which was then concentrated under reduced pressure. The concentrate was washed with hexane to give 6,8-dibromo-1-chloro-7-methoxy-4-phenylphthalazine (1.04 g).
- mp: : 156-157°C (dec.)
- IR (Nujol): : 1665, 1440, 1355, 1290 (cm⁻¹)
- ¹H-NMR(CDCl₃): : δ 4.05 (3H, s), 7.57-7.66 (5H, m), 8.25 (1H, s)

### Reference Example 36

Phosphorus oxychloride (1.30 g) and dimethylaniline (605 mg) were added to 6,8-dibromo-7-butoxy-4-phenyl-(2H)phthalazin-1-one (760 mg). The mixture was stirred for 2.5 hours at 110 °C. The reaction mixture was concentrated under reduced pressure. To the concentrate were added ethyl acetate (50 ml), cold water (20 ml) and an excess amount of sodium hydrogencarbonate. The mixture was stirred for one hour at room temperature. The upper layer was washed with water, dried (anhydrous sodium sulfate) and, then, concentrated under reduced pressure. The concentrate was recrystallized from hexane to give 6,8-dibromo-7-butoxy-1-chloro-4-phenylphthalazine (686 mg).
- mp: : 117-118°C
- IR (Nujol): : 1665, 1450, 1445, 1360, 1290 (cm⁻¹)
- ¹H-NMR(CDCl₃): : δ 1.04 (3H, t), 1.55-1.72 (2H, m), 1.90-2.03 (2H, m), 4.16 (2H, t), 7.58-7.67 (5H, m), 8.25 (1H, s)

### Reference Example 37

To a mixture of methyl 2-benzoyl-4,6-dibromo-5-butoxybenzoate (880 mg) and 2-(methylphenyl)boric acid (274 mg) were added toluene (7 ml) and 2M sodium carbonate (5 ml). To the whole mixture was added Pd(Ph₃P)₄ (50 mg) at room temperature under argon atmosphere. The reaction mixture was stirred for 6.5 hours at 95 °C, which was cooled in air. To the reaction mixture were added hexane (20 ml), ethyl acetate (5 ml) and water (10 ml). The mixture was shaken and, then, left standing to form two layers. The upper layer was washed with water and concentrated under reduced pressure. The concentrate was purified by means of a silica gel column chromatography, eluting with ethyl acetate/hexane, to give methyl 2-benzoyl-6-bromo-5-butoxy-4-(2-methylphenyl)benzoate (600 mg) as a colorless oily product.
- IR (Neat): : 1740, 1665, 1445, 1430, 1305, 1270 (cm⁻¹)
- ¹H-NMR(CDCl₃): : δ 0.72 (3H, t), 1.08-1.22 (2H, m), 1.35-1.50 (2H, m), 2.16 (3H, s), 3.50 (1H, broad), 3.62 (1H, broad), 3.86 (3H, s), 7.15-7.31 (4H, m), 7.38 (1H, s), 7.42-7.50 (2H, m), 7.54-7.62 (1H, m), 7.77-7.82 (2H, m)

### Reference Example 38

Methyl 2-benzoyl-6-bromo-5-butoxy-4-(2-methylphenyl)benzoate (570 mg) and hydrazine hydrate (60 mg) were heated for 19 hours in ethanol (4 ml) under reflux. The reaction mixture was cooled in air, and, then, resulting crystalline precipitate was collected by filtration, which was washed with a small volume of ethanol and dried to give 8-bromo-7-butoxy-6-(2-methylphenyl)-4-phenyl-(2H)phthalazin-1-one (434 mg) as colorless needles.
- mp: : 219-220°C
- IR (Nujol): : 1660, 1435, 1340 (cm⁻¹)
- ¹H-NMR(DMSO-d₆): : δ 0.70 (3H, t), 1.26 (2H, broad), 1.42 (2H, broad), 2.16 (3H, s), 3.49 (1H, broad), 3.67 (1H, broad), 7.14-7.33 (4H, m), 7.45-7.55 (6H, m), 9.90 (1H, s)

### Reference Example 39

Phosphorus oxychloride (765 mg) and dimethylaniline (363 mg) were added 8-bromo-7-butoxy-6-(2-methylphenyl-(2H)phthalazin-1-one (424 mg). The mixture was stirred for 2 hours at 110 °C. The reaction mixture was concentrated under reduced pressure. To the concentrate were added ethyl acetate (30 ml) and cold water (20 ml), which was shaken and, then, left standing to form two layers. The upper layer was washed with water, dried (anhydrous sodium sulfate) and concentrated under reduced pressure to give 8-bromo-7-butoxy-1-chloro-6-(2-methylphenyl)-4-phenylphthalazine (443 mg) as a yellow oily product.
- IR (Neat): : 1440, 1370, 1350, 1300, 1160 (cm⁻¹)
- ¹H-NMR(CDCl₃): : δ 0.72 (3H, t), 1.05-1.50 (4H, m), 2.13 (3H, s), 3.52 (1H, broad), 3.62 (1H, broad), 7.16-7.35 (4H, m), 7.51-7.55 (3H, m), 7.61-7.67 (2H, m), 7.85 (1H, s)

### Reference Example 40

To a mixture of methyl 2-benzoyl-6-iodobenzoate (3.03 g), sodium trifluoroacetate (4.52 g) and cuprous iodide (5.06 g) were added DMF (20 ml) and toluene (20 ml). The mixture was subjected to azeotropic distillation for 1.5 hour at 150 °C to remove moisture in the reaction system. Then, toluene was distilled off, and the remainder was heated for 6 hours at 165 °C under reflux. The reaction mixture was cooled in air, to which were added ethyl acetate (200 ml) and water (200 ml). Insolubles were filtered off through celite. The filtrate was left standing to form two layers. The upper layer was washed with water, dried (anhydrous magnesium sulfate) and concentrated under reduced pressure to give methyl 2-benzoyl-6-trifluoromethyl benzoate (2.14 g) as a pale yellow oily product.
- IR (Neat): : 1735, 1665, 1270, 1135 (cm⁻¹)
- ¹H-NMR(CDCl₃): : δ 3.72 (3H, s), 7.39-7.92 (8H, m)

### Reference Example 41

A mixture of methyl 2-benzoyl-6-trifluoromethyl benzoate (2.13 g) and hydrazine hydrate (250 mg) was heated for 16 hours in ethanol (8 ml) under reflux. The reaction mixture was cooled in air. The resulting crystalline precipitate was collected by filtration, which was washed with a small volume of ethanol, and, then dried to give 8-trifluoromethyl-4-phenyl-(2H)phthalazin-1-one (1.00 g) as yellow prisms.
- mp: : 229-230°C
- IR (Nujol): : 1670, 1300, 1165, 1150 (cm⁻¹)
- ¹H-NMR(DMSO-d₆): : δ 7.57 (5H, s), 7.92 (1H, d), 8.04 (1H, t), 8.30 (1H, d)

### Reference Example 42

To 8-trifluoromethyl-4-phenyl-(2H)phthalazin-1-one (1.00 g) were added phosphorus oxychloride (2.75 g) and dimethylaniline (1.33 g). The mixture was stirred for 4.5 hours at 110 °C. The reaction mixture was concentrated under reduced pressure. To the concentrate were added ethyl acetate (30 ml), benzene (20 ml) and cold water (20 ml). The mixture was shaken and, then, left standing to form two layers. The upper layer was washed with water, dried (anhydrous sodium sulfate) and, then, concentrated under reduced pressure. The concentrate was washed with hexane, which was eluted with ethyl acetate (30 ml). The eluate was concentrated under reduced pressure. To the concentrate was added 1,2-dimethoxyethane (4 ml). The resulting crystals were collected by filtration and washed with a small volume of 1,2-dimethoxyethane to give 1-chloro-8-trifluoromethyl-4-phenylphthalazine (420 mg).
- mp: : 149-150°C
- IR (Nujol): : 1300, 1145 (cm⁻¹)
- ¹H-NMR(CDCl₃): : δ 7.55-7.70 (5H, m), 7.97 (1H, t), 8.32 (1H, d), 8.46 (1H, d)

### Reference Example 43

DMF (15 ml) was added to a mixture of methyl 2-benzoyl-6-iodobenzoate (3.91 g), methyl mercaptan sodium (1.75 g) and powder copper (0.32 g), which was stirred for 3 hours at 80 °C. The reaction mixture was heated under reflux to remove the moisture in the reaction system. Then, toluene was distilled off, and the remainder was heated under reflux for 6 hours at 165 °C. The reaction mixture was cooled in air, to which were added ethyl acetate (200 ml), hexane (50 ml) and water (300 ml). The resulting insolubles were filtered off through celite. The filtrate was made into two layers, and the aqueous layer was made acid with 2N hydrochloric acid, followed by extraction with ethyl acetate. The ethyl acetate layer was washed with water, dried (anhydrous magnesium sulfate) and concentrated under reduced pressure. The concentrate was recrystallized from ethyl acetate to give 2-benzoyl-6-methylthiobenzoic acid (1.29 g) as a pale yellow powdery product.
- mp: : 173-174°C
- IR (Nujol): : 3400, 1730, 1450, 1230 (cm⁻¹)
- ¹H-NMR(CDCl₃): : δ 2.53 (3H,s), 7.17-7.60 (8H, m)

### Reference Example 44

Methyl iodide (1.42 g) was added to a suspension of 2-benzoyl-6-methylthiobenzoic acid (1.25 g) and potassium carbonate (1.38 g) in DMF (3 ml). The mixture was stirred for 2.5 hours at room temperature, to which were added ethyl acetate (50 ml), hexane (20 ml) and water (50 ml). The whole mixture was shaken and, then, left standing to form two layers. The upper layer was washed with water, dried (anhydrous magnesium sulfate) and, then, concentrated under reduced pressure to give methyl 2-benzoyl-6-methylthiobenzoate (1.25 g) as an oily product. This oily product, together with hydrazine hydrate (225 mg), was heated for 14 hours in ethanol (5 ml) under reflux. The reaction mixture was cooled in air. The resulting crystalline precipitate was collected by filtration, which was washed with a small volume of ethanol and, then, dried to give 8-methylthio-4-phenyl-(2H)phthalazin-1-one (948 mg) as pale yellow powdery crystals.
- mp: : 280-281°C
- IR (Nujol): : 3160, 1660, 1300 (cm⁻¹)
- ¹H-NMR(CDCl₃): : δ 2.51 (3H, s), 7.38 (1H, dd), 7.46-7.57 (6H, m), 7.66 (1H, t), 9.94 (1H, broad)

### Reference Example 45

Phosphorus oxychloride (1.3 g) and dimethylaniline (605 mg) were added to 8-methylthio-4-phenyl-(2H)phthalazin-1-one (450 mg). The mixture was stirred for 3.5 hours at 110 °C. The reaction mixture was concentrated under reduced pressure. To the concentrate were added ethyl acetate (30 ml) and cold water (20 ml). The mixture was shaken and, then, left standing to form two layers. The upper layer was washed with water, dried (anhydrous sodium sulfate), and, then, concentrated under reduced pressure. The concentrate was washed with 1,2-dimethoxyethane to give 1-chloro-8-methylthio-4-phenylphthalazine (310 mg).
- mp: : 177-178°C
- IR (Nujol): : 1655, 1450, 1355 (cm⁻¹)
- ¹H-NMR(CDCl₃): : δ 2.62 (3H, s), 7.53-7.77 (8H, m)

### Reference Example 46

DMF (15 ml) was added to a mixture of methyl 2-benzoyl-6-iodobenzoate (5.40 g) and cuprous cyanide (1.49 g). The mixture was stirred for one hour at 80 °C. The reaction mixture was cooled in air, which was poured into a solution of ferric chloride (5.7 g) in water (20 ml) supplemented with conc. hydrochloric acid (1.4 ml). To the whole mixture was then added ethyl acetate (100 ml), which was shaken and, then, left standing to form two layers. The upper layer was washed with water, dried (anhydrous magnesium sulfate) and concentrated under reduced pressure. The concentrate was purified by subjecting to a silica gel column chromatography eluting with ethyl acetate/hexane to give methyl 2-benzoyl-6-cyanobenzoate (3.09 g) as a colorless oily product.
- IR (Neat): : 2240, 1730, 1665, 1280 (cm⁻¹)
- ¹H-NMR(CDCl₃): : δ 3.68 (3H, s), 7.45-7.53 (2H, m), 7.59-7.69 (1H, m), 7.71-7.78 (4H, m), 7.92-7.96 (1H, m)

### Reference Example 47

Methyl 2-benzoyl-6-cyanobenzoate (1.07 g) and hydrazine hydrate (232 mg) were stirred for 3 hours at 80 °C in ethanol (4 ml). To the reaction mixture was added acetic acid (60 mg). The mixture was cooled in air. The resulting crystalline precipitate was collected by filtration, which was washed with a small volume of ethanol and dried to give 8-cyano-4-phenyl-(2H)phthalazin-1-one (775 mg) as yellow powdery crystals.
- mp: : 277-278°C
- IR (Nujol): : 3025, 2220, 1665, 1330, 1155 (cm⁻¹)
- ¹H-NMR(DMSO-d₆): : δ 7.58 (5H, s), 7.94 (1H, dd), 8.04 (1H, t), 8.37 (1H, dd)

### Reference Example 48

Phosphorus oxychloride (1.22 g) and dimethylaniline (666 mg) were added 8-cyano-4-phenyl-(2H)phthalazin-1-one (371 mg). The mixture was stirred for one hour at 110 °C. The reaction mixture was concentrated under reduced pressure. To the concentrate were added ethyl acetate (50 ml) and cold water (20 ml), which was shaken and, then, left standing to form two layers. Crystals insoluble in both layers were collected by filtration and washed with water and ethyl acetate successively, followed by drying to give 1-chloro-8-cyano-4-phenylphthalazine (175 mg).
- mp: : 210-211°C
- IR (Nujol): : 2225, 1440, 1370, 1340, 1290 (cm⁻¹)
- ¹H-NMR(CDCl₃): : δ 7.60-7.71 (5H, m), 8.00 (1H, t), 8.35 (1H, d), 8.44 (1H, d)

### Reference Example 49

Acetic anhydride (50 ml) was added to 3-nitrophthalic acid (31.3 g). The mixture was stirred for two hours at 165 °C. The reaction mixture was concentrated under reduced pressure. To the concentrate was added isobutanol (150 ml), to which was added dropwise, under ice-cooling, pyridine (13 ml). The ice-bath was removed, and the reaction mixture was stirred for one hour at room temperature. The reaction mixture was concentrated under reduced pressure. To the concentrate were added ethyl acetate (300 ml), water (100 ml) and conc. hydrochloric acid (15 ml). The mixture was shaken and, then, left standing to form two layers. The upper layer was washed with water, dried (anhydrous sodium sulfate) and concentrated under reduced pressure. The concentrate was washed with ethyl acetate and dried to give 2-isobutoxycarbonyl-3-nitrobenzoic acid (27.8 g) as colorless prisms.
- mp: : 180-181°C
- IR (Nujol): : 1725, 1700, 1540, 1350, 1310, 1290, 1280 (cm⁻¹)
- ¹H-NMR(CDCl₃): : δ 1.00 (6H, d), 2.00-2.20 (1H, m), 4.21 (2H, d), 4.95 (1H, broad), 7.74 (1H, t), 8.43 (2H, d)

### Reference Example 50

To a solution of 2-isobutoxycarbonyl-3-nitrobenzoic acid (13.4 g) in ethyl acetate (100 ml) was added 10% Pd-C (5 g). The mixture was stirred for 5 hours in hydrogen streams under normal pressure. The catalyst was filtered off, and the filtrate was concentrated under reduced pressure to give 3-amino-2-isobutoxycarbonylbenzoic acid (11.8 g) as colorless prisms.
- mp: : 90-91°C (EtOAc)
- IR (Nujol): : 3500, 3400, 1695, 1610, 1300, 1280 (cm⁻¹)
- ¹H-NMR(CDCl₃): : δ 0.97 (6H, d), 1.90-2.10 (1H, m), 4.05 (2H, d), 5.70 (3H, broad), 6.85 (1H, dd), 7.05 (1H, dd), 7.28 (1H, m)

### Reference Example 51

To a solution of 3-amino-2-isobutoxycarbonylbenzoic acid (10.7 g) in acetone (100 ml) was added 47% aqueous solution of hydrogen bromide (31.0 g). To the mixture was added dropwise, under ice-cooling, a solution of sodium nitrite (3.7 g) in water (20 ml). The whole mixture was stirred for 0.5 hour under same conditions. The reaction mixture was cooled to -25 °C, to which was added, while stirring, cuprous oxide (0.4 g) little by little. The whole mixture was stirred for 3.5 hours under the same conditions. The reaction mixture was warmed up to room temperature, to which was added water (100 ml). The mixture was concentrated under reduced pressure. The concentrated was subjected to extraction with ethyl acetate (150 ml), washed with water, dried (anhydrous magnesium sulfate) and concentrated under reduced pressure. The concentrate was washed with ethyl acetate to give 3-bromo-2-isobutoxycarbonylbenzoic acid (8.98 g) as pale yellow prisms.
- mp: : 143-144°C
- IR (Nujol): : 1725, 1695, 1305, 1285 (cm⁻¹)
- ¹H-NMR(CDCl₃): : δ 1.00 (6H, d), 2.00-2.20 (1H, m), 4.17 (2H, d), 5.60 (1H, broad), 7.39 (1H, t), 7.84 (1H, dd), 8.08 (1H, dd)

### Reference Example 52

A solution of 3-bromo-2-isobutoxycarbonylbenzoic acid (7.53 g) in 1N sodium hydroxide (66 ml) was heated for 13 hours under reflux. The reaction mixture was cooled in air, to which was added conc. hydrochloric acid to make it acid, followed by subjecting to extraction with ethyl acetate (150 ml). The extract was washed with water, dried (anhydrous magnesium sulfate) and concentrated under reduced pressure. To the concentrate was added acetic anhydride (40 ml). The mixture was stirred for 2 hours at 165 °C. The reaction mixture was cooled in air and, then, concentrated under reduced pressure. To the concentrate was added toluene (60 ml), and the mixture was concentrated to dryness under reduced pressure. The concentrate was washed with a small volume of ethyl acetate to give 3-bromophthalic anhydride (4.67 g) as pale yellow prisms.
- mp: : 129-130°C
- IR (Nujol): : 1845, 1770, 1280, 1215, 905 (cm⁻¹)
- ¹H-NMR(CDCl₃): : δ 7.75 (1H, t), 8.00 (1H, dd), 8.04 (1H, dd)

### Reference Example 53

Dry tetrahydrofuran (3 ml) was added to a mixture of 2-bromotoluene (884 mg) and magnesium (126 mg), which was stirred for one hour at 60 °C under argon atmosphere. On the other hand, a solution of 3-bromophthalic anhydride (908 mg) in dry tetrahydrofuran (9 ml) was cooled to -5 °C, to which was added dropwise, under argon atmosphere, the above-mentioned Grignard reagent. Then, the ice-bath was removed, and the reaction mixture was warmed up to room temperature. To the reaction mixture was added 1N hydrochloric acid (5 ml), which was stirred for 30 minutes. To the reaction mixture were added ethyl acetate and water, which was shaken and, then, left standing to form two layers. The upper layer was subjected to extraction with 0.2N sodium hydroxide (30 ml). The aqueous layer was made acid with hydrochloric acid, which was subjected to extraction with ethyl acetate. The ethyl acetate layer was washed with water, dried (anhydrous sodium sulfate) and concentrated under reduced pressure to give a pale yellow oily product (1.19 g). This product was dissolved in DMF (5 ml), to which were added potassium carbonate (690 mg) and methyl iodide (710 mg). The mixture was stirred for 14 hours at room temperature. To the reaction mixture were added ethyl acetate (30 ml) and water (30 ml), which was shaken and, then, left standing to form two layers. The upper layer was washed with water, dried (anhydrous sodium sulfate) and concentrated under reduced pressure to give a colorless oily product (1.12 g). This product was dissolved bin ethanol (5 ml), to which was added hydrazine hydrate (150 mg). The mixture was heated for 13 hours under reflux. The reaction mixture was cooled in air. Then, the resulting crystalline precipitate was collected by filtration, which was washed with a small volume of ethanol and dried to give 8-bromo-4-(2-methylphenyl)-(2H)phthalazin-1-one (550 mg) as pale yellow prisms.
- mp: : 241-242°C
- IR (Nujol): : 1660, 1305 (cm⁻¹)
- ¹H-NMR(DMSO-d₆): : δ 2.14 (3H, s), 7.23-7.45 (5H, m), 7.50 (1H, t), 8.01 (1H, dd)

### Reference Example 54

Dry tetrahydrofuran (4 ml) was added to a mixture of 2-bromobiphenyl (932 mg) and magnesium (126 mg), which was stirred for one hour at 60 °C under argon atmosphere. On the other hand, a solution of 3-phthalic anhydride (908 mg) in dry tetrahydrofuran (9 ml) was cooled to -5 °C, to which was added dropwise, under argon atmosphere, the above-mentioned Grignard reagent. The ice-bath was removed, and the reaction mixture was warmed up to room temperature. To the reaction mixture was added 1N hydrochloric acid (5 ml), which was stirred for 30 minutes, followed by addition of ethyl acetate and water. The mixture was shaken and, then, left standing to form two layers. The upper layer was subjected to extraction with 0.2N sodium hydroxide (30 ml). The aqueous layer was made acid with hydrochloric acid, which was subjected to extraction with ethyl acetate. The ethyl acetate layer was washed with water, dried (anhydrous sodium sulfate) and concentrated under reduced pressure to give a pale yellow oily product (1.39 g). This product was dissolved in DMF (5 ml), to which were added potassium carbonate (690 mg) and methyl iodide (710 mg). The mixture was stirred for 14 hours at room temperature. To the reaction mixture were added ethyl acetate (30 ml) and water (30 ml), which was shaken and, then, left standing to form two layers. The upper layer was washed with water, dried (anhydrous sodium sulfate) and concentrated under reduced pressure to give a colorless oily product (1.30 g). This product was dissolved in ethanol (5 ml), to which was added hydrazine hydrate (150 mg). The mixture was heated for 13 hours under reflux. The reaction mixture was cooled in air. The resulting crystalline precipitate was filtered off, and the filtrate was concentrated under reduced pressure. The concentrated was purified by means of a silica gel column chromatography, eluting with ethyl acetate/hexane, to give 8-bromo-4-(biphenyl-2-yl)-(2H)phthalazin-1-one (257 mg).
- mp: : 206-207°C (EtOAc)
- IR (Nujol): : 1660 (cm⁻¹)
- ¹H-NMR(DMSO-d₆): : δ 7.11-7.62 (11H, m), 7.84 (1H, dd), 9.90 (1H, broad)

### Reference Example 55

Phosphorus oxychloride (1.22 g) and dimethylaniline (775 mg) were added to 8-bromo-4-(2-methylphenyl)-(2H) phthalazin-1-one (500 mg). The mixture was stirred for one hour at 110 °C. The reaction mixture was concentrated under reduced pressure, to which were added ethyl acetate (60 ml) and cold water (30 ml). The mixture was shaken and, then, left standing to form two layers. The upper layer was washed with water, dried (anhydrous sodium sulfate) and concentrated under reduced pressure. The concentrate was washed with isopropyl ether and dried to give 8-bromo-1-chloro-4-(2-methylphenyl)phthalazine (398 mg).
- mp: : 185-186°C
- IR (Nujol): : 1375, 1275, 780 (cm⁻¹)
- ¹H-NMR(CDCl₃): : δ 2.08 (3H, s), 7.27-7.50 (4H, m), 7.62 (2H, d), 8.30 (1H, t)

### Reference Example 56

Phosphorus oxychloride (730 mg) and dimethylaniline (420 mg) were added to 8-bromo-4-(biphenyl-2-yl)-(2H)phthalazin-1-one (255 mg). The mixture was stirred for 3 hours at 110 °C. The reaction mixture was concentrated under reduced pressure. To the concentrate were added ethyl acetate (30 ml) and cold water (20 ml). The mixture was shaken and, then, left standing to form two layers. The upper layer was washed with water, dried (anhydrous sodium sulfate) and concentrated under reduced pressure. The concentrate was washed with ethyl acetate and dried to give 8-bromo-1-chloro-4-(biphenyl-2-yl)phthalazine (175 mg).
- mp: : 160-161°C
- IR (Nujol): : 1355, 1275, 765 (cm⁻¹)
- ¹H-NMR(CDCl₃): : δ 7.04-7.13 (5H, m), 7.38 (1H, t), 7.48 (1H, dd), 7.55-7.70 (4H, m), 8.09 (1H, dd)

### Reference Example 57

Dry tetrahydrofuran (4 ml) was added to a mixture of 2-bromobenzotrifluoride (903 mg) and magnesium (126 mg). The mixture was stirred for one hour at 60 °C under argon atmosphere. On the other hand, a solution of 3-bromophthalic anhydride (908 mg) in dry tetrahydrofuran (9 ml) was cooled to -5 °C, to which was added dropwise, under argon atmosphere, the above-mentioned Grignard reagent. The ice bath was removed, and the reaction mixture was warmed up to room temperature. To the reaction mixture was added 1N hydrochloric acid (5 ml), which was stirred for 30 minutes, followed by addition of ethyl acetate and water. The mixture was shaken and, then, left standing to form two layers. The upper layer was subjected to extraction with 0.2N sodium hydroxide (30 ml). The aqueous layer was made acid with hydrochloric acid, which was subjected to extraction with ethyl acetate. The ethyl acetate layer was washed with water, dried (anhydrous sodium sulfate) and concentrated under reduced pressure to give a pale yellow oily product (1.35 g). This product was dissolved in DMF (5 ml), to which were added potassium carbonate (690 mg) and methyl iodide (710 mg). The mixture was stirred for 14 hours at room temperature. To the reaction mixture were added ethyl acetate (30 ml) and water (30 ml). The mixture was shaken and, then, left standing to form two layers. The upper layer was washed with water, dried (anhydrous sodium sulfate) and concentrated under reduced pressure to give a colorless oily product (1.28 g). This product was dissolved in ethanol (5 ml), to which was added hydrazine hydrate (200 mg). The mixture was heated for 14 hours under reflux. The reaction mixture was cooled in air. The, the resulting crystalline precipitate was collected by filtration, which was washed with a small volume of ethanol and dried to give 8-bromo-4-(2-trifluoromethylphenyl)-(2H)phthalazin-1-one (543 mg).

### Reference Example 58

Phosphorus oxychloride (1.40 g) and dimethylaniline (726 mg) were added to 8-bromo-4-(2-trifluoromethylphenyl)-(2H)phthalazin-1-one (415 mg). The mixture was stirred for two hours at 110 °C. The reaction mixture was concentrated under reduced pressure. To the concentrate were added ethyl acetate (60 ml) and cold water (30 ml). The mixture was shaken and, then, left standing to form two layers. The upper layer was washed with water, dried (anhydrous sodium sulfate) and concentrated under reduced pressure. The concentrate was washed with ethyl acetate and dried to give 8-bromo-1-chloro-4-(2-trifluoromethylphenyl)phthalazine (200 mg).

### Reference Example 59

Dry tetrahydrofuran (7 ml) was added to a mixture of 2-chloroiodobenzene (1.19 g) and magnesium (146 mg). The mixture was stirred for one hour at 60 °C under argon atmosphere. On the other hand, a solution of 3-bromophthalic anhydride (908 mg) in dry tetrahydrofuran (9 ml) was cooled to -5 °C, to which was added dropwise, under argon atmosphere, the above-mentioned Grignard reagent. The ice bath was removed, and the reaction mixture was warmed up to room temperature. To the reaction mixture was added 1N hydrochloric acid (6 ml), which was stirred for 30 minutes. To the mixture were added ethyl acetate and water. The mixture was shaken and, then left standing to form two layers. The upper layer was subjected to extraction with 0.2N sodium hydroxide (35 ml). The aqueous layer was made acid with hydrochloric acid, which was subjected to extraction with ethyl acetate. The ethyl acetate layer was washed with water, dried (anhydrous sodium sulfate) and concentrated under reduced pressure to give a pale yellow solid product (0.80 g). This product was dissolved in DMF (5 ml), to which were added potassium carbonate (970 mg) and methyl iodide (710 mg). The mixture was stirred for 14 hours at room temperature. To the reaction mixture were added ethyl acetate (50 ml) and water (30 ml). The mixture was shaken and, then, left standing to form two layers. The upper layer was washed with water, dried (anhydrous sodium sulfate) and concentrated under reduced pressure to give a colorless oily product. This product was dissolved in ethanol (3 ml), to which was added hydrazine hydrate (50 ml). The mixture was heated for 13 hours under reflux. The reaction mixture was cooled in air. Then, the resulting crystalline precipitate was collected by filtration, washed with a small volume of ethanol and dried to give 8-bromo-4-(2-chlorophenyl)-(2H)phthalazin-1-one (120 mg) as colorless needles.
- mp: : 265-266°C
- IR (Nujol): : 3160, 1660 (cm⁻¹)
- ¹H-NMR(CDCl₃): : δ 7.24 (1H, dd), 7.41-7.72 (5H, m), 8.01 (1H, dd), 10.03 (1H, broad)

### Reference Example 60

Phosphorus oxychloride (460 mg) and dimethylaniline (242 mg) were added to 8-bromo-4-(2-chlorophenyl)-(2H)phthalazin-1-one (115 mg). The mixture was stirred for two hours at 110 °C. The reaction mixture was concentrated under reduced pressure. To the concentrate were added ethyl acetate (30 ml) and cold water (20 ml). The mixture was shaken and, then, left standing to form two layers. The upper layer was washed with water, dried (anhydrous sodium sulfate) and concentrated under reduced pressure. The concentrate was washed with ethyl acetate and dried to give 8-bromo-1-chloro-4-(2-chlorophenyl)phthalazine (98 mg).
- mp: : 196-197°C
- IR (Nujol): : 1360, 1285 (cm⁻¹)
- ¹H-NMR(CDCl₃): : δ 7.48-7.63 (5H, m), 7.66 (1H, t), 8.31 (1H, dd)

### Reference Example 61

3-Amino-2-isobutoxycarbonyl benzoic acid (4.48 g) was dissolved in acetone (50 ml), to which was added wet hydrochloric acid (6.3 ml). To the mixture was added dropwise, under ice-cooling, a solution of sodium nitrite (1.38 g) in water (10 ml). The mixture was stirred for 0.5 hour under the same conditions. The reaction mixture was cooled to -30 °C, to which was added, while stirring, a solution of sodium iodide (4.5 g) in water (10 ml). The mixture was stirred for one hour under the same conditions. The ice bath was removed, and the reaction mixture was warmed up to 0 °C, to which was added water (50 ml), followed by concentration under reduced pressure. The concentrate was subjected to extraction with ethyl acetate (150 ml). The extract was washed with water, dried (anhydrous magnesium sulfate) and concentrated under reduced pressure to give 3-iodo-2-isobutoxycarbonyl benzoic acid (6.04 g) as a pale yellow oily product.
- IR (Neat): : 1730, 1700, 1450 (cm⁻¹)
- ¹H-NMR(CDCl₃): : δ 1.01 (6H, d), 2.02-2.20 (1H, m), 4.16 (2H, d), 7.23 (1H, t), 8.06-8.12 (2H, m)

### Reference Example 62

3-Iodo-2-isobutoxycarbonyl benzoic acid (3.3 g) was dissolved in 1N potassium hydroxide (35 ml), which was heated for 13 hours under reflux. The reaction mixture was cooled in air, which was made acid with conc. hydrochloric acid, followed by extraction with ethyl acetate (150 ml). The extract was washed with water, dried (anhydrous magnesium sulfate) and concentrated under reduced pressure. To the concentrate was added acetic anhydride (15 ml), and the mixture was stirred for two hours at 160 °C. The reaction mixture was cooled in are and, then, concentrated under reduced pressure. To the concentrate was added toluene (30 ml), and the mixture was concentrated to dryness. The concentrate was washed with hexane to give 3-iodophthalic anhydride (0.67 g) as yellow prisms.
- mp: : 144-146°C
- IR (Nujol): : 1840, 1780, 1220, 905 (cm⁻¹)
- ¹H-NMR(CDCl₃): : δ 7.56 (1H, t), 8.01 (1H, dd), 8.31 (1H, dd)

### Reference Example 63

Dry tetrahydrofuran (2 ml) was added to a mixture of 2-bromotoluene (410 mg) and magnesium (76 mg). The whole mixture was stirred for one hour at 60 °C under argon atmosphere. On the other hand, a solution of 3-iodophthalic anhydride (655 mg) in dry tetrahydrofuran (5 ml) was cooled to -5 °C, to which was added dropwise, under argon atmosphere, the above-mentioned Grignard reagent. Then, the ice bath was removed, and the reaction mixture was warmed up to room temperature. To the reaction mixture was added 1N hydrochloric acid (4 ml). The mixture was stirred for 30 minutes, to which were added ethyl acetate and water. The mixture was shaken and, then, left standing to form two layers. The upper layer was subjected to extraction with 2N sodium hydroxide (20 ml). The aqueous layer was made acid with hydrochloric acid, which was subjected to extraction with ethyl acetate. The ethyl acetate layer was washed with water, dried (anhydrous sodium sulfate) and concentrated under reduced pressure to give a pale yellow oily product. This product was dissolved in DMF (4 ml), to which were added potassium carbonate (500 mg) and methyl iodide (510 mg). The mixture was stirred for 14 hours at room temperature, to which were added ethyl acetate (30 ml), hexane (20 ml) and water (30 ml). The upper layer was washed with water, dried (anhydrous sodium sulfate) and concentrated under reduced pressure to give a pale yellow oily product. This product was dissolved in ethanol (5 ml), to which was added hydrazine hydrate (100 mg). The mixture was heated for 13 hours under reflux. The reaction mixture was cooled in air, then, the resulting crystalline product was collected by filtration, washed with a small volume of ethanol and dried to give 8-iodo-4-(2-methylphenyl)-(2H)phthalazin-1-one (245 mg).
- mp: : 198-199°C
- IR (Nujol): : 3160, 1655 (cm⁻¹)
- ¹H-NMR(DMSO-d₆): : δ 2.06 (3H, s), 7.17 (1H, dd), 7.31-7.49 (5H, m), 8.41 (1H, dd)

### Reference Example 64

Phosphorus oxychloride (765 mg) and dimethylaniline (424 mg) were added to 8-iodo-4-(2-methylphenyl)-(2H)phthalazin-1-one (235 mg). The mixture was stirred for two hours at 110 °C. The reaction mixture was concentrated under reduced pressure. To the concentrate were added ethyl acetate (50 ml) and cold water (30 ml). The mixture was shaken and, then, left standing to form two layers. The upper layer was washed with water, dried (anhydrous sodium sulfate) and concentrated under reduced pressure. The concentrate was washed with a small volume of ethyl acetate and dried to give 1-chloro-8-iodo-4-(2-methylphenyl)phthalazine (165 mg).
- mp: : 138-139°C
- IR (Nujol): : 1655, 1450, 1350, 1275 (cm⁻¹)
- ¹H-NMR(CDCl₃): : δ 2.08 (3H, s), 7.32-7.50 (5H, m), 7.65 (1H, dd), 8.74 (1H, dd)

### Reference Example 65

Dry tetrahydrofuran (7 ml) was added to a mixture of 4-fluoro-2-methylbromobenzene (567 mg) and magnesium (85 mg). The whole mixture was stirred for 3 hours at 65 °C under argon atmosphere. On the other hand, a solution of 3-bromophthalic anhydride (617 mg) in dry tetrahydrofuran (6 ml) was cooled to -5 °C, to which was added dropwise, under argon atmosphere, the above-mentioned Grignard reagent. The mixture was stirred for 30 minutes under the same conditions. The ice-bath was removed, and the reaction mixture was warmed up to room temperature. To the reaction mixture was added 1N hydrochloric acid (5 ml), which was stirred for 30 minutes. To the reaction mixture were added ethyl acetate and water. The whole mixture was shaken and, then, left standing to form two layers. The upper layer was subjected to extraction with 2N sodium hydroxide (20 ml). The aqueous layer was made acid with hydrochloric acid, which was subjected to extraction with ethyl acetate. The ethyl acetate layer was washed with water, dried (anhydrous sodium sulfate) and concentrated under reduced pressure to give a pale yellow oily product. This product was dissolved in DMF (5 ml), to which were added potassium carbonate (500 mg) and methyl iodide (510 mg). The mixture was stirred for 14 hours at room temperature. To the reaction mixture were added ethyl acetate (30 ml) and water (30 ml), which was shaken and, then, left standing to form two layers. The upper layer was washed with water, dried (anhydrous sodium sulfate) and concentrated under reduced pressure to give a pale yellow oily product. This product was dissolved in ethanol (5 ml), to which was added hydrazine hydrate (100 mg). The mixture was heated for 13 hours under reflux. The reaction mixture was cooled in air and, then, the resulting crystalline precipitate was collected by filtration, washed with a small volume of ethanol and dried to give 8-bromo-4-(4-fluoro-2-methylphenyl)-(2H)phthalazin-1-one (167 mg) as colorless powdery crystals.
- mp: : 238-239°C
- IR (Nujol): : 3280, 1675 (cm⁻¹)
- ¹H-NMR(DMSO-d₆): : δ 2.14 (3H, s), 7.00-7.09 (2H, m), 7.52 (1H, t), 8.02 (1H, dd), 10.11 (1H, broad)

### Reference Example 66

Phosphorus oxychloride (500 mg) and dimethylaniline (242 mg) were added to 8-bromo-4-(4-fluoro-2-methylphenyl)-(2H)phthalazin-1-one (161 mg). The mixture was stirred for two hours at 110 °C. The reaction mixture was concentrated under reduced pressure. To the concentrate were added ethyl acetate (50 ml) and cold water (30 ml). The mixture was shaken and, then, left standing to form two layers. The upper layer was washed with water, dried (anhydrous sodium sulfate) and concentrated under reduced pressure. The concentrate was washed with isopropyl ether and dried to give 8-bromo-1-chloro-4-(4-fluoro-2-methylphenyl)phthalazine (140 mg).
- mp: : 228-229°C
- IR (Nujol): : 1355, 1270 (cm⁻¹)
- ¹H-NMR(CDCl₃): : δ 2.08 (3H, s), 7.03-7.12 (2H, m), 7.25-7.33 (1H, m), 7.58-7.68 (2H, m), 8.31 (1H, dd)

### Reference Example 67

Methyl 2-chloroformyl-6-nitrobenzoate (2.44 g) and tributyl(2-pyridyl)tin (b.p. 120-130 °C/0.2 mmHg; 4.42 g) were dissolved in toluene (20 ml). The solution was stirred for one hour at room temperature. The reaction mixture was concentrated under reduced pressure. The concentrate was purified by means of a silica gel chromatography, eluting with ethyl acetate/hexane, to give methyl 6-nitro-2-(2-pyridylcarbonyl)benzoate (1.84 g).
- ¹H-NMR(CDCl₃): : δ 3.74 (3H, s), 7.45 (1H, m), 7.72 (1H, t), 7.96 (1H, dt), 8.33 (1H, d), 8.41 (1H, dd), 8.44-8.49 (2H, m)

### Reference Example 68

A mixture of methyl 6-nitro-2-(2-pyridylcarbonyl)benzoate (1.84 g) and hydrazine hydrate (0.71 g) was heated in ethanol (20 ml) under reflux. The reaction mixture was cooled in air, then, the resulting crystalline precipitate was collected by filtration, which was washed with ethanol and isopropyl ether successively, followed by drying to give 8-nitro-4-(2-pyridyl)-(2H)phthalazin-1-one (1.55 g).
- ¹H-NMR(DMSO-d₆): : δ 3.30 (1H, s), 7.40 (1H, m), 7.85 (1H, d), 7.96 (1H, dt), 8.05 (1H, t), 8.37-8.42 (2H, m), 8.65 (1H, dd)

### Reference Example 69

Phosphorus oxychloride (0.91 ml) and dimethylaniline (0.25 ml) were added to 8-nitro-4-(2-pyridyl)-(2H)phthalazin-1-one (0.27 g). The mixture was stirred for two hours at 110 °C., which was concentrated under reduced pressure. To the concentrate were added ethyl acetate (30 ml) and an aqueous solution of sodium hydrogencarbonate (20 ml). The mixture was shaken and, then, left standing to form two layers. The upper layer was washed with an aqueous saline solution, dried (anhydrous magnesium sulfate) and concentrated under reduced pressure. The concentrate was washed with ethyl acetate and isopropyl ether successively and dried to give 1-chloro-8-nitro-4-(2-pyridyl)phthalazine (0.21 g).
- ¹H-NMR(CDCl₃): : δ 7.41 (1H, m), 7.98 (1H, dt), 8.12 (1H, t), 8.34 (1H, d), 8.45 (1H, dd), 8.55 (1H, d), 8.68 (1H, dd)

### Reference Example 70

4,5-Dichlorophthalic anhydride (25.0 g) was dispersed in methanol (200 ml), which was stirred for 3 hours at 70 °C. The reaction mixture was cooled in air, which was then concentrated under reduced pressure. The resulting crystalline precipitate was collected by filtration, washed with hexane and dried to give 4,5-dichloro-2-methoxycarbonyl benzoic acid (28.6 g).
- ¹H-NMR(CDCl₃): : δ 3.94( 3H, s), 7.81 (1H, s), 8.01 (1H, s)

### Reference Example 71

4,5-Dichloro-2-methoxycarbonyl benzoic acid (12.5 g) was dispersed in benzene (40 ml), to which was added thionyl chloride (7.9 ml). The mixture was heated for two hours under reflux. The reaction mixture was concentrated under reduced pressure to give methyl 4,5-dichloro-2-chloroformyl benzoate (13.4 g) as a colorless oily product.

### Reference Example 72

Methyl 4,5-dichloro-2-chloroformyl benzoate (0.8 g) and tributyl(2-pyridyl)tin (1.33 g) were dissolved in toluene (8 ml). The solution was stirred for one hour at room temperature. The reaction mixture was concentrated under reduced pressure. The concentrate was purified by means of a silica gel chromatography, eluting with ethyl acetate/hexane, to give methyl 4,5-dichloro-2-(2-pyridylcarbonyl)benzoate (0.54 g).
- ¹H-NMR(CDCl₃): : δ 3.54 (3H, s), 7.45 (1H, m), 7.60 (1H, s), 7.92 (1H, dt), 8.10 (1H, s), 8.25 (1H, td), 8.56 (1H,m)

### Reference Example 73

A mixture of methyl 4,5-dichloro-2-(2-pyridylcarbonyl)benzoate (0.53 g) and hydrazine hydrate (0.19 g) was heated for two hours in ethanol (10 ml) under reflux. The reaction mixture was cooled in air. The resulting crystalline precipitate was then collected by filtration, which was washed with ethanol and isopropyl ether, successively, and dried to give 6,7-dichloro-4-(2-pyridyl)-(2H)phthalazin-1-one (0.49 g).
- ¹H-NMR(DMSO-d₆): : δ 7.57 (1H, m), 7.95 (1H, t), 8.04 (1H, dt), 8.45 (1H, s), 8.79 (1H, m), 8.92 (1H, s)

### Reference Example 74

Phosphorus oxychloride (1.36 ml) and dimethylaniline (0.38 ml) were added to 6,7-dichloro-4-(2-pyridyl)-(2H)phthalazin-1-one (0.29 g). The mixture was stirred for three hours at 110 °C. The reaction mixture was concentrated under reduced pressure. To the concentrate were added ethyl acetate (30 ml) and an aqueous solution of sodium hydrogencarbonate (20 ml). The mixture was shaken and, then, left standing to form two layers. The upper layer was washed with an aqueous saline solution, dried (anhydrous magnesium sulfate) and concentrated under reduced pressure. The concentrate was washed with ethyl acetate and isopropyl ether, successively, and dried to give 1,6,7-trichloro-4-(2-pyridyl)phthalazine (0.25 g).
- ¹H-NMR(CDCl₃): : δ 7.52 (1H, dd), 7.99 (1H, dt), 8.31 (1H, d), 8.49 (1H, s), 8.84 (1H, dd), 9.24 (1H, s)

### Reference Example 75

Methyl 2-chloroformyl-6-nitrobenzoate (4.87 g) and tributyl(2-thienyl)tin (b.p.125-135 °C/0.2mmHg; 8.96 g) were dissolved in toluene (40 ml). To the solution was added Pd(Ph₃P)₂Cl₂ (70 mg), and the mixture was stirred for 1.5 hour at 70 °C under argon atmosphere. The reaction mixture was cooled in air, which was concentrated under reduced pressure. The concentrate was purified by means of a silica gel chromatography, eluting with ethyl acetate/hexane, to give methyl 6-nitro-2-(3-thienylcarbonyl)benzoate (3.46 g) and methyl 6-nitro-2-(2-nitro-2-(2-thienylcarbonyl)benzoate (0.60 g).
- ¹H-NMR(CDCl₃): : δ
(3-thienyl form) 3.84 (3H, s), 7.18 (1H, dd), 7.50 (1H, dd), 7.72 (1H, t), 7.83 (1H, dd), 7.95 (1H, dd), 8.23 (1H, dd) (2-thienyl form) 3.86 (3H, s), 7.10 (1H, dd), 7.20 (1H, m), 7.44 (1H, dd), 7.61 (1H, t), 7.82 (1H, dd), 8.18 (1H, dd)

### Reference Example 76

A mixture of methyl 6-nitro-2-(3-thienylcarbonyl)benzoate (2.40 g) and 10% Pd-C (0.36 g) was stirred for 16 hours in a mixture solvent consisting of methanol (30 ml) and ethyl acetate (30 ml) in hydrogen streams under atmospheric pressure. The catalyst was filtered off through celite and washed with ethyl acetate. The filtrate was concentrated under reduced pressure and dried to give methyl 6-amino-2-(3-thienylcarbonyl)benzoate (2.15 g) as an oily product.
- ¹H-NMR(CDCl₃): : δ 3.52 (3H, s), 5.67 (2H, broad), 6.73 (1H, dd), 6.81 (1H, dd), 7.07 (1H, dd), 7.33 (1H, dd), 7.33 (1H, d), 7.66 (1H, dd)

### Reference Example 77

Methyl 6-amino-2-(3-thienylcarbonyl)benzoate (2.09 g) was dissolved in acetone (20 ml), to which was added 47% aqueous solution of hydrogen bromide (10.8 g). The mixture was cooled to -5 °C, to which was added dropwise a solution of sodium nitrite (0.61 g) in water (3 ml). The mixture was stirred for 0.5 hour under the same conditions, to which was added water (3 ml). The reaction mixture was cooled to -15 °C, to which was added, little by little, cuprous oxide (50 mg). The mixture was stirred for 0.5 hour under the same conditions. The reaction mixture was warmed up to room temperature, to which was added water (20 ml), followed by concentration under reduced pressure. The concentrate was subjected to extraction with ethyl acetate. The extract was washed with an aqueous saline solution, dried (anhydrous magnesium sulfate) and concentrated under reduced pressure. The concentrate was purified by means of a silica gel column chromatography, eluting with ethyl acetate/hexane, to give methyl 6-bromo-2-(3-thienylcarbonyl)benzoate (1.40 g).
- ¹H-NMR(CDCl₃): : δ 3.80 (3H, s), 7.15 (1H, dd), 7.40 (1H, t), 7.52 (1H, dd), 7.66 (1H, dd), 7.76-7.82 (2H, m)

### Reference Example 78

A mixture of methyl 6-bromo-2-(3-thienylcarbonyl)benzoate (1.39 g) and hydrazine hydrate (0.47 g) was heated in ethanol (15 ml) under reflux. The reaction mixture was cooled in air, then, the resulting crystalline precipitate was collected by filtration, which was washed with ethanol and isopropyl ether, successively, and dried to give 8-bromo-4-(3-thienyl)-(2H)phthalazin-1-one (1.25 g).
- ¹H-NMR(DMSO-d₆): : δ 7.26 (1H, dd), 7.53 (1H, d), 7.76 (1H, d), 7.81 (1H, d), 8.09 (1H, d), 8.14 (1H, d)

### Reference Example 79

Phosphorus oxychloride (0.91 ml) and dimethylaniline (0.25 ml) were added to 8-bromo-4-(3-thienyl)-(2H)phthalazin-1-one (0.61 g). The mixture was stirred for one hour at 110 °C. The reaction mixture was concentrated under reduced pressure, to which were added chloroform (30 ml) and an aqueous solution of sodium hydrogencarbonate (20 ml). The mixture was shaken and, then left standing to form two layers. The lower layer was washed with an aqueous saline solution, dried (anhydrous magnesium sulfate) and concentrated under reduced pressure. The concentrate was washed with ethyl acetate and isopropyl ether, successively, and dried to give 8-bromo-1-chloro-4-(3-thienyl)phthalazine (0.56 g).
- ¹H-NMR(CDCl₃): : δ 7.27 (1H, t), 7.59 (1H, dd), 7.65 (1H, dd), 7.75 (1H, t), 8.33 (1H, dd), 8.49 (1H, dd)

### Reference Example 80

Ethanol (500 ml) and hydrazine hydrate (6.04 g) were added to 4,5-dichlorophthalic anhydride (25.0 g). The mixture was stirred for 4 hours at 100 °C. The reaction mixture was cooled in air, then, the resulting crystalline precipitate was collected by filtration, followed by washing with ethanol and isopropyl ether, successively, which was then dried to give 6,7-dichlorophthalazine-1,4-dione (24.4 g).
- ¹H-NMR(DMSO-d₆): : δ 7.91 (2H, d), 8.19 (2H, s)

### Reference Example 81

Phosphorus oxychloride (35.0 ml) and dimethylaniline (14.7 ml) were added to 6.7-dichlorophthalazine-1,4-dione (8.94 g). The mixture was stirred for two hours at 110 °C. The reaction mixture was cooled in air, then, the reaction mixture was concentrated under reduced pressure. To the concentrate were added ethyl acetate and an aqueous solution of sodium hydrogencarbonate. The mixture was shaken and, then, left standing to form two layers. The upper layer was washed with an aqueous saline solution, dried (anhydrous magnesium sulfate) and concentrated under reduced pressure. The concentrate was purified by means of a silica gel chromatography, eluting with ethyl acetate/hexane, to give 1,4,6,7-tetrachlorophthalazine (4.94 g).
- ¹H-NMR(CDCl₃): : δ 8.43 (2H, s)

### Reference Example 82

Toluene (20 ml) and water (5 ml) were added to a mixture of 1,4,6,7-tetrachlorophthalazine (0.54 g), phenylboric acid (0.27 g) and potassium carbonate (0.81 g). The mixture was stirred for 30 minutes at room temperature under argon atmosphere, to which was then added Pd(Ph₃P)₂Cl₂ (42 mg). The mixture was stirred for 20 hours at 90 °C. The reaction mixture was cooled in air, to which was then added ethyl acetate and water. The mixture was shaken and, then, left standing to form two layers. The upper layer was washed with an aqueous saline solution, dried (anhydrous magnesium sulfate) and concentrated under reduced pressure. The concentrate was purified by means of a silica gel chromatography, eluting ethyl acetate/hexane, to give 1,6,7-trichloro-4-phenylphthalazine (0.21 g).
- ¹H-NMR(CDCl₃): : δ 7.60-7.74 (5H, m), 8.17 (1H, s), 8.50 (1H, s)

### Reference Example 83

Toluene (20 ml) and water (5 ml) were added to a mixture of 1,4,6,7-tetrachlorophthalazine (0.50 g), 4-fluorophenylboric acid (0.39 g) and potassium carbonate (0.65 g). The whole mixture was stirred for 30 minutes at room temperature under argon atmosphere, to which was added Pd(Ph₃P)₂Cl₂ (66 mg). The mixture was stirred for 20 hours at 90 °C. The reaction mixture was cooled in air, to which were added ethyl acetate and water. The mixture was shaken and, then left standing to form two layers. The upper layer was washed with an aqueous saline solution, dried (anhydrous magnesium sulfate) and concentrated under reduced pressure. The concentrate was purified by means of a silica gel chromatography, eluting ethyl acetate/hexane, to give 1,6,7-trichloro-4-(4-fluorophenyl)phthalazine (0.28 g).
- ¹H-NMR(CDCl₃): : δ 7.32 (2H, t), 7.71 (2H, dd), 8.14 (1H, s), 8.50 (1H, s)

### Reference Example 84

A solution of methyl 2-chloroformyl-6-nitrobenzoate (2.44 g) in 1,2-dichloroethane (6 ml) was added, under ice-cooling, to a suspension of anhydrous aluminum chloride (2.93 g) in fluorobenzene (5.6 ml). The mixture was stirred for 30 minutes under the same conditions. The, the ice-bath was removed, and the reaction mixture was stirred for two hours at room temperature. The reaction mixture was poured into cold water containing conc. hydrochloric acid (2.2 ml), to which was added ethyl acetate. The mixture was stirred for 30 minutes. The organic layer was washed with an aqueous saline solution, dried (anhydrous magnesium sulfate) and concentrated under reduced pressure. The concentrate was purified by means of a silica gel column chromatography, eluting with ethyl acetate/hexane, to give methyl 2-(4-fluorobenzoyl)-6-nitrobenzoate (1.92 g).
- mp: : 106-107°C
- ¹H-NMR(CDCl₃): : δ 3.78 (3H, s), 7.18 (2H, t), 7.66-7.86 (4H, m), 8.21 (1H, dd)

### Reference Example 85

A mixture of methyl 2-(4-fluorobenzoyl)-6-nitrobenzoate (1.90 g) and 10% Pd-C (0.19 g) was stirred for 12 hours in a mixture solvent consisting of methanol (20 ml) and ethyl acetate (20 ml) under atmospheric pressure in hydrogen streams. The catalyst was filtered off through celite and washed with ethyl acetate. The filtrate was concentrated under reduced pressure. The concentrate was purified by means of a silica gel column chromatography, eluting with ethyl acetate/hexane, to give methyl 6-amino-2-(4-fluorobenzoyl)benzoate (1.65 g).
- mp: : 78-79°C
- ¹H-NMR(CDCl₃): : δ 3.44 (3H, s), 5.70 (2H, broad), 6.61 (1H, dd), 6.80 (1H, dd), 7.10 (2H, t), 7.32 (1H, dd), 7.80 (2H, dd)

### Reference Example 86

Methyl 6-amino-2-(4-fluorobenzoyl)benzoate (1.64 g) was dissolved in acetone (16 ml). To the solution were added conc. hydrochloric acid (4.1 ml) and water (4 ml). To the mixture placed on an ice-bath was added dropwise a solution of sodium nitrite (0.46 g) in water (2.3 ml). The whole mixture was stirred for 0.5 hour under the same conditions. The ice-bath was removed, and the reaction mixture was warmed up to room temperature, to which was added water (16 ml). The mixture was concentrated under reduced pressure. The concentrate was subjected to extraction with ethyl acetate. The ethyl acetate layer was washed with an aqueous saline solution, dried (anhydrous magnesium sulfate) and concentrated under reduced pressure. The concentrate was purified by means of a silica gel column chromatography, eluting with ethyl acetate/hexane, to give methyl 2-(4-fluorobenzoyl)-6-iodobenzoate (2.09 g).
- ¹H-NMR(CDCl₃): : δ 3.74 (3H, s), 7.15 (2H, t), 7.22 (1H, t), 7.48 (1H, dd), 7.81 (2H, dd), 8.07 (1H, dd)

### Reference Example 87

DMF (15 ml) and toluene (5 ml) were added to a mixture of methyl 2-(4-fluorobenzoyl)-6-iodobenzoate (1.70 g), sodium trifluoroacetate (3.62 g) and cuprous iodide (4.05 g). The whole mixture was subjected to azeotropic distillation for 1.5 hour at 150 °C to remove the moisture in the reaction system. Then, toluene was distilled off, and the residue was heated for 8 hours at 165 °C under reflux. The reaction mixture was cooled in air, to which were added ethyl acetate and water. Insolubles were filtered off through celite. The filtrate was shaken and, then, left standing to form two layers. The upper layer was washed with an aqueous saline solution, dried (anhydrous magnesium sulfate) and concentrated under reduced pressure. The concentrate was purified by means of a silica gel column chromatography, eluting with ethyl acetate/hexane, to give methyl 2-(4-fluorobenzoyl)-8-trifluoromethylbenzoate (1.17 g) as a pale yellow oily product.
- ¹H-NMR(CDCl₃): : δ 3.74 (3H, s), 7.17 (2H, t), 7.69 (2H, m), 7.78-7.93 (3H, m)

### Reference Example 88

A mixture of methyl 2-(4-fluorobenzoyl)-6-trifluoromethyl benzoate (1.116 g) and hydrazine hydrate (9.3 g) was heated for 14 hours in ethanol (12 ml) under reflux. The reaction mixture was cooled in air, then the resulting crystalline precipitate was collected by filtration, which was washed with ethanol and isopropyl ether, successively, and dried to give 8-trifluoromethyl-4-(4-fluorophenyl)-(2H)phthalazin-1-one (1.16 g).
- ¹H-NMR(CDCl₃): : δ 7.25 (2H, t), 7.54 (2H, dd), 7.92 (1H, m), 8.22 (1H, d), 8.55 (1H, m)

### Reference Example 89

Phosphorus oxychloride (4.81 ml) and dimethylaniline (0.89 ml) were added to 8-trifluoromethyl-4-(4-fluorophenyl)-(2H)phthalazin-1-one (1.15 g). The mixture was stirred for two hours at 110 °C. The reaction mixture was cooled in air, which was concentrated under reduced pressure. To the concentrate were added ethyl acetate and an aqueous solution of sodium hydrogencarbonate. The mixture was shaken and, then, left standing to form two layers. The upper layer was washed with an aqueous saline solution, dried (anhydrous magnesium sulfate) and concentrated under reduced pressure. The concentrate was purified by means of a silica gel chromatography, eluting with ethyl acetate/hexane, to give 1-chloro-8-trifluoromethyl-4-(4-fluorophenyl)phthalazine (0.80 g).
- mp: : 146-148°C
- ¹H-NMR(CDCl₃): : δ 7.30 (2H, t), 7.68 (2H, dd), 8.00 (1H, t), 8.29 (1H, d), 8.48 (1H, d)

### Reference Example 90

A mixture of methyl 2-(4-fluorobenzoyl)-6-iodobenzoate (0.29 g) and hydrazine hydrate (83 mg) was heated for 5 hours in ethanol (4 ml) under reflux. The reaction mixture was cooled in air, and, then, the resulting crystalline precipitate was collected by filtration, which was washed with ethanol and isopropyl ether, successively, followed by drying to give 4-(4-fluorophenyl)-8-iodo-(2H)phthalazin-1-one (0.21 g).
- ¹H-NMR(CDCl₃): : δ 7.22 (2H, t), 7.35 (1H, t), 7.52 (2H, dd), 7.66 (1H, dd), 8.42 (1H, dd)

### Reference Example 91

Phosphorus oxychloride (0.50 ml) and dimethylaniline (0.14 ml) were added to 4-(4-fluorophenyl)-8-iodo-(2H)phthalazin-1-one (0.20 g). The mixture was stirred for one hour at 110 °C. The reaction mixture was cooled in air, and the reaction mixture was concentrated under reduced pressure. To the concentrate were added chloroform and an aqueous solution of sodium hydrogencarbonate. The mixture was shaken and, then, left standing to form two layers. The lower layer was washed with an aqueous saline solution, dried (anhydrous magnesium sulfate) and concentrated under reduced pressure. The concentrate was purified by means of a silica gel chromatography, eluting with ethyl acetate, to give 1-chloro-4-(4-fluorophenyl)-8-iodophthalazine (0.11 g).
- ¹H-NMR(CDCl₃): : δ 7.28 (2H, t), 7.47 (1H, t), 7.73 (2H, dd), 8.02 (1H, d), 8.41 (1H, d)

### Reference Example 92

A solution of methyl 2-chloroformyl-6-nitrobenzoate (12.2 g) in 1,2-dichloroethane (40 ml) was added dropwise, under ice-cooling, to a suspension of anhydrous aluminum chloride (14.7 g) in toluene (53 ml). The mixture was stirred for 30 minutes under the same conditions. Then, the ice-bath was removed, and the reaction mixture was stirred for two hours at room temperature. The reaction mixture was poured into cold water containing conc. hydrochloric acid (11 ml). To the mixture was added ethyl acetate, which was stirred for 30 minutes. The organic layer was washed with an aqueous saline solution, dried (anhydrous magnesium sulfate) and concentrated under reduced pressure. The concentrate was purified by means of a silica gel column chromatography, eluting with ethyl acetate/hexane, to give methyl 2-(4-methylbenzoyl)-6-nitrobenzoate (10.3 g).
- mp: : 97-98°C
- ¹H-NMR(CDCl₃): : δ 2.45 (3H, s), 3.77 (3H, s), 7.30 (2H, t), 7.69 (2H, dd), 7.78 (1H, dd), 8.20 (1H, dd)

### Reference Example 93

A mixture of methyl 2-(4-methylbenzoyl)-6-nitrobenzoate (0.30 g) and hydrazine hydrate (0.11 g) was heated for 5 hours in ethanol (4 ml) under reflux. The reaction mixture was cooled in air, then, the resulting crystalline precipitate was collected by filtration, which was washed with isopropyl ether and dried to give 4-(4-methylphenyl)-8-nitro-(2H)phthalazin-1-one (0.23 g).
- ¹H-NMR(CDCl₃): : δ 2.47 (3H, s), 7.36 (2H, d), 7.44 (2H, d), 7.76 (1H, dd), 7.88 (1H, t), 7.96 (1H, dd)

### Reference Example 94

Phosphorus oxychloride (0.71 ml) and dimethylaniline (0.20 ml) were added to 4-(4-methylphenyl)-8-nitro-(2H)phthalazin-1-one (0.22 g). The mixture was stirred for two hours at 110 °C. The reaction mixture was concentrated under reduced pressure. To the concentrate were added chloroform (30 ml) and an aqueous solution of sodium hydrogencarbonate (20 ml). The mixture was shaken and, then, left standing to form two layers. The lower layer was washed with an aqueous saline solution, dried (anhydrous magnesium sulfate) and concentrated under reduced pressure. The concentrate was purified by means of a silica gel chromatography, eluting with ethyl acetate/hexane, to give 1-chloro-4-(4-(4-methylphenyl)-8-nitrophthalazine (71 mg).
- mp: : 159-161°C
- ¹H-NMR(CDCl₃): : δ 2.50 (3H, s), 7.42 (2H, d), 7.61 (2H, d), 7.98 (1H, t), 8.09 (1H, dd), 8.33 (1H, dd)

### Reference Example 95

Methyl 2-(4-methylbenzoyl)-6-nitrobenzoate (5.70 g) was dissolved in ethyl acetate (150 mg). To the solution was added 10% Pd-C (0.57 g), which was subjected to catalytic reduction for 12 hours under atmospheric pressure. The catalyst was filtered off through celite and washed with ethyl acetate. The filtrate was concentrated under reduced pressure. The concentrate was crystallized from isopropyl ether to give methyl 6-amino-2-(4-methylbenzoyl)benzoate.
- mp: : 92-93°C
- ¹H-NMR(CDCl₃): : δ 2.40 (3H, s), 3.42 (3H, s), 5.67 (2H, broad), 6.63 (1H, dd), 6.79 (1H, dd), 7.22 (2H, d), 7.31 (1H, dd), 7.67 (2H, d)

### Reference Example 96

Methyl 6-amino-2-(4-methylbenzoyl)benzoate (4.10 g) was dissolved in acetone (40 ml). To the solution were added conc. hydrochloric acid (10.5 ml) and water (10 ml). To the mixture was added dropwise, on an ice-bath, a solution of sodium nitrite (1.15 g) in water (5.5 ml). The whole mixture was stirred for 0.5 hour under the same conditions. The reaction mixture was cooled to -25 °C, to which was added dropwise a solution of sodium iodide (2.51 g) in water (7.5 ml). The whole mixture was stirred for 0.5 hour under the same conditions. The ice-bath was removed, and the reaction mixture was warmed up to room temperature, to which was added water (40 ml). The mixture was concentrated under reduced pressure, which was subjected to extraction with ethyl acetate. The ethyl acetate layer was washed with an aqueous saline solution, dried (anhydrous magnesium sulfate) and concentrated under reduced pressure. The concentrate was purified by means of a silica gel column chromatography, eluting with ethyl acetate/hexane, to give methyl 6-iodo-2-(4-methylbenzoyl)benzoate (4.88 g) as a pale yellow oily product.
- ¹H-NMR(CDCl₃): : δ 2.43 (3H,s), 3.72 (3H, s), 7.19 (2H, d), 7.31 (1H, t), 7.50 (1H, dd), 7.68 (2H, d), 8.05 (1H, dd)

### Reference Example 97

A mixture of methyl 6-iodo-2-(4-methylbenzoyl)-6-nitrobenzoate (1.33 g) and hydrazine hydrate (0.39 g) was heated under reflux in ethanol (15 ml) for 8 hours. The reaction mixture was cooled in air and, then, the resulting crystalline precipitate was collected by filtration, washed with isopropyl ether and dried to give 8-iodo-4-(4-methylphenyl)-(2H)phthalazin-1-one (0.68 g).
- ¹H-NMR(CDCl₃): : δ 2.45 (3H, s), 7.27-7.38 (3H, m), 7.42 (2H, d), 7.73 (1H, dd), 8.40 (1H, d)

### Reference Example 98

Phosphorus oxychloride (0.94 ml) and dimethylaniline (0.17 ml) were added to 8-iodo-4-(4-methylphenyl)-(2H)phthalazin-1-one (0.25 g). The mixture was stirred for one hour at 110 °C. The reaction mixture was concentrated under reduced pressure. To the concentrate were added chloroform and solution of sodium hydrogencarbonate. The mixture was shaken and, then, left standing to form two layers. The lower layer was washed with an aqueous saline solution, dried (anhydrous magnesium sulfate) and concentrated under reduced pressure. The concentrate was purified by means of a silica gel colum chromatography, eluting ethyl acetate/hexane, to give 1-chloro-8-iodo-4-(4-methylphenyl)phthalazine (0.18 g).
- mp: : 95-97°C
- ¹H-NMR(CDCl₃): : δ 2.47 (3H, s), 7.37 (2H, d), 7.44 (1H, t), 7.55 (2H, d), 8.08 (1H, d), 8.73 (1H, d)

### Reference Example 99

DMF (3 ml) was added to a mixture of 8-iodo-4-(4-methylphenyl)-(2H)phthalazin-1-one (0.42 g) and cuprous cyanide (0.12 g). The whole mixture was stirred for 30 minutes at 90 °C. The reaction mixture was cooled in air, to which were added ethyl acetate and water. Insolubles were filtered off through celite. The filtrate was shaken and,, then, left standing to form two layers. The ethyl acetate layer was washed with an aqueous saline solution, dried (anhydrous magnesium sulfate) and concentrated under reduced pressure. The resulting crystalline precipitate was collected by filtration, washed with isopropyl ether and dried to give 8-cyano-4-(4-methylphenyl)-(2H)phthalazin-1-one (0.26 g).
- ¹H-NMR(CDCl₃): : δ 2.46 (3H, s), 7.33 (2H, d), 7.44 (2H, d), 7.86 (1H, t), 8.01 (1H, d), 8.15 (1H, d)

### Reference Example 100

Phosphorus oxychloride (1.30 ml) and dimethylaniline (0.24 ml) were added to 8-cyano-4-(4-methylphenyl)-(2H)phthalazin-1-one (0.25 g). The mixture was stirred for one hour at 110 °C. The reaction mixture was concentrated under reduced pressure. To the concentrate were added chloroform and cold water. The mixture was shaken and, then, left standing to form two layers. The lower layer was washed with an aqueous saline solution, dried (anhydrous magnesium sulfate) and concentrated under reduced pressure. The concentrate was purified by means of a silica gel column chromatography, eluting ethyl acetate/hexane, to give 1-chloro-8-cyano-4-(4-methylphenyl)phthalazine (0.19 g).
- mp: : 184-186°C
- ¹H-NMR(CDCl₃): : δ 2.50 (3H, s), 7.41 (2H, d), 7.58 (2H, d), 7.93-8.00 (2H, m), 8.43 (1H, dd)

### Reference Example 101

DMF (30 ml) and toluene (10 ml) were added to a mixture of methyl 6-iodo-2-(4-methylbenzoyl)benzoate (3.04 g), sodium trifluoroacetate (8.53 g) and cuprous iodide (7.31 g). The whole mixture was subjected to azeotropic distillation for 1.5 hour at 150 °C to eliminate the moisture in the reaction system. Then, toluene was distilled off, and the remainder was heated under reflux at 165 °C for 8 hours. The reaction mixture was cooled in air, to which were added ethyl acetate and water. Insolubles were filtered off through celite. The filtrate was shaken and, then, left standing to form two layers. The upper layer was washed with an aqueous saline solution, dried (anhydrous magnesium sulfate) and concentrated under reduced pressure. The residue was purified by means of a silica gel column chromatography, eluting with ethyl acetate/hexane, to give methyl 6-trifluoromethyl-2-(4-methylbenzoyl)benzoate (1.89 g) as a pale yellow oily product.
- ¹H-NMR(CDCl₃): : δ 2.44 (3H, s), 3.72 (3H, s), 7.29 (2H, d), 7.56-7.71 (4H, m), 7.88 (1H, dd)

### Reference Example 102

A mixture of methyl 6-trifluoromethyl-2-(4-methylbenzoyl)benzoate (1.88 g) and hydrazine hydrate (0.64 g) was heated for 8 hours under reflux in ethanol (25 ml). The reaction mixture was cooled in air. which was then concentrated under reduced pressure. The concentrate was purified by means of a silica gel column chromatography, eluting with ethyl acetate/hexane, to give 8-trifluoromethyl-4-(4-methylphenyl)-(2H)phthalazin-1-one (1.29 g).
- ¹H-NMR(CDCl₃): : δ 2.47 (3H, s), 7.35 (2H, d), 7.43 (2H, d), 7.85 (1H, t), 8.04 (1H, d), 8.20 (1H, d)

### Reference Example 103

Phosphorus oxychloride (5.66 ml) and dimethylaniline (1.06 ml) were added to 8-trifluoromethyl-4-(4-methylphenyl)-(2H)phthalazin-1-one (1.27 g). The mixture was stirred for two hours at 110 °C. The reaction mixture was cooled in air, which was then concentrated under reduced pressure. To the concentrate were added chloroform and an aqueous solution of sodium hydrogencarbonate. The lower layer was washed with an aqueous saline solution, dried (anhydrous magnesium sulfate and concentrated under reduced pressure. The concentrate was purified by means of a silica gel chromatography, eluting with ethyl acetate/hexane, to give 1-chloro-8-trifluoromethyl-4-(4-methylphenyl)phthalazine (0.65 g).
- mp: : 119-121°C
- ¹H-NMR(CDCl₃): : δ 2.49 (3H, s), 7.36 (2H, d), 7.57 (2H, d), 7.96 (1H, t), 8.35 (1H, d), 8.45 (1H, d)

### Reference Example 104

A mixture of methyl 2-chloroformyl-6-nitrobenzoate (4.88 g) and tributyl(4-chlorophenyl)tin (b.p.145-155 °C/0.2 mmHg; 10.0 g) was dissolved in toluene (40 ml). To the solution was added Pd(Ph₃P)₂Cl₂ (70 mg). The mixture was stirred for 1.5 hour at 70 °C under argon atmosphere. The reaction mixture was cooled in air, which was concentrated under reduced pressure. The concentrate was purified by means of a silica gel chromatography, eluting with ethyl acetate/hexane, to give methyl 2-(4-chlorobenzoyl)-6-nitrobenzoate (3.55 g).
- mp: : 93-94°C
- ¹H-NMR(CDCl₃): : δ 3.78 (3H, s), 7.48 (2H, d), 7.67-7.79 (4H, m), 8.22 (1H, dd)

### Reference Example 105

Methyl 2-(4-chlorobenzoyl)-6-nitrobenzoate (1.20 g) was dissolved in acetic acid (20 ml). To the solution was added reduced iron (2.10 g), little by little, over two hours. The whole mixture was stirred for further 12 hours at room temperature. The reaction mixture was concentrated under reduced pressure. To the concentrate were added ethyl acetate and water. Insolubles were filtered off through celite. The filtrate was shaken and, then, left standing to form two layers. The ethyl acetate layer was washed with an aqueous solution of sodium hydrogencarbonate and an aqueous saline solution, successively, dried (anhydrous magnesium sulfate) and concentrated under reduced pressure. The concentrate was purified by means of a silica gel chromatography, eluting with ethyl acetate/hexane, to give methyl 6-amino-2-(4-chlorobenzoyl)benzoate (1.00 g).
- mp: : 109-110°C
- ¹H-NMR(CDCl₃): : δ 3.44 (3H, s), 5.71 (2H, broad), 6.61 (1H, dd), 6.81 (1H, dd), 7.33 (1H, t), 7.40 (2H, d), 7.72 (2H, d)

### Reference Example 106

Methyl 6-amino-2-(4-chlorobenzoyl)benzoate (0.98 g) was dissolved in acetone (10 ml), to which was added a 47% aqueous solution of hydrogen bromide (4.5 g). The mixture was cooled to -5 °C, to which was added dropwise a solution of sodium nitrite (0.25 g) in water (1.2 ml). The whole mixture was stirred for 9.5 hour under the same conditions. To the reaction mixture was added water (3 ml), which was cooled to -15 °C, followed by adding thereto cuprous oxide (30 mg) little by little. The mixture was stirred for 0.5 hour under the same conditions. The reaction mixture was warmed up to room temperature, to which was added water (10 ml), followed by concentration under reduced pressure. The concentrate was subjected to extraction with ethyl acetate. The extract was washed with an aqueous saline solution, dried (anhydrous magnesium sulfate) and concentrated under reduced pressure. The concentrate was was purified by means of a silica gel chromatography, eluting with ethyl acetate/hexane, to give methyl 6-bromo-2-(4-chlorobenzoyl)benzoate (0.69 g).
- mp: : 73-74°C
- ¹H-NMR(CDCl₃): : δ 3.75 (3H, s), 7.38 (1H, t), 7.44-7.49 (1H, m), 7.46 (2H, d), 7.73 (2H, d), 7.81 (1H, dd)

### Reference Example 107

A mixture of methyl 6-bromo-2-(4-chlorobenzoyl)benzoate (0.67 g) and hydrazine hydrated (0.21 g) was heated in ethanol (13 ml) under reflux. The reaction mixture was cooled in air, and, then, the resulting crystalline precipitate was collected by filtration, washed with ethanol and isopropyl ether, successively and dried to give 8-bromo-4-(4-chlorophenyl)-(2H)phthalazin-1-one (0.58 g).
- ¹H-NMR(DMSO-d₆): : δ 7.56-7.75 (6H, m), 8.10 (1H, dd)

### Reference Example 108

Phosphorus oxychloride (2.31 ml) and dimethylaniline (0.43 ml) were added to 8-bromo-4-(4-chlorophenyl)-(2H)phthalazin-1-one. The mixture was stirred for one hour at 110 °C. The reaction mixture was concentrated under reduced pressure. To the concentrate were added chloroform (30 ml) and an aqueous solution of sodium hydrogencarbonate (20 ml). The mixture was shaken and, then, left standing to form two layers. The lower layer was washed with an aqueous saline solution, dried (anhydrous magnesium sulfate) and concentrated under reduced pressure. The concentrate was purified by means of a silica gel column chromatography, eluting with ethyl acetate/hexane, to give 8-bromo-1-chloro-4-(4-chlorophenyl)phthalazine (0.27 g).
- mp: : 191-192°C
- ¹H-NMR(CDCl₃): : δ 7.56 (2H, d), 7.63 (2H, d), 7.69 (1H, d), 8.00 (1H, d), 8.33 (1H, d)

### Reference Example 109

A mixture of methyl 2-chloroformyl-6-nitrobenzoate (4.88 g) and tributyl (4-fluorophenyl)tin (b.p.140-150 °C/0.2 mmHg; 9.24 g) was dissolved in toluene (50 ml). To the solution was added Pd(Ph₃P)₂Cl₂ (70 mg). The mixture was stirred for 1.5 hour at 70 °C under argon atmosphere. The reaction mixture was cooled in air, which was then concentrated under reduced pressure. The concentrate was purified by means of a silica gel chromatography, eluting with ethyl acetate/hexane, to give methyl 2-(4-fluorobenzoyl)-6-nitrobenzoate (3.36 g).
- mp: : 106-107°C

### Reference Example 110

Methyl 6-amino-2-(4-fluorobenzoyl)benzoate (0.76 g) was dissolved in acetone, to which was added a 47% aqueous solution of hydrogen bromide (3.1 g). The mixture was cooled to -5 °C, to which was added dropwise a solution of sodium nitrite (0.17 g) in water (0.8 ml). The whole mixture was stirred for 0.5 hour under the same conditions. Water (0.8 ml) was to the reaction mixture, which was cooled to -15 °C. To the reaction mixture was added cuprous oxide (20 mg), little by little, and the mixture was stirred for 0.5 hour under the same conditions. The reaction mixture was warmed up to room temperature, to which was added water (6 ml), followed by concentration under reduced pressure. The concentrate was subjected to extraction with ethyl acetate. The extract was washed with an aqueous saline solution, dried (anhydrous magnesium sulfate) and concentrated under reduced pressure. The concentrate was purified by means of a silica gel column chromatography, eluting with ethyl acetate/hexane, to give methyl 6-bromo-2-(4-fluorobenzoyl)benzoate (0.60 g) as a pale yellow oily product.
- ¹H-NMR(CDCl₃): : δ 3.74 (3H, s), 7.16 (2H, d), 7.38 (1H, t), 7.47 (1H, dd), 7.78-7.85 (3H, m)

### Reference Example 111

A mixture of methyl 6-bromo-2-(4-fluorobenzoyl)benzoate (0.60 g) and hydrazine hydrate (0.20 g) was heated for 6 hour under reflux in ethanol (12 ml). The reaction mixture was cooled in air and, then, the resulting crystalline precipitate was collected by filtration, which was washed with ethanol and isopropyl ether, successively, and dried to give 8-bromo-(4-fluorophenyl)-(2H)phthalazin-1-one (0.53 g).
- ¹H-NMR(CDCl₃): : δ 7.22 (2H, t), 7.52 (2H, dd), 7.59 (1H, t), 7.63 (1H, dd), 8.03 (1H, dd)

### Reference Example 112

Phosphorus oxychloride (2.17 ml) and dimethylaniline (0.41 ml) were added to 8-bromo-4-(4-fluorophenyl)-(2H)phthalazin-1-one (0.51 g), which was stirred for one hour at 110 °C. The reaction mixture was concentrated under reduced pressure. To the concentrate were added chloroform (30 ml) and an aqueous solution of sodium hydrogencarbonate (20 ml). The mixture was shaken and, then, left standing to form two layers. The lower layer was washed with an aqueous saline solution, dried (anhydrous magnesium sulfate) and concentrated under reduced pressure. The concentrate was purified by means of a silica gel column chromatography, eluting with ethyl acetate/hexane, to give 8-bromo-1-chloro-4-(4-fluorophenyl)phthalazine (0.34 g).
- mp: : 213-214°C
- ¹H-NMR(CDCl₃): : δ 7.28 (2H, t), 7.66 (2H, dd), 7.69 (1H, t), 8.01 (1H, d), 8.33 (1H, d)

### Reference Example 113

DMF (21 ml) and toluene (7 ml) were added to a mixture of methyl 6-iodo-2-(4-fluorobenzoyl)benzoate (1.90 g), sodium pentafluoropropionate (5.52 g) and cuprous iodide (4.53 g). The whole mixture was subjected to azeotropic distillation for 1.5 hour at 150 °C to eliminate the moisture in the reaction system. Then, toluene was distilled off, and the residue was heated for 20 hours under reflux at 165 °C. The reaction mixture was cooled in air, to which were added ethyl acetate and water. The resulting insolubles were filtered off through celite. The filtrate was shaken and, then, left standing to form two layers. The upper layer was washed with an aqueous saline solution, dried (anhydrous magnesium sulfate), and concentrated under reduced pressure. The concentrate was purified by means of a silica gel column chromatography, eluting with ethyl acetate/hexane, to give methyl 2-(4-fluorobenzoyl)-6-pentafluoroethylbenzoate (1.18 g) as a pale yellow oily product.
- ¹H-NMR(CDCl₃): : δ 3.76 (3H, s), 7.16 (2H, t), 7.79 (2H, dd), 7.91-8.01 (2H, m), 8.19 (1H, m)

### Reference Example 114

A mixture of methyl 2-(4-fluorobenzoyl)-6-pentafluoroethyl benzoate (0.69 g) and hydrazine hydrate (0.18 g) was heated for 5 hours under reflux in ethanol (12 ml). The reaction mixture was cooled in air, which was concentrated under reduced pressure. The concentrate was purified by means of a silica gel column chromatography, eluting with ethyl acetate/hexane, to give 4-(4-fluorophenyl)-8-pentafluoroethyl-(2H)phthalazin-1-one (0.35 g).
- ¹H-NMR(CDCl₃): : δ 7.24 (2H, t), 7.53 (2H, dd), 7.84-7.96 (2H, m), 8.16 (1H, dd), 10.12 (1H, broad)

### Reference Example 115

Phosphorus oxychloride (1.25 ml) and dimethylaniline (0.23 ml) were added to 4-(4-fluorophenyl)-8-pentafluoroethyl-(2H)phthalazin-1-one (0.33 g), which was stirred for one hour at 110 °C. The reaction mixture was concentrated under reduced pressure. To the concentrate were added ethyl acetate (30 ml) and an aqueous solution of sodium hydrogencarbonate (20 ml). The mixture was shaken, and, then, left standing to form two layers. The upper layer was washed with an aqueous saline solution, dried (anhydrous magnesium sulfate) and concentrated under reduced pressure. The concentrate was purified by means of a silica gel column chromatography, eluting with ethyl acetate/hexane, to give 4-(4-fluorophenyl)-8-pentafluoroethylphthalazine (0.34 g).
- ¹H-NMR(CDCl₃): : δ 7.30 (2H, t), 7.68 (2H, dd), 7.92-8.05 (2H, m), 8.31 (1H, d)

### Reference Example 116

Maleic anhydride (9.24 g) was added to 2-methylfuran (10.8 g), which was stirred for two hours at room temperature. The resulting crystalline precipitate was collected by filtration, washed with hexane/benzene (2:1) and dried to give a Diels-Alder adduct (14.2 g; m.p.75-76 °C). This adduct (13.4 g) was dissolved in nitromethane (20 ml), to which was added dropwise under ice-cooling chlorosulfonic acid (15 ml). The mixture was stirred for 30 minutes under the same conditions. The reaction mixture was poured into ice-water, to which was added ethyl acetate. The mixture was shaken and, then, left standing to form two layers The ethyl acetate layer was washed with water, dried (anhydrous magnesium sulfate) and concentrated under reduced pressure. The resulting crystalline precipitate was collected by filtration, washed with ethyl acetate and dried to give 3-methyl phthalic anhydride (2.34 g) as colorless prisms.
- mp: : 100-101°C
- IR (Nujol): : 1840, 1765 (cm⁻¹)
- ¹H-NMR(CDCl₃): : δ 2.74 (3H, s), 7.67 (1H, dd), 7.77 (1H, t), 7.84 (1H, dd)

### Reference Example 117

Dry tetrahydrofuran (6 ml) was added to a mixture of 2-bromotoluene (1.37 g) and magnesium (0.25 g), which was stirred for one hour at 60 °C under argon atmosphere. On the other hand, a solution of 3-methyl phthalic anhydride (1.34 g) in dry tetrahydrofuran (5 ml) was cooled to -5 °C, to which was added dropwise, under argon atmosphere, the above-mentioned Grignard reagent. The ice-bath was removed, then the reaction mixture was warmed up to room temperature. To the reaction mixture was added 2N hydrochloride (5 ml). The whole mixture was stirred for 30 minutes, to which was added ethyl acetate and water. The mixture was shaken and, then, left standing to form two layers. The ethyl acetate layer was washed with water, dried (anhydrous sodium sulfate) and concentrated under reduced pressure to give an oily product. This product was dissolved in DMF (5 ml), to which were added potassium carbonate (1.4 g) and methyl iodide (1 ml), followed by stirring for 13 hours at room temperature. To the reaction mixture were added ethyl acetate (30 ml), hexane (20 ml) and water (50 ml). The mixture was shaken and, then, left standing to form two layers. The upper layer was washed with water, dried (anhydrous sodium sulfate) and concentrated under reduced pressure to give an oily product. This product was dissolved in ethanol (10 ml), to which was added hydrazine hydrate (350 mg). The mixture was heated for 13 hours under reflux. The reaction mixture was cooled in air and, then, the resulting crystalline precipitate was collected by filtration, washed with a small volume of ethanol and, then, dried to give 8-methyl-4-(2-methylphenyl)-(2H)phthalazin-1-one (1.00 g) as pale yellow prisms.
- mp: : 221-222°C
- IR (Nujol): : 1660 (cm⁻¹)
- ¹H-NMR(DMSO-d₆): : δ 2.14 (3H, s), 3.01 (3H, s), 7.12 (1H, dd), 7.25-7.46 (5H, m), 10.08 (1H, broad)

### Reference Example 118

Phosphorus oxychloride (3.5 g) and dimethylaniline (1.8 g) were added to 8-methyl-4-(2-methylphenyl)-(2H)phthalazin-1-one (940 mg), which was stirred for two hours at 110 °C. The reaction mixture was concentrated under reduced pressure. To the concentrate were added ethyl acetate and cold water. The mixture was shaken and, then, left standing to form two layers. The upper layer was washed with water, dried (anhydrous sodium sulfate) and concentrated under reduced pressure. The concentrate was washed with ethyl acetate and dried to give 1-chloro-8-methyl-4-(2-methylphenyl)phthalazine (870 mg).
- mp: : 178-179°C
- IR (Nujol): : 1450, 1360, 1340, 1275 (cm⁻¹)
- ¹H-NMR(CDCl₃): : δ 2.08 (3H, s), 3.13 (3H, s), 7.31-7.50 (5H, m), 7.64-7.80 (2H, m)

### Example 1 (Production of Compound 1)

To a solution of 1-chloro-4-phenylphthalazine (mp 153-154°C; 6.04 g) in 1,2-dimethoxyethane (100 ml) was added guanidine (3.54 g), followed by stirring at 95°C for 20 hours. The reaction mixture was cooled to room temperature. The crystal precipitated during the reaction was collected by filtration, wahsed with water and 1,2-dimethoxyethane in that order, and dried to yield 4-phenylphthalazin-1-ylguanidine (Compound 1) (5.03 g) as a colorless powdery crystal.
- mp: : 263-264°C
- IR (Nujol): : 3345, 1640, 1595, 1540, 1485, 1415 (cm⁻¹)
- ¹H-NMR(DMSO-d₆): : δ 7.32 (4H, broad), 7.53-7.68 (5H, m), 7.70-7.88 (3H, m), 8.64-8.68 (1H, m)

### Example 2 (Production of hydrochloride of Compound 1)

To a suspension of 4-phenylphthalazin-1-ylguanidine (5.00 g) in water (60 ml) was added 2N hydrochloric acid (25 ml), and the mixture was heated to dissolve after which it was cooled to room temperature. The crystal precipitated was collected by filtration and recrystallized from water (60 ml) to yield 4-phenylphthalazin-1-ylguanidine hydrochloride (hydrochloride of Compound 1) (3.07 g) as a colorless needle crystal.
mp : 242-243°C
IR (Nujol) : 3310, 1705, 1625, 1390, 1380 (cm⁻¹)
¹H-NMR(DMSO-d₆) : δ 7.63 (5H, broad), 8.07 (4H, broad), 8.10-8.95 (4.5H, broad), 11.65 (0.5H, broad)

| Elemental analysis for C₁₅H₁₃N₅·HCl·2 1/2H₂O | | | |
|---|---|---|---|
| Calcd. | C, 52.25; | H, 5.55; | N, 20.31 |
| Found | C, 52.46; | H, 5.50; | N, 20.60 |

### Example 3 (Production of Compound 2)

To 1-chloro-4-(4-methylphenyl)phthalazine (880 mg) and guanidine (640 mg), was added 1,2-dimethoxyethane (80 ml), followed by stirring at 100°C for 2 hours and at 85°C for 15 hours. The reaction mixture was cooled in air. The crystal precipitated was collected by filtration, washed successively with water and ethyl acetate, and dried to yield 4-(4-methylphenyl)phthalazin-1-ylguanidine (compound 2) (700 mg) as a colorless powdery crystal.
mp : 280-282°C
IR (Nujol) : 3425, 3325, 3160, 1650, 1590, 1535, 1410 (cm⁻¹)
¹H-NMR(DMSO-d₆) : δ 2.42 (3H, s), 7.18 (4H, broad), 7.36 (2H, d), 7.54 (2H, d), 7.70-7.85 (3H, m), 8.63-8.68 (1H, m)

| Elemental analysis for C₁₆H₁₅N₅ | | | |
|---|---|---|---|
| Calcd. | C, 69.30; | H, 5.45; | N, 25.25 |
| Found | C, 69.57; | H, 5.50; | N, 25.16 |

### Example 4 (Production of Compound 3)

To 8-chloro-5-(4-methylphenyl)pyrido[2,3-d]pyridazine (880 mg) and guanidine (480 mg), were added 1,2-dimethoxyethane (15 ml) and methanol (1.5 ml), followed by stirring at room temperature for 13 hours. The reaction mixture was concentrated under reduced pressure and water (15 ml) was added to the residue. The insoluble precipitate was collected by filtration and dried to yield 800 ml of a light-yellow powder. This powder was dispersed in water (20 ml) and 2N hydrochloric acid (1.7 ml) was added. The insoluble substances were filtered off. The filtrate was concentrated under reduced pressure and ethanol (5 ml) was added to the residue. The crystal precipitated was collected by filtration and dried to yield 5-(4-methylphenyl)pyrido[2,3-d]pyridazin-8-ylguanidine hydrochloride (Compound 3) (300 mg) as a colorless prismatic crystal.
mp : 289-290°C
IR (Nujol) : 3195, 1685, 1610, 1590, 1570, 1530, 1410 (cm⁻¹)
¹H-NMR(DMSO-d₆) : δ 7.46 (2H, d), 7.65 (2H, d), 8.14 (1H, dd), 8.51 (1H, dd), 8.70 (2H, broad), 9.24 (2H, broad), 9.39 (1H, dd)

| Elemental analysis for C₁₅H₁₄N₆·9/5HCl·1/5EtOH·2/3H₂O | | | |
|---|---|---|---|
| Calcd. | C, 50.65; | H, 5.06; | N, 23.01 |
| Found | C, 50.53; | H, 5.31; | N, 23.14 |

### Example 5 (Production of compound 4)

To 1-chloro-8-iodo-4-phenylphthalazine (500 mg) and guanidine (207 mg) was added 1,2-dimethoxyethane (10 ml), followed by stirring at 95°C for 3 hours. After the reaction mixture was cooled in air, the crystal precipitated was collected by filtration and washed successively with water and 1,2-dimethoxyethane. To a suspension of the crystal in a mixed solvent (10 ml) of tetrahydrofuran/ethanol (7/3) was added 2N hydrochloric acid (1 ml) and the mixture was heated to dissolve. This solution was concentrated under reduced pressure until the liquid volume became about one-third; the crystal precipitated was collected by filtration and dried to yield 8-iodo-4-phenylphthalazin-1-ylguanidine hydrochloride (Compound 4) (265 mg) as a colorless prismatic crystal.
mp : 228-230°C
IR (Nujol) : 3280, 1685, 1630, 1595 (cm⁻¹)
¹H-NMR(DMSO-d₆) : δ 7.54-7.61 (6H, m), 7.74 (1H, d), 7.93 (4H, broad), 8.63 (1H, dd)

| Elemental analysis for C₁₅H₁₂N₅I·2HCl | | | |
|---|---|---|---|
| Calcd. | C, 38.96; | H, 3.03; | N, 15.15 |
| Found | C, 38.81; | H, 3.13; | N, 15.21 |

### Example 6 (Production of Compound 5)

1,2-Dimethoxyethane (5 ml) was added to a mixture of 1-chloro-4-(4-methoxyphenyl)phthalazine (237 mg) and guanidine (150 mg). The whole mixture was stirred for 9 hours at 100 °C. The reaction mixture was cooled in air, to which was then added water (5 ml), followed by distilling off 1,2-dimethoxyethane under reduced pressure. To the residue were added ethyl acetate (5 ml) and water (5 ml). The resulting insolubles were collected by filtration, washed with water and ethyl acetate, successively and dried to give pale yellow powdery product (159 mg). This powdery product (140 mg) was dissolved in 1N hydrochloric acid (1.5 ml), which was concentrated under reduced pressure. The concentrate was recrystallized from ethanol/ethyl acetate to give 4-(4-methoxyphenyl)phthalazin-1-ylguanidine dihydrochloride (hydrochloride of Compound 5) (154 mg) as pale yellow needles.
mp : 260-261°C
IR (Nujol) : 1695, 1600, 1430 (cm⁻¹)
¹H-NMR(DMSO-d₆) : δ 3.88 (3H, s), 7.18 (2H, d), 7.66 (2H, d), 8.00-8.18 (3H, m), 8.40 (4H, broad), 8.96 (1H, broad)

| Elemental analysis for C₁₆H₁₅N₅O.2HCl·3.5H₂O | | | |
|---|---|---|---|
| Calcd. | C, 44.76; | H, 5.63; | N, 16.31 |
| Found | C, 44.78; | H, 5.37; | N, 16.40 |

### Example 7 (Production of Compound 6)

1,2-Dimethoxyethane (5 ml) was added to a mixture of 1-chloro-4-(2-methoxyphenyl)phthalazine (230 mg) and guanidine (160 mg), which was stirred for 18 hours at 95 °C. The reaction mixture was cooled in air, and, then, 1,2-dimethoxyethane was distilled off. To the residue were added ethyl acetate (5 ml) and water (10 ml). Insolubles were collected by filtration, washed with water and ethyl acetate, successively, and dried to give colorless powdery crystals (170 mg). This powdery product (153 mg) was dissolved in 1N hydrochloric acid (1.5 ml), and the solution was concentrated under reduced pressure. The concentrate was recrystallized from ethanol/ethyl acetate to give 4-(2-methoxyphenyl)phthalazin-1-ylguanidine dihydrochloride (hydrochloride of Compound 6) (188 mg) as yellow prisms.
mp : 263-264°C
IR (Nujol) : 3320, 3060, 1705, 1695, 1600, 1560, 1430, 1375 (cm⁻¹)
¹H-NMR(DMSO-d₆) : δ 3.69 (3H, s), 7.18 (1H, t), 7.27 (1H, d), 7.40 (1H, dd), 7.54-7,65 (2H, m), 7.97-8.12 (2H, m), 8.40 (4H, broad), 8.91 (1H, d)

| Elemental analysis for C₁₆H₁₅N₅O.2HCl·3/4H₂O | | | |
|---|---|---|---|
| Calcd. | C, 50.60; | H, 4.91; | N, 18.44 |
| Found | C, 50.33; | H, 4.77; | N, 18.46 |

### Example 8 (Production of Compound 7)

1,2-Dimethoxyethane (6 ml) was added to a mixture of 1-chloro-4-(2-methylphenyl)phthalazine (115 mg) and guanidine (120 mg), which was stirred for 15 hours at 105 °C. The reaction mixture was cooled in air, and, then, 1,2-dimethoxyethane was distilled off. To the residue were added ethyl acetate (5 ml) and water (10 ml). Insolubles were collected by filtration, washed with water and ethyl acetate, successively, and dried to give pale yellow powdery crystals (90 mg). This powdery product (78 mg) was dissolved in 1N hydrochloric acid (1.0 ml), and the solution was concentrated under reduced pressure. The concentrate was recrystallized from ethanol to give 4-(2-methylphenyl)phthalazin-1-ylguanidine dihydrochloride (hydrochloride of compound 7) (75 mg) as colorless powdery crystals.
mp : 267-268°C
IR (Nujol) : 3340, 3200, 1700, 1570, 1435, 1375 (cm⁻¹)
¹H-NMR(DMSO-d₆) : δ 2.06 (3H, s), 7.33-7.54 (5H, m), 7.98-8.14 (2H, m), 8.35 (4H, broad), 8.90 (1H, broad)

| Elemental analysis for C₁₆H₁₅N₅·2HCl.H₂O | | | |
|---|---|---|---|
| Calcd. | C, 52.18; | H, 5.20; | N, 19.02 |
| Found | C, 52.29; | H, 5.18; | N, 19.21 |

### Example 9 (Production of Compound 8)

1,2-Dimethoxyethane (8 ml) was added to a mixture of 1-chloro-4-(3-nitrophenyl)phthalazine (213 mg) and guanidine (190 mg), which was stirred for 20 hours at 105 °C. The supernatant was taken out, from which was distilled off 1,2-dimethoxyethane. To the residue were added ethyl acetate (5 ml) and water (10 ml). Insolubles were collected by filtration, washed with water and ethyl acetate, successively, and dried to give yellow powdery crystals (153 mg). This powdery product (122 mg) was dissolved in 1N hydrochloric acid (1.0 ml), which was concentrated under reduced pressure. The concentrate was recrystallized from ethanolic water to give 4-(3-nitrophenyl)phthalazin-1-ylguanidine hydrochloride (hydrochloride of Compound 8) (77 mg) as pale yellow powdery crystals.
mp : 271-272°C
IR (Nujol) : 3260, 1710, 1620, 1535, 1390, 1345 (cm⁻¹)
¹H-NMR(DMSO-d₆) : δ 7.93 (1H, broad), 8.17 (4H, broad), 8.48 (2H, broad), 9.11 (1H, broad), 9.40 (4H, broad)

| Elemental analysis for C₁₅H₁₂N₆O₂·HCl·0.5H₂O | | | |
|---|---|---|---|
| Calcd. | C, 50.93; | H, 3.99; | N, 23.76 |
| Found | C, 50.91; | H, 3.97; | N, 23.49 |

### Example 10 (Production of Compound 9)

Guanidine (180 mg) was added, under ice-cooling, a solution of 1-chloro-7-nitro-4-phenylphthalazine (286 mg) in 1,2-dimethoxyethane (20 ml), which was stirred for 2.5 hours, followed by stirring for further two hours at room temperature. The supernatant was taken out, from which 1,2-dimethoxyethane was distilled off. To the residue were added ethyl acetate (10 ml) and water (8 ml). The insoluble matter was collected by filtration, which was purified by means of a silica gel column chromatography [eluting solvent: hexane/ethyl acetate/methanol (6:6:1)] to give pale yellow powdery crystals (77 mg). This powdery product (77 mg) was recrystallized from a mixture of 1N hydrochloric acid (0.8 ml) and ethanol (1 ml) to give 7-nitro-4-phenylphthalazin-1-ylguanidine hydrochloride (hydrochloride of compound 9) (38 mg) as yellow powdery prisms.
mp : 261-262°C
IR (Nujol) : 3270, 1685, 1615, 1580, 1535, 1405, 1340 (cm⁻¹)
¹H-NMR(DMSO-d₆) : δ 7.66 (5H, s), 8.21 (4H, broad), 8.74 (1H, d), 9.10 (1H, broad), 9.83 (1H, broad), 11.83 (1H, broad)

| Elemental analysis for C₁₅H₁₂N₆O₂·HCl | | | |
|---|---|---|---|
| Calcd. | C, 52.26; | H, 3.80; | N, 24.38 |
| Found | C, 52.15; | H, 3.77; | N, 24.28 |

### Example 11 (Production of Compound 10)

1,2-Dimethoxyethane (8 ml) was added to a mixture of 1,8-dichloro-4-phenylphthalazine (213 mg) and guanidine (100 mg), which was stirred for 20 hours at 100-105 °C. From the reaction mixture, 1,2-dimethoxyethane was distilled off under reduced pressure. To the residue were added ethyl acetate (5 ml) and water (10 ml). The resulting insoluble matter was collected by filtration, washed with water and ethyl acetate, successively, and dried to give pale yellow powdery crystals (170 mg). This powdery product (150 mg) was dissolved in a mixture of ethanol (3 ml) and 2N hydrochloric acid (0.5 ml), which was concentrated to dryness. The concentrate was recrystallized from ethanol to give 8-chloro-4-phenylphthalazin-1-ylguanidine dihydrochloride (hydrochloride of Compound 10) (150 mg) as pale yellow prisms.
- mp: : 233-234°C
- IR (Nujol): : 3200, 3085, 1690, 1600, 1555, 1370, 1335 (cm⁻¹)
- ¹H-NMR(DMSO-d₆): : δ 7.62 (5H, s), 7.79 (1H, d), 7.95 (1H, t), 8.03 (4H, broad), 8.11 (1H, d)

### Example 12 (Production of Compound 11)

To a solution of 1-chloro-8-nitro-4-phenylphthalazine (304 mg) in 1,2-dimethoxyethane (35 ml) was added, under ice-cooling, to a solution of guanidine (220 mg) in 1,2-dimethoxyethane (1 ml)/DMF (1 ml). The mixture was stirred for 0.5 hour under the same conditions, which was then warmed up to room temperature. 1,2-Dimethoxyethane was distilled off under reduced pressure. To the residue were added ethyl acetate (40 ml) and water (40 ml). The mixture was shaken and, then, left standing to form two layers. The upper layer was washed with ethyl acetate and dried to give yellow powdery crystals (43 mg). This powdery product was dissolved in a mixture of methanol (3 ml) and 2N hydrochloric acid (0.15 ml). The solution was concentrated to dryness under reduced pressure. The concentrate was recrystallized from ethanol to give 8-nitro-4-phenylphthalazin-1-ylguanidine hydrochloride (hydrochloride of Compound 11) (29 mg).
mp : 216-217°C
IR (Nujol) : 3320, 3160, 1620, 1535 (cm⁻¹)
¹H-NMR(DMSO-d₆) : δ 7.63 (5H, s), 7.95 (1H, d), 8.00 (4H, broad), 8.14 (1H, t), 8.28 (1H, d)

| Elemental analysis for C₁₅H₁₂N₆O₂·HCl·3/4H₂O | | | |
|---|---|---|---|
| Calcd. | C, 50.29; | H, 4.08; | N, 23.46 |
| Found | C, 50.10; | H, 4.00; | N, 23.23 |

### Example 13 (Production of Compound 12)

1,2-Dimethoxyethane (8 ml) was added to a mixture of 1-chloro-4,8-diphenylphthalazine (317 mg) and guanidine (140 mg). The mixture was stirred for 24 hours at 100 °C. 1,2-Dimethoxyethane was distilled off under reduced pressure. To the residue were added ethyl acetate (5 ml) and water (10 ml). The resulting insoluble matter was collected by filtration, which was washed with water and ethyl acetate, successively, and dried to to give colorless powdery crystals [m.p.230-231 °C (decomp.); 210 mg)]. This powdery product (200 mg) was dissolved in tetrahydrofuran (6 ml), to which was added 2N hydrochloric acid (0.6 ml). The mixture was concentrated to dryness, which was recrystallized from ethanolic water to give 4,8-diphenylphthalazin-1-ylguanidine dihydrochloride (hydrochloride of Compound 12) (170 mg) as colorless powdery crystals.
mp : 264-265°C
IR (Nujol) : 3055, 1685, 1635, 1590, 1380 (cm⁻¹)
¹H-NMR(DMSO-d₆) : δ 7.37-7.45 (9H, m), 7.61-7.69 (5H, m), 7.81 (1H, d), 7.84 (1H, d), 8.03 (1H, t)

| Elemental analysis for C₂₁H₁₇N₅·2HCl·1/3H₂O | | | |
|---|---|---|---|
| Calcd. | C, 60.30; | H, 4.74; | N, 16.74 |
| Found | C, 60.29; | H, 4.77; | N, 16.81 |

### Example 14 (Production of Compound 13)

1,2-Dimethoxyethane (5 ml) was added to a mixture of 8-bromo-1-chloro-4-phenylphthalazine (639 mg) and guanidine (295 mg), which was stirred for 13 hours at 80 °C. 1,2-Dimethoxyethane was distilled off under reduced pressure. To the residue were added ethyl acetate (10 ml) and water (15 ml). The resulting insoluble matter was collected by filtration, washed with water and ethyl acetate, successively, and dried to give yellow powdery crystals (547 mg). This powdery product (225 mg) was dissolved in a mixture of ethanol (10 ml) and 2N hydrochloric acid (0.7 ml). The solution was concentrated to dryness under reduced pressure. The concentrate was recrystallized from ethanol to give 8-bromo-4-phenylphthalazin-1-ylguanidine dihydrochloride (hydrochloride of Compound 13) (250 mg) as yellow prisms.
mp : 180-181°C
IR (Nujol) : 3300, 3120, 1685, 1600, 1595, 1385, 1365, 1330 (cm⁻¹)
¹H-NMR(DMSO-d₆) : δ 7.61 (5H, s), 7.75-7.87 (2H, m), 7.98 (4H, broad), 8.31 (1H, dd)

| Elemental analysis for C₁₅H₁₂N₅Br·2HCl | | | |
|---|---|---|---|
| Calcd. | C, 43.40; | H, 3.40; | N, 16.87 |
| Found | C, 43.74; | H, 3.49; | N, 16.76 |

### Example 15 (Production of Compound 14)

1,2-Dimethoxyethane (10 ml) was added to a mixture of 6,8-dibromo-1-chloro-7-methoxy-4-phenylphthalazine (1.04 g) and guanidine (354 mg), which was stirred for 14 hours at room temperature. 1,2-Dimethoxyethane was distilled off under reduced pressure. To the residue were added ethyl acetate (100 ml), tetrahydrofuran (10 ml) and water (30 ml). The mixture was shaken and, then, left standing to form two layers. The upper layer was washed with water, which was concentrated under reduced pressure. The concentrate was recrystallized from ethanol to give yellow powdery crystals (510 mg). This powdery product (400 mg) was dissolved in a mixture of ethanol (10 ml) and 2N hydrochloric acid (1.5 ml). The solution was concentrated to dryness, which was recrystallized from ethanol/ethyl acetate to give 6,8-dibromo-7-methoxy-4-phenylphthalazin-1-ylguanidine dihydrochloride (hydrochloride of Compound 14) (370 mg) as yellow prisms.
mp : 248-251°C
IR (Nujol) : 3320, 1690, 1600, 1450, 1355 (cm⁻¹)
¹H-NMR(DMSO-d₆) : δ 3.93 (3H, s), 7.63 (5H, s), 7.92 (5H, broad)

| Elemental analysis for C₁₆H₁₃N₅OBr₂·2HCl·0.5H₂O | | | |
|---|---|---|---|
| Calcd. | C, 36.05; | H, 3.03; | N, 13.14 |
| Found | C, 36.34; | H, 2.82; | N, 13.08 |

### Example 16 (Production of Compound 15)

1,2-Dimethoxyethane (8 ml) was added to a mixture of 6,8-dibromo-7-butoxy-1-chloro-4-phenylphthalazine (676 mg) and guanidine (20 mg), which was stirred for 0.5 hour at 70 °C. From the reaction mixture, 1,2-dimethoxyethane was was distilled off. To the residue were added ethyl acetate (10 ml) and water (20 ml). The resulting insoluble matter was collected by filtration, washed with water and ethyl acetate, successively, and dried to give yellow powdery crystals (430 mg). This powdery product (71 mg) was dissolved in a mixture of ethanol (4 ml) and 2N hydrochloric acid (0.1 ml). The solution was concentrated to dryness under reduced pressure. The concentrate was recrystallized from ethanol/ethyl acetate to give 6,8-dibromo-7-butoxy-4-phenylphthalazin-1-ylguanidine dihydrochloride (hydrochloride of Compound 15) (57 mg) as colorless prisms.
mp : 280°C (dec.)
IR (Nujol) : 3300, 1685, 1600, 1450, 1345, 1330 (cm⁻¹)
¹H-NMR(DMSO-d₆) : δ 0.99 (3H, t), 1.48-1.63 (2H, m), 1.80-1.94 (2H, m), 4.08 (2H, t), 7.62 (5H, s), 7.90 (5H, broad)

| Elemental analysis for C₁₉H₁₉N₅OBr₂·2HCl | | | |
|---|---|---|---|
| Calcd. | C, 40.31; | H, 3.74; | N, 12.37 |
| Found | C, 40.60; | H, 3.73; | N, 12.59 |

### Example 17 (Production of Compound 16)

In tetrahydrofuran/ethanol (1:1) (18 ml) was dissolved 6,8-Dibromo-7-methoxy-4-phenylphthalazin-1-ylguanidine (90 mg). To the solution was added 5% Pd-C (55 mg), and the mixture was stirred for 15 minutes at room temperature in hydrogen streams. The catalyst was filtered off, and the filtrate was concentrated under reduced pressure. The concentrate was washed with ethyl acetate and dried to give 6-bromo-7-methoxy-4-phenylphthalazin-1-ylguanidine bromate (bromate of Compound 16) (63 mg).
mp : 255-256°C
IR (Nujol) : 3300, 1690, 1610, 1450, 1390, 1265 (cm⁻¹)
¹H-NMR(DMSO-d₆) : δ 4.14 (3H, s), 7.63 (5H, s), 7.86-8.90 (6H, m)

| Elemental analysis for C₁₆H₁₄N₅OBr·HBr | | | |
|---|---|---|---|
| Calcd. | C, 42.41; | H, 3.34; | N, 15.46 |
| Found | C, 42.30; | H, 3.27; | N, 15.23 |

### Example 18 (Production of Compound 17)

In tetrahydrofuran/methanol (3:1) (18 ml) was dissolved 6,8-dibromo-7-butoxy-4-phenylphthalazin-1-ylguanidine (99 mg). To the solution was added 5% Pd-C (57 mg), and the mixture was stirred for 30 minutes at room temperature in hydrogen streams. The catalyst was filtered off, and the filtrate was concentrated under reduced pressure. The concentrate was washed with ethyl acetate and dried to give 6-bromo-7-butoxy-4-phenylphthalazin-1-ylguanidine bromate (bromate of Compound 17) (67 mg).
mp : 233-234°C
IR (Nujol) : 3300, 3175, 1690, 1610, 1450, 1390, 1260 (cm⁻¹)
¹H-NMR(DMSO-d₆) : δ 0.99 (3H, t), 1.45-1.62 (2H, m), 1.85 (2H, broad), 4.37 (2H, broad), 7.63 (5H, s), 7.84-8.80 (6H, m)

| Elemental analysis for C₁₉H₂₀N₅OBr·HBr·2/3H₂O | | | |
|---|---|---|---|
| Calcd. | C, 44.99; | H, 4.44; | N, 13.81 |
| Found | C, 44.74; | H, 4.31; | N, 14.21 |

### Example 19 (Production of Compound 18)

1,2-Dimethoxyethane (8 ml) was added to a mixture of 8-bromo-7-butoxy-1-chloro-6-(2-methylphenyl)-4-phenylphthalazine (435 mg) and guanidine (120 mg). The mixture was stirred for 3 hours at 70 °C. Under reduced pressure, 1,2-dimethoxyethane was distilled off. To the residue were added ethyl acetate (60 ml) and water (20 ml). The mixture was shaken and, then, left standing to form two layers. The upper layer was washed with water and concentrated under reduced pressure. To the concentrate was added ethyl acetate/isopropyl ether (1:2) (9 ml). The resulting crystalline precipitate was collected by filtration, which was washed with a small volume of the same mixed solvent, followed by drying to give pale yellow powdery crystals (316 mg). This powdery product (148 mg) was dissolved in a mixture of ethanol (3 ml) and 2N hydrochloric acid (0.4 ml). The solution was concentrated to dryness under reduced pressure. The concentrate was washed with ethyl acetate to give 8-bromo-7-butoxy-6-(2-methylphenyl)-4-phenylphthalazin-1-ylguanidine dihydrochloride (hydrochloride of compound 18) (150 mg) as colorless powdery crystals.
mp : 209-210°C
IR (Nujol) : 3300, 3100, 1690, 1595, 1345 (cm⁻¹)
¹H-NMR(DMSO-d₆) : δ 0.65 (3H, t), 1.07 (2H, broad), 1.35 (2H, broad), 2.11 (3H, s), 3.47 (1H, broad), 3.56 (1H, broad), 7.26-7.37 (4H, m), 7.54-7.64 (6H, m), 7.90 (4H, broad)

| Elemental analysis for C₂₆H₂₆N₅OBr·2HCl·H₂O | | | |
|---|---|---|---|
| Calcd. | C, 52.45; | H, 5.08; | N, 11.76 |
| Found | C, 52.60; | H, 4.88; | N, 11.94 |

### Example 20 (Production of Compound 19)

1,2-Dimethoxyethane (2 ml) was added to a mixture of 1-chloro-8-trifluoromethyl-4-phenylphthalazine (180 mg) and guanidine (88 mg), which was stirred for one hour at 85 °C. The reaction mixture was cooled in air, which was then purified by means of a silica gel column chromatography, eluting with ethyl acetate/hexane/methanol (4:8:1), to give 8-trifluoromethyl-4-phenylphthalazin-1-ylguanidine (55 mg). This powdery product (45 mg) was dissolved in a mixture of ethanol (4 ml) and 2N hydrochloric acid (0.2 ml). The solution was concentrated to dryness under reduced pressure, which was washed with ethyl acetate and dried to give 8-trifluoromethyl-4-phenylphthalazin-1-ylguanidine dihydrochloride (hydrochloride of compound 19) (47 mg) as colorless powdery crystals.
mp : 186-188°C
IR (Nujol) : 3300, 3090, 1690, 1630, 1605, 1590, 1570, 1350, 1300, 1155 (cm⁻¹)
¹H-NMR(DMSO-d₆) : δ 7.62 (5H, s), 7.94 (4H, broad), 8.04 (1H, d), 8.14 (1H, t), 8.46 (1H, d)

| Elemental analysis for C₁₆H₁₂N₅F₃·2HCl | | | |
|---|---|---|---|
| Calcd. | C, 47.54; | H, 3.49; | N, 17.33 |
| Found | C, 47.34; | H, 3.54; | N, 17.05 |

### Example 21 (Production of Compound 20)

1,2-Dimethoxyethane (4 ml) was added to a mixture of 1-chloro-8-methylthio-4-phenylphthalazine (300 mg) and guanidine (148 mg), which was stirred for 14 hours at 70 °C. The reaction mixture was cooled in air, which was concentrated under reduced pressure. To the concentrate were added ethyl acetate (5 ml) and water (10 mg). The mixture was shaken and, then, left standing to form two layers. The resulting insoluble crystalline matter was collected by filtration, washed with water and ethyl acetate, successively, and dried to give 8-methylthio-4-phenylphthalazin-1-ylguanidine (246 mg). To this powdery product (62 mg) were added ethanol (5 ml), methanol (4 ml) and 2N hydrochloric acid (0.3 ml) to make a solution. The solution was concentrated to dryness under reduced pressure, which was washed with ethyl acetate and dried to give 8-methylthio-4-phenylphthalazin-1-ylguanidine dihydrochloride (hydrochloride of Compound 20) (71 mg) as yellow powdery crystals.
mp : 251-252°C
IR (Nujol) : 3300, 3110, 1685, 1595, 1550 (cm⁻¹)
¹H-NMR(DMSO-d₆) : δ 2.47 (3H, s), 7.38 (1H, d), 7.58 (5H, s), 7.71 (1H, d), 7.86 (1H, t), 7.88 (4H, broad)

| Elemental analysis for C₁₆H₁₅N₅S·2HCl·0.2H₂O | | | |
|---|---|---|---|
| Calcd. | C, 49.80; | H, 4.54; | N, 18.15 |
| Found | C, 49.91; | H, 4.75; | N, 18.02 |

### Example 22 (Production of compound 21)

8-Methylthio-4-phenylphthalazin-1-ylguanidine (56 mg) was dissolved in acetic acid (0.5 ml). To the solution was added 70% m-chloroperbenzoic acid (93 mg). The mixture was stirred for 30 minutes at room temperature. To the reaction mixture was added an aqueous solution of sodium sulfite (0.2 ml), which was stirred and, then, concentrated under reduced pressure. The concentrate was purified by means of a silica gel column chromatography, eluting with ethyl acetate/hexane/methanol, to give 8-methylsulfonyl-4-phenylphthalazin-1-ylguanidine (48 mg). To this powdery product (48 mg) were added ethanol (2 ml) and 2N hydrochloric acid (0.15 ml) to make a solution, which was concentrated to dryness. The concentrate was washed with ethyl acetate and dried to give 8-methylsulfonyl-4-phenylphthalazin-1-ylguanidine hydrochloride (hydrochloride of compound 21) (44 mg) as colorless powdery crystals.
- mp: : 223-225°C
- IR (Nujol): : 3400, 3320, 3220, 1640, 1625, 1540, 1295, 1130 (cm⁻¹)
- ¹H-NMR(DMSO-d₆): : δ 3.71 (3H, s), 7.62 (5H, s), 7.91 (4H, broad), 8.06 (1H, d), 8.20 (1H, t), 8.76 (1H, dd)

### Example 23 (Production of compound 22)

8-Methylthio-4-phenylphthalazin-1-ylguanidine (78 mg) was dissolved in acetic acid (3 ml). To the solution was added 70% m-chloroperbenzoic acid (55 mg). The mixture was stirred for 30 minutes at room temperature. The reaction mixture was concentrated under reduced pressure. The concentrate was purified by means of a silica gel column chromatography, eluting with ethyl acetate/hexane/methanol, to give 8-methylsulfinyl-4-phenylphthalazin-1-ylguanidine (61 mg). To this powdery product (40 mg) were added ethanol (5 ml) and 1N hydrochloric acid (0.25 ml) to make a solution, which was concentrated to dryness. The concentrate was washed with ethyl acetate and dried to give 8-methylsulfinyl-4-phenylphthalazin-1-ylguanidine hydrochloride (hydrochloride of Compound 22) (42 mg) as pale yellow powdery crystals.
mp : 203-204°C
IR (Nujol) : 3340, 3120, 1610, 1550, 1505, 1010 (cm⁻¹)
¹H-NMR(DMSO-d₆) : δ 2.90 (3H, s), 7.62 (5H, s), 7.99 (1H, dd), 8.07 (4H, broad), 8.23 (1H, t), 8.58 (1H, dd)

| Elemental analysis for C₁₆H₁₅N₅OS·HCl | | | |
|---|---|---|---|
| Calcd. | C, 50.59; | H, 4.48; | N, 18.44 |
| Found | C, 50.91; | H, 4.84; | N, 18.29 |

### Example 24 (compound 23)

1,2-dimethoxyethane (5 ml) was added to a mixture of 1-chloro-8-cyano-4-phenylphthalazine (325 mg) and guanidine (180 mg). The mixture was stirred for 14 hours at 65 °C. The reaction mixture was cooled in air, which was then concentrated under reduced pressure. To the concentrate were added ethyl acetate (45 ml) and water (10 ml). The mixture was shaken and, then, left standing to form two layers. The resulting insoluble crystalline product was collected by filtration, which was washed with water and ethyl acetate, successively, and dried to give 8-cyano-4-phenylphthalazin-1-ylguanidine (Compound 23) (195 mg) as yellow powdery crystals.
- mp: : 276-277°C
- IR (Nujol): : 3500, 3400, 2220, 1620, 1535, 1475, 1430 (cm⁻¹)
- ¹H-NMR(DMSO-d₆): : δ 7.15 (4H, broad), 7.52-7.67 (5H, m), 7.89 (1H, t), 8.00 (1H, dd), 8.34 (1H, dd)

### Example 25 (Production of compound 24)

1,2-Dimethoxyethane (8 ml) was added to a mixture of 8-bromo-1-chloro-4-(2-methylphenyl)phthalazine (300 mg) and guanidine (150 mg). The mixture was stirred for 14 hours at 70 °C under argon atmosphere. The reaction mixture was cooled in air, which was concentrated under reduced pressure. To the concentrate were added ethyl acetate (40 ml) and water (20 ml). The mixture was shaken and, then, left standing, to form two layers. The upper layer was washed with water, dried (anhydrous sodium sulfate) and concentrated under reduced pressure. The concentrate was purified by means of a silica gel column chromatography, eluting with ethyl acetate/hexane/methanol, to give 8-bromo-4-(2-methylphenyl)phthalazin-1-ylguanidine (230 mg). This powdery product (128 mg) was dissolved in a mixture of ethanol (5 ml) and 2N hydrochloric acid (0.4 ml). The solution was concentrated to dryness, which was washed with ethyl acetate and dried to give 8-bromo-4-(2-methylphenyl)phthalazin-1-ylguanidine dihydrochloride (hydrochloride of Compound 24) (130 mg) as colorless powdery crystals.
mp : 170-171°C
IR (Nujol) : 3070, 1690, 1635, 1595, 1550, 1380, 1330 (cm⁻¹)
¹H-NMR(DMSO-d₆) : δ 2.09 (3H, s), 7.30-7.55 (5H, m), 7.77 (1H, t), 7.94 (4H, broad), 8.28 (1H, d)

| Elemental analysis for C₁₆H₁₄N₅Br·2HCl·H₂O | | | |
|---|---|---|---|
| Calcd. | C, 42.98; | H, 4.06; | N, 15.66 |
| Found | C, 43.05; | H, 3.94; | N, 15.60 |

### Example 26 (Production of Compound 25)

1,2-Dimethoxyethane (4 ml) was added to a mixture of 8-bromo-1-chloro-4-(biphenyl-2-yl)phthalazine (168 mg) and guanidine (100 mg), which was stirred for 14 hours at 70 °C under argon atmosphere. The reaction mixture was cooled in air, which was concentrated under reduced pressure. To the concentrate was added ethyl acetate (30 ml) and water (20 ml). The mixture was shaken and, then, left standing to form two layers. The upper layer was washed with water, dried (anhydrous sodium sulfate) and concentrated under reduced pressure. The concentrate was purified by means of a silica gel column chromatography, eluting with ethyl acetate/hexane/methanol, to give 8-bromo-4-(biphenyl-2-yl)phthalazin-1-ylguanidine (Compound 25) (150 mg).
- IR (Nujol): : 3320, 1590, 1540, 1505, 1420 (cm⁻¹)
- ¹H-NMR(DMSO-d₆): : δ 6.55 (4H, broad), 7.02-7.18 (6H, m), 7.25 (1H, t), 7.31 (1H, dd), 7.50-7.60 (3H, m), 7.91 (1H, dd)

### Example 27 (Production of Compound 26)

1,2-Dimethoxyethane (6 ml) was added to a mixture of 8-bromo-1-chloro-4-(2-trifluoromethylphenyl)phthalazine (200 mg) and guanidine (150 mg), which was stirred for 13 hours at 70 °C under argon atmosphere. The reaction mixture was cooled in air, which was concentrated under reduced pressure. To the concentrate were added ethyl acetate (30 ml) and water (20 ml). The mixture was shaken and, then, left standing to form two layers. The upper layer was washed with water, dried (anhydrous sodium sulfate) and concentrated under reduced pressure. The concentrate was purified by means of a silica gel column chromatography, eluting with ethyl acetate/hexane/methanol, to give 8-bromo-4-(2-trifluoromethylphenyl)phthalazin-1-ylguanidine (Compound 26) (130 mg).
mp : 114-118°C
IR (Nujol) : 3340, 3190, 1610, 1590, 1545, 1420, 1310, 1170, 1125 (cm⁻¹)
¹H-NMR(DMSO-d₆) : δ 6.46 (4H, broad), 7.21 (1H, dd), 7.39 (1H, t), 7.43-7.56 (1H, m), 7.60-7.70 (2H, m), 7.84-7.88 (1H, m), 8.07 (1H, dd)

| Elemental analysis for C₁₆H₁₁₁N₅BrF₃·H₂O | | | |
|---|---|---|---|
| Calcd. | C, 44.88; | H, 3.06; | N, 16.35 |
| Found | C, 45.03; | H, 2.78; | N, 16.41 |

### Example 28 (Production of Compound 27)

1,2-Dimethoxyethane (3 ml) was added to a mixture of 8-bromo-1-chloro-4-(2-chlorophenyl)phthalazine (93 mg) and guanidine (100 mg), which was stirred for two hours at 80 °C under argon atmosphere. The reaction mixture was cooled in air, which was concentrated under reduced pressure. To the concentrate were added ethyl acetate (30 ml) and water (20 ml). The mixture was shaken and, then, left standing to form two layers. The upper layer was washed with water, dried (anhydrous sodium sulfate) and concentrated under reduced pressure. The concentrate was crystallized from ethyl acetate to give 8-bromo-4-(2-chlorophenyl)phthalazin-1-ylguanidine (compound 27) (60 mg).
- mp: : 226-227°C
- IR (Nujol): : 3500, 3360, 3180, 1620, 1590, 1535, 1465, 1430 (cm⁻¹)
- ¹H-NMR(CDCl₃): : δ 6.46 (4H, broad), 7.33-7.57 (6H, m), 8.09 (1H, dd)

### Example 29 (Production of Compound 28)

1,2-Dimethoxyethane (5 ml) was added to a mixture of 1-chloro-8-iodo-4-(2-methylphenyl)phthalazine (158 mg) and guanidine (100 mg), which was stirred for 14 hours at 70 °C under argon atmosphere. The reaction mixture was cooled in air, which was concentrated under reduced pressure. The concentrate was purified by means of a silica gel column chromatography, eluting with ethyl acetate/hexane/methanol, to give 8-iodo-4-(2-methylphenyl)phthalazin-1-ylguanidine (150 mg). This powdery product (140 mg) was dissolved in a mixture of methanol (5 ml) and 2N hydrochloric acid (0.35 ml). The solution was concentrated to dryness under reduced pressure. The concentrate was washed with ethyl acetate and dried to give 8-iodo-4-(2-methylphenyl)phthalazin-1-ylguanidine dihydrochloride (hydrochloride of compound 28) (130 mg) as pale yellow powdery crystals.
mp : 191-193°C
IR (Nujol) : 3310, 1600 (cm⁻¹)
¹H-NMR(DMSO-d₆) : δ 2.08 (3H, s), 7.26-7.57 (6H, m), 7.89 (4H, broad), 8.60 (1H, d)

| Elemental analysis for C₁₆H₁₄N₅I·2HCl·1/3H₂O | | | |
|---|---|---|---|
| Calcd. | C, 39.86; | H, 3.48; | N, 14.53 |
| Found | C, 39.96; | H, 3.61; | N, 14.68 |

### Example 30 (Production of Compound 29)

1,2-Dimethoxyethane (5 ml) was added to a mixture of 8-bromo-1-chloro-4-(4-fluoro-2-methylphenyl)phthalazine (125 mg) and guanidine (100 mg), which was stirred for 5 hours at 70 °C under argon atmosphere. The reaction mixture was cooled in air, which was then concentrated under reduced pressure. The concentrate was purified by means of a silica gel column chromatography, eluting with ethyl acetate/hexane/methanol, to give 8-bromo-4-(4-fluoro-2-methylphenyl)phthalazin-1-ylguanidine (118 mg). This powdery product (118 mg) was dissolved in a mixture of methanol (5 ml) and 2N hydrochloric acid (0.35 ml). The solution was concentrated to dryness under reduced pressure. The concentrate was washed with ethyl acetate and dried to give 8-bromo-4-(4-fluoro-2-methylphenyl)phthalazin-1-ylguanidine dihydrochloride (hydrochloride of Compound 29) (130 mg) as colorless powdery crystals.
- mp: : 175-177°C
- IR (Nujol): : 3050, 1680, 1590, 1380, 1330 (cm⁻¹)
- ¹H-NMR(DMSO-d₆): : δ 2.09 (3H, s), 7.20-7.42 (4H, m), 7.77 (1H, t), 7.93 (3H, broad), 8.28 (1H, d)

### Example 31 (Production of Compound 30)

1,2-Dimethoxyethane (8 ml) was added to a mixture of 1-chloro-8-nitro-4-(2-pyridyl)phthalazine (0.19 g) and guanidine (0.12 g), which was stirred for 4 hours at 70 °C under argon atmosphere. The reaction mixture was cooled in air, which was then concentrated under reduced pressure. The concentrate was purified by means of a silica gel column chromatography, eluting with ethyl acetate/methanol, to give 8-nitro-4-(2-pyridyl)phthalazin-1-ylguanidine (Compound 30) (0.12 g).
mp : 253-254°C (dec.)
¹H-NMR(DMSO-d₆) : δ 7.36 (1H, m), 7.48 (4H, broad), 7.95 (1H, dt), 8.00 (1H, d), 8.05 (1H, t), 8.33 (1H, dd), 8.40 (1H, d), 9.00 (1H, d)

| Elemental analysis for C₁₄H₁₁N₇O₂ | | | |
|---|---|---|---|
| Calcd. | C, 54.37; | H, 3.58; | N, 31.70 |
| Found | C, 54.49; | H, 3.85; | N, 31.55 |

### Example 32 (Production of Compound 31)

1,2-Dimethoxyethane (10 ml) was added to a mixture of 1,6,7-trichloro-4-(2-pyridyl)phthalazine (0.22 g) and guanidine (0.18 g), which was stirred for 5 hours at 70 °C under argon atmosphere. The reaction mixture was cooled in air, which was then concentrated under reduced pressure. The concentrate was purified by means of a silica gel column chromatography, eluting with ethyl acetate/methanol, to give 6,7-dichloro-4-(2-pyridyl)phthalazin-1-ylguanidine (Compound 31) (0.12 g).
mp : 251-252°C (dec.)
¹H-NMR(DMSO-d₆) : δ 7.3-7.8 (4H, broad), 7.53 (1H, m), 8.02 (1H, dt), 8.16 (1H, d), 8.79 (1H, d), 8.81 (1H, s), 9.05 (1H, s)

| Elemental analysis for C₁₄H₁₀N₆Cl₂·0.7H₂O | | | |
|---|---|---|---|
| Calcd. | C, 48.63; | H, 3.32; | N, 24.30 |
| Found | C, 48.67; | H, 2.98; | N, 24.00 |

### Example 33 (Production of Compound 32)

1,2-Dimethoxyethane (16 ml) was added to a mixture of 8-bromo-1-chloro-4-(3-thienyl)phthalazine (0.52 g) and guanidine (0.28 g), which was stirred for one hour at 70 °C under argon atmosphere. The reaction mixture was cooled in air, which was concentrated under reduced pressure. The resulting crystalline precipitate was washed with water, ethyl acetate and isopropyl ether, successively, to give 8-bromo-4-(3-thienyl)phthalazin-1-ylguanidine (Compound 32) (0.46 g).
- mp: : 202-204°C
- ¹H-NMR(DMSO-d₆): : δ 7.11 (4H, broad), 7.25 (1H, dd), 7.56 (1H, d), 7.65 (1H, d), 7.71 (1H, d), 8.15 (1H, d), 8.19 (1H, d)

### Example 34 (Production of hydrochloride of Compound 32)

8-Bromo-4-(3-thienyl)phthalazin-1-ylguanidine (0.20 g) was dissolved in 1,2-dimethoxyethane (30 ml). To the solution was added a 4N HCl ethyl acetate solution (1 ml). The mixture was stirred for 30 minutes at room temperature. The resulting crystalline precipitate was collected by filtration, which was washed with 1,2-dimethoxyethane, ethyl acetate and isopropyl ether, successively, followed by drying to give 8-bromo-4-(3-thienyl)phthalazin-1-ylguanidine dihydrochloride (hydrochloride of Compound 32) (0.22 g).
mp : 211-212°C
¹H-NMR(DMSO-d₆) : δ 7.32 (1H, t), 7.67 (1H, d), 7.85 (1H, d), 7.90 (5H, broad), 8.33 (2H, d)

| Elemental analysis for C₁₃H₁₀N₅BrS·2HCl | | | |
|---|---|---|---|
| Calcd. | C, 37.08; | H, 2.87; | N, 16.63 |
| Found | C, 36.93; | H, 2.99; | N, 16.51 |

### Example 35 (Production of Compound 33)

1,2-Dimethoxyethane (6 ml) was added to a mixture of 1,6,7-trichloro-4-phenylphthalazine (0.20 g) and guanidine (0.13 g), which was stirred for one hour at 70 °C under argon atmosphere. The reaction mixture was cooled in air, which was concentrated under reduced pressure. The concentrate was purified by means of a silica gel column chromatography, eluting with ethyl acetate/methanol, to give 6,7-dichloro-4-phenylphthalazin-1-ylguanidine (Compound 33) (0.11 g).
- mp: : 272-273°C (dec.)
- ¹H-NMR(DMSO-d₆): : δ 7.41 (4H, broad), 7.55-7.64 (5H, m), 7.83 (1H, s), 8.80 (1H, s)

### Example 36 (Production of Compound 34)

1,2-Dimethoxyethane (6 ml) was added to a mixture of 1,6,7-trichloro-4-phenylphthalazine (0.24 g) and guanidine (0.13 g), which was stirred for one hour at 70 °C under argon atmosphere. The reaction mixture was cooled in air and concentrated under reduced pressure. The concentrate was purified by means of a silica gel column chromatography, eluting with ethyl acetate/methanol, to give 6,7-dichloro-4-(4-fluorophenyl)phthalazin-1-ylguanidine (Compound 34) (0.13 g).
- mp: : 239-241°C
- ¹H-NMR(DMSO-d₆): : δ 7.40 (2H, t), 7.45 (4H, broad), 7.72 (2H, dd), 7.85 (1H, s), 8.79 (1H, s)

### Example 37 (Production of Compound 35)

1,2-Dimethoxyethane (20 ml) was added to a mixture of 1-chloro-8-trifluoromethyl-4-(4-fluorophenyl)phthalazine (0.78 g) and guanidine (0.42 g), which was stirred for two hours at 70 °C under argon atmosphere. The reaction mixture was cooled in air, which was then concentrated under reduced pressure. The concentrate was purified by means of a silica gel column chromatography, eluting with ethyl acetate, to give 8-trifluoromethyl-4-(4-fluorophenyl)phthalazin-1-ylguanidine (Compound 35) (0.54 g).
mp : 241-242°C
¹H-NMR(CDCl₃) : δ 6.25 (4H, broad), 7.24 (2H, t), 7.64 (2H, dd), 7.76 (1H, t), 8.03 (1H, d), 8.27 (1H, d)

| Elemental analysis for C₁₆H₁₁N₅F₃ | | | |
|---|---|---|---|
| Calcd. | C, 55.02; | H, 3.17; | N, 20.05 |
| Found | C, 54.92; | H, 3.16; | N, 19.83 |

### Example 38 (Production of methanesulfonate of Compound 35)

In a mixture of 1,2-dimethoxyethane (5 ml) and ethyl acetate (5 ml) was dissolved 8-trifluoromethyl-4-(4-fluorophenyl)phthalazin-1-ylguanidine (0.35 g). To the solution was added methanesulfonic acid (68 ml), which was stirred for 30 minutes at room temperature. The resulting crystalline precipitate was collected by filtration, which was washed with ethyl acetate and isopropyl ether, successively, and dried to give 8-trifluoromethyl-4-(4-fluorophenyl)phthalazin-1-ylguanidine methanesulfonate (methanesulfonate of Compound 35) (0.44 g).
- mp: : 152-154°C
- ¹H-NMR(DMSO-d₆): : δ 2.40 (3H, s), 7.45 (2H, t), 7.67 (2H, dd), 7.89 (4H, broad), 8.03 (1H, d), 8.14 (1H, t), 8.45 (1H, d)

### Example 39 (Production of Compound 36)

1,2-Dimethoxyethane (3 ml) was added to a mixture of 1-chloro-4-(4-fluorophenyl)-8-iodophthalazine (0.10 g) and guanidine (46 mg)., which was stirred for one hour at 70 °C under argon atmosphere. The reaction mixture was cooled in air, which was then concentrated under reduced pressure. The concentrate was purified by means of a silica gel column chromatography, eluting with ethyl acetate/methanol, to give 4-(4-fluorophenyl)-8-iodophthalazin-1-ylguanidine (Compound 36) (67 mg).
mp : 212-214°C
¹H-NMR(DMSO-d₆) : δ 7.06 (4H, broad), 7.32 (2H, t), 7.35 (1H, t), 7.63 (2H, dd), 7.70 (1H, dd), 8.50 (1H, d)

| Elemental analysis for C₁₅H₁₁NFI | | | |
|---|---|---|---|
| Calcd. | C, 44.25; | H, 2.72; | N, 17.20 |
| Found | C, 44.33; | H, 2.69; | N, 16.88 |

### Example 40 (Production of Compound 37)

1,2-Dimethoxyethane (3 ml) was added to a mixture of 1-chloro-4-(4-methylphenyl)-8-nitrophthalazine (60 mg) and guanidine (35 mg), which was stirred for one hour at 70 °C under argon atmosphere. The reaction mixture was cooled in air, which was then concentrated under reduced pressure. The concentrate was purified by means of silica gel column chromatography, eluting with ethyl acetate, to give 4-(4-methylphenyl)-8-nitrophthalazin-1-yl-guanidine (Compound 37) (48 mg).
- mp: : 223-225°C
- ¹H-NMR(DMSO-d₆): : δ 2.43 (3H, s), 7.11 (4H, broad), 7.38 (2H, d), 7.54 (2H, d), 7.93 (2H, m), 8.05 (1H, m)

### Example 41 (Production of Compound 38)

1,2-Dimethoxyethane (5 ml) was added to a mixture of 1-chloro-8-iodo-4-(4-methylphenyl)phthalazine (0.17 g) and guanidine (80 mg), which was stirred for 1.5 hour at 70 °C under argon atmosphere. The reaction was cooled in air, which was then concentrated under reduced pressure. The concentrate was purified by means of a silica gel column chromatography, eluting with ethyl acetate/methanol, to give 8-iodo-4-(4-methylphenyl)phthalazin-1-ylguanidine (Compound 38) (0.15 g).
mp : 157-159°C
¹H-NMR(DMSO-d₆) : δ 2.41 (3H, s), 7.05 (4H, broad), 7.35 (2H, d), 7.39 (1H, t), 7.47 (2H, d), 7.71 (1H, d), 8.50 (1H, d)

| Elemental analysis for C₁₆H₁₄N₅I·0.6H₂O | | | |
|---|---|---|---|
| Calcd. | C, 46.42; | H, 3.70; | N, 16.92 |
| Found | C, 46.60; | H, 3.49; | N, 16.58 |

### Example 42 (Production of compound 39)

1,2-Dimethoxyethane (6 ml) was added to a mixture of 1-chloro-8-cyano-4-(4-methylphenyl)phthalazine (0.18 g) and guanidine (0.11 g), which was stirred for 1.5 hour at 70 °C under argon atmosphere. The reaction mixture was cooled in air, which was then concentrated under reduced pressure. The concentrate was purified by means of a silica gel column chromatography, eluting with ethyl acetate, to give 8-cyano-4-(4-methylphenyl)phthalazin-1-ylguanidine (Compound 39) (0.11 g).
mp : 233-235°C (dec.)
¹H-NMR(DMSO-d₆) : δ 2.42 (3H, s), 7.15 (4H, broad), 7.37 (2H, d), 7.52 (2H, d), 7.92 (1H, t), 8.02 (1H, dd), 8.32 (1H, dd)

| Elemental analysis for C₁₇H₁₄N₆I | | | |
|---|---|---|---|
| Calcd. | C, 67.54; | H, 4.67; | N, 27.80 |
| Found | C, 67.55; | H, 4.81; | N, 27.49 |

### Example 43 (Production of Compound 40)

1,2-Dimethoxyethane (13 ml) was added to a mixture of 1-chloro-8-trifluoromethyl-4-(4-methylphenyl)phthalazine (0.43 g) and guanidine (0.24 g), which was stirred for two hours at 70 °C under argon atmosphere. The reaction mixture was cooled in air, which was then concentrated under reduced pressure. The concentrate was purified by means of a silica gel column chromatography, eluting with ethyl acetate, to give 8-trifluoromethyl-4-(4-methylphenyl)phthalazin-1-ylguanidine (compound 40) (0.33 g).
mp : 209-210°C
¹H-NMR(DMSO-d₆) : δ 2.43 (3H, s), 7.08 (4H, broad), 7.37 (2H, d), 7.50 (2H, d), 7.89 (1H, t), 7.99 (1H, d), 8.29 (1H, d)

| Elemental analysis for C₁₇H₁₄N₅F₃ | | | |
|---|---|---|---|
| Calcd. | C, 59.13; | H, 4.09; | N, 20.28 |
| Found | C, 59.27; | H, 4.27; | N, 20.15 |

### Example 44 (Production of Compound 41)

1,2-Dimethoxyethane (7 ml) was added to a mixture of 8-bromo-1-chloro-4-(4-chlorophenyl)phthalazine (0.25 g) and guanidine (0.12 g), which was stirred for two hours at 70 °C under argon atmosphere. The reaction mixture was cooled in air, which was then concentrated under reduced pressure. The concentrate was purified by means of a silica gel column chromatography, eluting with ethyl acetate/methanol, to give 8-bromo-4-(4-chlorophenyl)phthalazin-1-ylguanidine (Compound 41) (0.18 g).
mp : 205-207°C
¹H-NMR(DMSO-d₆) : δ 7.12 (4H, broad), 7.55-7.70 (6H, m), 8.12 (1H, dd)

| Elemental analysis for C₁₅H₁₁N₅BrCl | | | |
|---|---|---|---|
| Calcd. | C, 47.83; | H, 2.94; | N, 18.59 |
| Found | C, 48.06; | H, 2.92; | N, 18.22 |

### Example 45 (Production of Compound 42)

1,2-Dimethoxyethane (10 ml) was added to a mixture of 8-bromo-1-chloro-4-(4-fluorophenyl)phthalazine (0.32 g) and guanidine (0.17 g), which was stirred for one hour at 70 °C under argon atmosphere. The reaction mixture was cooled in air, which was then concentrated under reduced pressure. The concentrate was purified by means of a silica gel column chromatography, eluting with ethyl acetate/methanol, to give 8-bromo-4-(4-fluorophenyl)phthalazin-1-ylguanidine (Compound 42) (0.26 g).
mp : 224-225°C
¹H-NMR(DMSO-d₆) : δ 7.13 (4H, broad), 7.36 (2H, t), 7.55-7.69 (4H, m), 8.11 (1H, d)

| Elemental analysis for C₁₅H₁₁N₅BrF | | | |
|---|---|---|---|
| Calcd. | C, 50.02; | H, 3.08; | N, 19.44 |
| Found | C, 50.07; | H, 3.21; | N, 19.12 |

### Example 46 (Production of Compound 43)

1,2-Dimethoxyethane (9 ml) was added to a mixture of 4-(4-fluorophenyl)-8-pentafluoroethylphthalazine (0.33 g) and guanidine (0.16 g), which was stirred for one hour at 70 °C under argon atmosphere. The reaction mixture was cooled in air, which was then concentrated under reduced pressure. The concentrate was purified by means of a silica gel column chromatography, eluting with ethyl acetate, to give 4-(4-fluorophenyl)-8-pentafluoroethylphthalazin-1-ylguanidine (Compound 43) (0.10 g).
mp : 208-209°C
¹H-NMR(CDCl₃) : δ 6.58 (4H, broad), 7.25 (2H, t), 7.62 (2H, dd), 7.78 (1H, t), 8.03 (1H, d), 8.18 (1H, dd)

| Elemental analysis for C₁₇H₁₁N₅F₆·0.2H₂O | | | |
|---|---|---|---|
| Calcd. | C, 50.68; | H, 2.85; | N, 17.38 |
| Found | C, 50.97; | H, 2.68; | N, 17.08 |

### Example 47 (Production of Compound 44)

1,2-Dimethoxyethane (15 ml) was added to a mixture of 1-chloro-8-methyl-4-(2-methylphenyl)phthalazine (537 mg) and guanidine (350 mg), which was stirred for 17 hours at 85 °C under argon atmosphere. The reaction mixture was cooled in air, which was then concentrated under reduced pressure. The concentrate was solidified by the addition of isopropyl ether to give 8-methyl-4-(2-methylphenyl)phthalazin-1-ylguanidine (320 mg). This powdery product (291 mg) was dissolved in ethanol (12 ml). To the solution was added 2N hydrochloric acid (0.35 ml). The mixture was stirred for 15 minutes at room temperature, which was concentrated to dryness under reduced pressure. The concentrate was washed with ethanol and dried to give 8-methyl-4-(2-methylphenyl)phthalazin-1-ylguanidine hydrochloride (hydrochloride of Compound 44) (263 mg) as colorless prisms.
mp : 210-212°C
IR (Nujol) : 3070, 1680, 1640, 1560 (cm⁻¹)
¹H-NMR(DMSO-d₆) : δ 2.09 (3H, s), 2.91 (3H, s), 7.14 (1H, dd), 7.28-7.52 (4H, m), 7.75-7.85 (1H, m), 7.96 (4H, broad)

| Elemental analysis for C₁₅H₁₇N₅·2HCl·5H₂O | | | |
|---|---|---|---|
| Calcd. | C, 56.05; | H, 5.26; | N, 19.23 |
| Found | C, 55.98; | H, 5.04; | N, 19.21 |

### Test Example 1

Male Wistar rats (350-450 g) were anesthetized with sodium pentobarbital (50 mg/kg, i.p.). Using a disposable syringe pre-filled with 1.5 ml of a 3.8% citric acid solution, 8.5 ml of blood was collected from the abdominal aorta. The blood was centrifuged at 3000 r.p.m. for 5 seconds to obtain platelet rich plasma (PRP). The number of platelets in the PRP was counted by an automatic hemocytometer (Sysmex 2500, TOA Medical Electronics). The PRP was diluted with physiological saline to make the concentration of 40 x 10⁴ platelets per microliter. A platelet aggregometer (Hematoracer, Niko Bioscience) was used for mesurement of the platelet swelling. 200 Microliters of the PRP was poured into a cuvette and 600 µl of a sodium propionate solution (Na propionate 135, glucose 10: HEPES 20, CaCl₂ 1, MgCl₂ 1, Units mM, pH 6.7) was added while stirring at 37 °C. The change in the light transmittance of PRP which was an indicator of the platelet swelling was recorded on a plotter. The test compound was added 3 minutes before addition of the sodium propionate solution. Test compounds were dissolved in dimethyl sulfoxide (DMSO). The final concentration of DMSO was adjusted to 1%. The values obtained one minute after addition of the sodium propionate solution were subjected to analysis. The rate of the inhibition of the test compound on the increase of the light transmittance was calculated designating the difference obtained between the treatments with 1% DMSO and HOE-642 (10⁻⁵ M) as 100. The results are given as percentage inhibition in Table 1. From these results, it is clear that the compound of this invention inhibits Na-H exchange.

**Table 1**

| Cpd No. | Inhibitory ratio (%) | |
|---|---|---|
| | 3µM | 1µM |
| 4 | 78 | 66 |
| 13 | 99 | 60 |
| 24 | 78 | 62 |
| 28 | 96 | 66 |
| 36 | 78 | 58 |
| 44 | 73 | 55 |

## Claims

1. A compound of the formula: 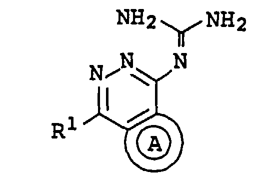 wherein ring A is a benzene ring or a nitrogen-containing 6-membered aromatic ring, each of which may be substituted; and R¹ is an aromatic ring group which may be substituted, or a salt thereof.

2. A compound of claim 1, wherein the ring A is a benzene ring, a pyridine ring, a pyrazine ring or a pyridazine ring, each of which may be substituted.

3. A compound of claim 1, wherein the ring A is a benzene ring, a pyridine ring, a pyrazine ring or a pyridazine ring, each of which may be substituted with 1 to 4 substituents selected from (1) a halogen atom, (2) a hydroxy group, (3) a nitro group, (4) a cyano group, (5) a lower alkyl group which may be substituted with halogen, (6) a lower alkoxy group which may be substituted with halogen, (7) a lower acyl group, (8) a mercapto group which may be substituted with a lower alkyl group, (9) an amino group which may be substituted with a lower alkyl group, a lower alkoxy group, a halogeno lower alkyl group, a C₃₋₆ cycloalkyl group, a hydroxy group, a carbamoyl group, a phenyl group, a phenyl-C₁₋₆ alkyl group, a lower alkanoyl group, a C₃₋₆ cycloalkyl-carbonyl group, a benzoyl group, a phenyl C₂₋₄ alkanoyl group, a lower alkoxy-carbonyl group, a phenoxy-carbonyl group, a phenyl-C₁₋₄ alkoxy-carbonyl group, a lower alkylsulfinyl group, a C₃₋₆ cycloalkylsulfinyl group, a phenylsulfinyl group, a lower sulfinyl group, a C₃₋₆ cycloalkylsulfonyl group, a lower alkoxysulfonyl group or a phenylsulfonyl group, or (10) a phenyl group which may be substituted with a halogen atom, a hydroxy group, a nitro group, a cyano group, a lower alkyl group which may be substituted with a halogen atom, a lower alkoxy group which may be substituted with a halogen atom, a lower acyl group or a mercapto group which may be substituted with a lower alkyl group.

4. A compound of claim 1, which is a compound of the formula 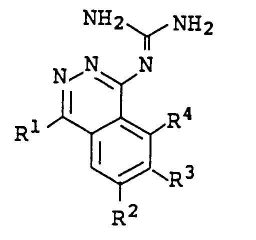 wherein R², R³ and R⁴ are independently (1) a halogen atom, (2) a hydroxy group, (3) a nitro group, (4) a cyano group, (5) a lower alkyl group which may be substituted with halogen, (6) a lower alkoxy group which may be substituted with halogen, (7) a lower acyl group, (8) a mercapto group which may be substituted with a lower alkyl group, (9) an amino group which may be substituted or (10) a phenyl group which may be substituted; and the other symbol is as defined in claim 1, or a salt thereof.

5. A compound of claim 1, wherein R¹ is a phenyl group, a pyridyl group or a thienyl group, each of which may be substituted with 1 to 4 substituents selected from (1) a halogen atom, (2) a hydroxy group, (3) a nitro group, (4) a cyano group, (5) a lower alkyl group which may be substituted with halogen, (6) a lower alkoxy group which may be substituted with halogen, (7) a lower acyl group or (8) a mercapto group which may be substituted with a lower alkyl group.

6. A compound of claim 1, wherein R¹ is a phenyl group or a thienyl group which may be substituted with a halogen atom or a lower alkyl group.

7. A compound of claim 4, wherein R¹ is a phenyl group substituted with a lower alkyl group at the 2-position of the phenyl group.

8. A compound of claim 4, wherein R² and R³ are hydrogen atoms.

9. A compound of claim 4, wherein R⁴ is a halogen atom or a lower alkyl group.

10. A compound of claim 1, which is 8-bromo-4-(2-methylphenyl)phthalazin-1-ylguanidine or a salt thereof.

11. A compound of claim 1, which is 8-methyl-4-(2-methylphenyl)phthalazin-1-ylguanidine or a salt thereof.

12. A pharmaceutical composition which comprises a compound of claim 1, in admixture with a pharmaceutically acceptable carrier, excipient or diluent.

13. A pharmaceutical composition for inhibiting sodium-hydrogen exchange, which comprises a compound of claim 1, in admixture with a pharmaceutically acceptable carrier, excipient or diluent.

14. A pharmaceutical composition for treating or preventing ischemic cardiovascular disease, which comprises a compound of claim 1, in admixture with a pharmaceutically acceptable carrier, excipient or diluent.

15. A pharmaceutical composition of claim 14, wherein the ischemic cardiovascular disease is myocardial infarction or arrhythmia.

16. Use of a compound of claim 1 for manufacturing a pharmaceutical composition.

17. Use of a compound of claim 1 for manufacturing a pharmaceutical composition for inhibiting sodium-hydrogen exchange.

18. Use of a compound of claim 1 for manufacturing a pharmaceutical composition for treating or preventing ischemic cardiovascular disease.

19. Use of claim 18, wherein the ischemic cardiovascular disease is myocardial infarction or arrhythmia.

20. A method for treating or preventing ischemic cardiovascular disease in a mammal which comprises administering an effective amount of a compound of claim 1 to said mammal.

21. A method of claim 20, wherein the ischemic cardiovascular disease is myocardial infarction or arryhythmia.

22. A process for producing a compound of claim 1, which comprises reacting a compound of the formula: 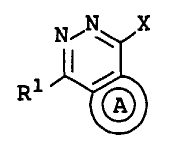 wherein X is a leaving group and the other symbols are as defined in claim 1 or a salt thereof, with a compound of the formula: 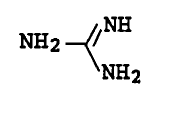 or a salt thereof.
